# EUROPEAN PATENT SPECIFICATION

(11) **EP 2 794 657 B1**
(45) Date of publication and mention of the grant of the patent: **11.10.2017**
(21) Application number: 12806868.1
(22) Date of filing: 19.12.2012
(51) Int. Cl.: C07K 16/24, A61K 39/395, A61P 17/10

(54) **METHODS FOR TREATING ACNE**
VERFAHREN ZUR BEHANDLUNG VON AKNE
MÉTHODES DE TRAITEMENT DE L'ACNÉ

(30) Priority: 19.12.2011 US 201161577450 P
(43) Date of publication of application: 29.10.2014
(60) Divisional application: 16158254.9
(73) Proprietor: XOMA (US) LLC, Berkeley, CA 94710 (US)
(72) Inventor: RUBIN, Paul, Berkeley, CA 94710 (US)
(74) Representative: Bühler, Dirk
(86) International application number: PCT/US2012/070734
(87) International publication number: WO 2013/096516

(56) References cited:
- WO-A2-2006/002057
- WO-A2-2007/002261
- SUPRIYA RAMANATHAN ET AL: "Management of Acne Vulgaris", JOURNAL OF PEDIATRIC HEALTH CARE, vol. 25, no. 5, 30 May 2011 (2011-05-30), pages 332-337, XP028271729, ISSN: 0891-5245, DOI: 10.1016/J.PEDHC.2011.05.007 [retrieved on 2011-05-30]
- DATABASE clinicaltrials.gov [Online] NIH; 21 December 2011 (2011-12-21), XOMA: "Efficacy and safety of gevokizumab to treat moderate to severe acne vulgaris", XP007921762, retrieved from Clinicaltrials.gov Database accession no. NCT0198874

## Description

### FIELD

The present disclosure relates to materials for use in treating acne in a subject including, for example, moderate and/or severe acne, using anti-IL-1β binding molecules such as an anti-IL-1β antibody or binding fragment thereof.

### BACKGROUND

Acne vulgaris is one of the most common skin diseases treated by physicians (Fleisher et al. (2000) Am J Manag Care 6(10): 1149-56). Acne vulgaris is a disease of the pilosebaceous unit of the skin, a structure that consists of the hair follicle and its associated sebaceous gland. The pathogenesis of acne is complex, incompletely understood and defined by the interplay of at least the following four factors: increased sebum production by the sebaceous glands, hyperkeratinization of the upper part of the follicle, colonization of the pilosebaceous unit by *Propionibacterium acnes (P. acnes),* and release of inflammatory mediators into the skin (Leyden (1995) J Am Acad Dermatol 32(5; Pt 3): S15-25; and Leyden (1997) N Engl J Med 336(16): 1156-62). Various mediators implicated in orchestrating the inflammatory response of the pilosebaceous unit in acne include the cytokines IL-1, IL-6, IL-8 and TNF-α. IL-1α has been reported to be present, in a bioactive form, in the majority of open comedones in acne vulgaris, where it could initiate inflammation following rupture of the pilosebaceous unit (Ingham et al. (1992) J Invest. Dermatol. 98(6): 895-901). Additionally, while the pathogenesis of follicular hyperkeratinization is still unclear, IL-1α has been reported to induce hyperkeratinization in follicular infundibulum in vitro and in vivo (Kurokawa (2009) Exp Dermatol 18(10): 821-832). Incubation of human keratinocytes with *P. acnes* isolated from acne lesions resulted in an increase in the gene expression levels of IL-1β, IL-8, TNF, and GM-CSF (Schaller et al. (2005) Brit. J. Dermatol. 153: 66-71).

In the US, acne affects more than 85% of teenagers and young adults, with a mean age at presentation for treatment of 24 years (McConnell et al. (1998) J Am Acad Dermatol 38(2 Pt 1): 221-6). Subjects with acne are prone to depression, social withdrawal, poor self-image, anxiety, and anger, and the social and psychological impact of the disease is considered comparable to that of epilepsy, asthma, diabetes, or arthritis (Mallon et al. (1999) Br J Dermatol 140(4): 62-6). The estimated direct cost of acne exceeds $2.2 billion annually (Bhambri et al. (2009) J Drugs Dermatol 8(7): 615-8).

Currently available options for the treatment of acne include, for example, topical therapies such as retinoids, benzoyl peroxide and antibiotics, certain oral antibiotics and hormonal therapies, and isotretinoin. For example, WO 2006/002057 A2 discusses methods for the treatment of acne comprising administration of inhibitors of gamma interferon, tumor necrosis alpha and interleukin-1, and the administration of DMSO, retinoids and antibiotics ,alone or in combination. Although current therapeutic agents target one or more of the pathogenic mechanisms of acne, there remains a need for effective methods of treating acne including, acne that is not responsive to treatment with anti-microbial agents and/or oral retinoids.

### SUMMARY

The present disclosure relates to materials and use of such materials for treating acne in a subject, including, for example, moderate and/or severe acne, comprising administering to the subject an IL-1β binding molecule such as an anti-IL-1β antibody or binding fragment thereof. Anti-IL-1β antibodies or binding fragments thereof may comprise a heavy chain variable region comprising: a heavy chain complementarity determining region 1 (HCDR1) comprising TSGMGVG (SEQ ID NO: 13), a heavy chain complementarity determining region 2 (HCDR2) comprising HIWWDGDESYNPSLK (SEQ ID NO: 14), and/or a heavy chain complementarity determining region 3 (HCDR3) comprising NRYDPPWFVD (SEQ ID NO: 15); and/or a light chain variable region comprising: a light chain complementarity determining region 1 (LCDR1) comprising RASQDISNYLS (SEQ ID NO: 16), a light chain complementarity determining region 2 (LCDR2) comprising YTSKLHS (SEQ ID NO: 17), and/or a light chain complementarity determining region 3 (LCDR3) comprising LQGKMLPWT (SEQ ID NO: 18). Anti-IL-1β antibodies or binding fragments thereof may comprise a heavy chain variable region of SEQ ID NO: 6 and/or a light chain variable region of SEQ ID NO: 5. Additionally, anti-IL-1β antibodies or binding fragments thereof may bind to the same epitope that an antibody comprising a heavy chain variable region of SEQ ID NO: 6 and a light chain variable region of SEQ ID NO: 5 binds to. Additionally, anti-IL-1β antibodies or binding fragments thereof may compete for the binding of an antibody having a heavy chain variable region of SEQ ID NO: 6 and a light chain variable region of SEQ ID NO: 5. Surprisingly, the uses disclosed herein provide an effective means for treating acne. Such acne may include, for example, (i) moderate and/or severe acne, and/or (ii) acne that is not responsive to conventional therapy (*e.g.,* not responsive to one or more antimicrobial agents such as antibiotics, including topical or systemic (*e.g.,* oral) antibiotics). As such, the materials and uses disclosed herein may be used (a.) to treat a mammalian (*e.g.,* human) subject suffering from acne, wherein (i) the acne is moderate and/or severe acne, and/or (ii) the acne is not responsive to conventional therapy (*e.g.,* not responsive to one or more antimicrobial agents such as antibiotics, including topical or systemic (*e.g.,* oral) antibiotics) or (b.) to prevent occurrence or reduce the frequency and/or severity of acne in an at risk subject, wherein (i) the acne is moderate and/or severe acne, and/or (ii) the acne is not responsive to conventional therapy (*e.g.,* not responsive to one or more antimicrobial agents such as antibiotics, including topical or systemic (*e.g.,* oral) antibiotics).

The present disclosure provides an anti-lL-1β antibody or binding fragment thereof for use in treating acne in a subject, wherein the acne is not responsive to treatment with one or more anti-microbial agents.

In some embodiments, which may be used or combined with any of the above or below mentioned embodiments, the uses comprise selecting a subject with acne that is not responsive to treatment with one or more anti-microbial agents.

In some embodiments, which may be used or combined with any of the above or below mentioned embodiments, the anti-microbial agent is an antibiotic.

In some embodiments, which may be used or combined with any of the above or below mentioned embodiments, the antibiotic is an oral antibiotic.

In some embodiments, which may be used or combined with any of the above or below mentioned embodiments, the acne is acne vulgaris.

In some embodiments, which may be used or combined with any of the above or below mentioned embodiments, the acne is moderate, severe, or moderate to severe acne vulgaris.

In some embodiments, which may be used or combined with any of the above or below mentioned embodiments, the use results in a reduction (*e.g.,* decrease) in inflammatory lesion count including, for example, where prior to treatment the subject had an inflammatory lesion count of at least 20.

In some embodiments, which may be used or combined with any of the above or below mentioned embodiments, the use results in an improvement in facial Investigator's Global Assessment (IGA) grade including, for example, where prior to treatment the subject had an IGA grade of 3 or 4.

In some embodiments, which may be used or combined with any of the above or below mentioned embodiments, the use results in a reduction in total lesion count.

In some embodiments, which may be used or combined with any of the above or below mentioned embodiments, the use results in a reduction in number of facial or non-facial (*e.g.,* back, neck, shoulders, and/or chest) acne lesions in the subject.

In some embodiments, which may be used or combined with any of the above or below mentioned embodiments, the use results in a reduction in severity (*e.g.,* size and/or number) of acne lesions in the subject.

In some embodiments, which may be used or combined with any of the above or below mentioned embodiments, the acne includes acne lesions that are inflammatory lesions.

In some embodiments, which may be used or combined with any of the above or below mentioned embodiments, the inflammatory lesions include lesions that are facial lesions.

In some embodiments, which may be used or combined with any of the above or below mentioned embodiments, the inflammatory lesions include lesions that are paples, pustules, or nodules/cysts.

In some embodiments, which may be used or combined with any of the above or below mentioned embodiments, the acne lesions include lesions that are non-inflammatory lesions.

In some embodiments, which may be used or combined with any of the above or below mentioned embodiments, the non-inflammatory acne lesions include lesions that are facial lesions.

In some embodiments, which may be used or combined with any of the above or below mentioned embodiments, the non-inflammatory acne lesions include lesions that are open or closed comedones.

In some embodiments, which may be used or combined with any of the above or below mentioned embodiments, the reduction in number of acne lesions is a reduction in facial acne lesion count (*e.g*., total facial acne lesion count).

In some embodiments, which may be used or combined with any of the above or below mentioned embodiments, the use results in an improvement in Investigator's Global Assessment (IGA) severity grade for facial acne.

In some embodiments, which may be used or combined with any of the above or below mentioned embodiments, the use results in an improvement in Investigator's Global Assessment (IGA) severity grade for non-facial acne.

In some embodiments, which may be used or combined with any of the above or below mentioned embodiments, the use results in an improvement in a quality of life assessment.

In some embodiments, which may be used or combined with any of the above or below mentioned embodiments, the use results in an improvement in a Cardiff Acne Disability Index (CADI) score.

In some embodiments, which may be used or combined with any of the above or below mentioned embodiments, the use results in a reduction of sebum production, a reduction in hyperkeratinization, a reduction in colonization by *P. acnes,* or a reduction in release of inflammatory mediators into the skin.

In some embodiments, which may be used or combined with any of the above or below mentioned embodiments, the use is effective to reduce a symptom of skin inflammation including, for example, redness, swelling, leukocyte infiltration, and/or lesion development.

In some embodiments, which may be used or combined with any of the above or below mentioned embodiments, the use is effective to reduce the severity of a condition of the skin associated with acne including, for example, scaling, erythema, itching, burning, and/or stinging.

In some embodiments, which may be used or combined with any of the above or below mentioned embodiments, the use is effective to improve one or more acne parameters including, for example, scaling, erythema, itching, burning, and/or stinging.

In some embodiments, which may be used or combined with any of the above or below mentioned embodiments, the use is effective to reduce the size of acne lesions, redness of acne lesions, and/or itchiness of acne lesions.

In some embodiments, which may be used or combined with any of the above or below mentioned embodiments, the use results in a delay in the recurrence of an acute acne outbreak in the subject.

In some embodiments, which may be used or combined with any of the above or below mentioned embodiments, the use results in a reduction in the severity of a recurrence of an acute acne outbreak in the subject.

In some embodiments, which may be used or combined with any of the above or below mentioned embodiments, the antibiotic is selected from the group consisting of: a penicillin, a polyketide antibiotic, a cephalosporin, a lincosamide, a quinolone, a folic acid synthesis inhibitor, a tetracycline, a rifamycin, a sulfonamide, an aminoglycoside, fusidic acid, a polypeptide antibiotic, a lipopeptide antibiotic, chloramphenicol and mupirocin.

In some embodiments, which may be used or combined with any of the above or below mentioned embodiments, the antibiotic is an oral antibiotic.

In some embodiments, which may be used or combined with any of the above or below mentioned embodiments, the antibiotic is a topical antibiotic.

In some embodiments, which may be used or combined with any of the above or below mentioned embodiments, the penicillin is penicillin, amoxicillin, benzylpenicillin, ampicillin, or augmentin.

In some embodiments, which may be used or combined with any of the above or below mentioned embodiments, the polyketide antibiotic is macrolide, azithromycin, erythromycin, or clarithromycin.

In some embodiments, which may be used or combined with any of the above or below mentioned embodiments, the cephalosporin is cefadroxil, cefixime, or cephalexin.

In some embodiments, which may be used or combined with any of the above or below mentioned embodiments, the lincosamide is clindamycin.

In some embodiments, which may be used or combined with any of the above or below mentioned embodiments, the quinolone is ciprofloxacin, levofloxacin, or moxifloxacin.

In some embodiments, which may be used or combined with any of the above or below mentioned embodiments, the folic acid synthesis inhibitor is a dihydrofolate reductase inhibitor, trimethoprim, dapsone, or co-trimoxazole.

In some embodiments, which may be used or combined with any of the above or below mentioned embodiments, the tetracycline is tetracycline, minocycline, doxycycline, demeclocycline, or oxytetracycline.

In some embodiments, which may be used or combined with any of the above or below mentioned embodiments, the rifamycin is rifampicin, rifabutin, or rifapentine.

In some embodiments, which may be used or combined with any of the above or below mentioned embodiments, the sulfonamide is sulfamethoxazole, or sulfacetamide.

In some embodiments, which may be used or combined with any of the above or below mentioned embodiments, the aminoglycoside is neomycin, amikacin, or tobramycin.

In some embodiments, which may be used or combined with any of the above or below mentioned embodiments, the polypeptide antibiotic is bacitracin, or polymixin B.

In some embodiments, which may be used or combined with any of the above or below mentioned embodiments, the lipopeptide antibiotic is daptomycin.

In some embodiments, which may be used or combined with any of the above or below mentioned embodiments, the acne is associated with *P. acnes* or *S. aureus.*

In some embodiments, which may be used or combined with any of the above or below mentioned embodiments, the antibody or binding fragment thereof binds to human IL-1β with a dissociation constant of about 1 nM or less, about 250 pM or less, about 50 pM or less, about 10 pM or less, about 1 pM or less, or about 0.3 pM or less.

In some embodiments, which may be used or combined with any of the above or below mentioned embodiments, the anti-IL-1β antibody or binding fragment thereof is a neutralizing antibody.

In some embodiments, which may be used or combined with any of the above or below mentioned embodiments, the anti-IL-1β antibody or binding fragment thereof binds to an IL-1β epitope such that the bound antibody or binding fragment substantially permits the binding of IL-1β to IL-1 receptor I (IL-1RI).

In some embodiments, which may be used or combined with any of the above or below mentioned embodiments, the antibody or binding fragment thereof does not detectably bind to IL-1α, IL-1R or IL-1Ra.

In some embodiments, which may be used or combined with any of the above or below mentioned embodiments, the anti-IL-1β antibody or binding fragment thereof comprises a heavy chain variable region comprising: a heavy chain complementarity determining region 1 (HCDR1) comprising TSGMGVG (SEQ ID NO: 13), a heavy chain complementarity determining region 2 (HCDR2) comprising HIWWDGDESYNPSLK (SEQ ID NO: 14), and/or a heavy chain complementarity determining region 3 (HCDR3) comprising NRYDPPWFVD (SEQ ID NO: 15); and/or a light chain variable region comprising: a light chain complementarity determining region 1 (LCDR1) comprising RASQDISNYLS (SEQ ID NO: 16), a light chain complementarity determining region 2 (LCDR2) comprising YTSKLHS (SEQ ID NO: 17), and/or a light chain complementarity determining region 3 (LCDR3) comprising LQGKMLPWT (SEQ ID NO: 18).

In some embodiments, which may be used or combined with any of the above or below mentioned embodiments, the antibody or binding fragment thereof comprises the light chain variable region of SEQ ID NO: 5 and the heavy chain variable region of SEQ ID NO: 6.

In some embodiments, which may be used or combined with any of the above or below mentioned embodiments, the antibody or binding fragment thereof competes with the binding of an antibody having the light chain variable region of SEQ ID NO: 5 and the heavy chain variable region of SEQ ID NO: 6.

In some embodiments, which may be used or combined with any of the above or below mentioned embodiments, the antibody or binding fragment thereof binds to an epitope of IL-1β that is substantially the same as the epitope bound by an antibody having the light chain variable region of SEQ ID NO: 5 and the heavy chain variable region of SEQ ID NO: 6.

In some embodiments, which may be used or combined with any of the above or below mentioned embodiments, the antibody or binding fragment thereof binds to an epitope contained in the amino acid sequence ESVDPKNYPKKKMEKRFVFNKIE (SEQ ID NO: 1) which corresponds to residues 83-105 of human IL-1β (SEQ ID NO: 35).

In some embodiments, which may be used or combined with any of the above or below mentioned embodiments, the antibody or binding fragment thereof binds to an epitope of human IL-1β that comprises: (a) a valine residue at a position corresponding to position 72 (Val72) of the human IL-1β sequence set forth in SEQ ID NO: 35, (b) a leucine residue at position 73 (Leu73) of the human IL-1β sequence set forth in SEQ ID NO: 35, (c) a lysine residue at a position corresponding to position 74 (Lys74) of the human IL-1β sequence set forth in SEQ ID NO: 35, (d) an aspartic acid residue at a position corresponding to position 75 (Asp75) of the human IL-1β sequence set forth in SEQ ID NO: 35, (e) a glutamine residue at a position corresponding to position 81 (Gln81) of the human IL-1β sequence set forth in SEQ ID NO: 35, (f) a glutamic acid residue at a position corresponding to position 83 (Glu83) of the human IL-1β sequence set forth in SEQ ID NO: 35, (g) a serine residue at a position corresponding to position 84 (Ser84) of the human IL-1β sequence set forth in SEQ ID NO: 35, (h) an asparagine residue at a position corresponding to position 89 (Asn89) of the human IL-1β sequence set forth in SEQ ID NO: 35, (i) a tyrosine residue at a position corresponding to position 90 (Tyr90) of the human IL-1β sequence set forth in SEQ ID NO: 35, (j) a lysine residue at a position corresponding to position 92 (Lys92) of the human IL-1β sequence set forth in SEQ ID NO: 35, (k) a lysine residue at a position corresponding to position 94 (Lys94) of the human IL-1β sequence set forth in SEQ ID NO: 35, (I) a glutamic acid residue at a position corresponding to position 96 (Glu96) of the human IL-1β sequence set forth in SEQ ID NO: 35, (m) a lysine residue at a position corresponding to position 97 (Lys97) of the human IL-1β sequence set forth in SEQ ID NO: 35, (n) an arginine residue at a position corresponding to position 98 (Arg98) of the human IL-1β sequence set forth in SEQ ID NO: 35, (o) an alanine residue at a position corresponding to position 115 (Ala115) of the human IL-1β sequence set forth in SEQ ID NO: 35, (p) a glutamine residue at a position corresponding to position 116 (glen116) of the human IL-1β sequence set forth in SEQ ID NO: 35, and/or (q) a phenylalanine residue at a position corresponding to position 117 (Phe117) of the human IL-1β sequence set forth in SEQ ID NO: 35.

In some embodiments, which may be used or combined with any of the above or below mentioned embodiments, the antibody or binding fragment thereof is human or humanized.

In some embodiments, which may be used or combined with any of the above or below mentioned embodiments, the anti-IL-1β antibody or binding fragment thereof is administered by subcutaneous, intravenous, intradermal or intramuscular injection.

In some embodiments, which may be used or combined with any of the above or below mentioned embodiments, the anti-IL-1β antibody or binding fragment thereof is administered in a dose of 1 mg/kg or less or a dose less than or equal to 1 mg/kg (*e.g.,* 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg such as 0.2 mg/kg or 0.6 mg/kg). Such administration may be monthly, every two months, every three months, every four months, every five months, every six months, or on recurrence of the acne.

In some embodiments, which may be used or combined with any of the above or below mentioned embodiments, the anti-IL-1β antibody or binding fragment thereof is administered in a dose of less than 100 mg (*e.g.,* 10 mg, 20 mg, 30 mg, 40 mg, 50 mg, 60 mg, 70 mg, 80 mg, or 90 mg). Such administration may be monthly, every two months, every three months, every four months, every five months, every six months, or on recurrance of the acne.

. In some embodiments, which may be used or combined with any of the above or below mentioned embodiments, the anti-IL-1β antibody or binding fragment thereof is administered monthly, every two months, every three months, every four months, every five months, every six months, or on recurrance of the acne.

In some embodiments, which may be used or combined with any of the above or below mentioned embodiments, the uses may further comprise administering one or more active agents for the treatment of acne.

In some embodiments, which may be used or combined with any of the above or below mentioned embodiments, the one or more active agents are selected from the group consisting of: benzoyl peroxide, a retinoid, isotretinoin, a corticosteroid, a hormone therapy, UV light, azelaic acid, a topical antibiotic, and an oral antibiotic.

In some embodiments, which may be used or combined with any of the above or below mentioned embodiments, the retinoid is retinol, retinal, tretinoin, isotretinoin, adapalene, or tazarotene.

In some embodiments, which may be used or combined with any of the above or below mentioned embodiments, the corticosteroid is prednisone.

In some embodiments, which may be used or combined with any of the above or below mentioned embodiments, the hormone therapy is an estrogen and/or progestin, a glucocorticoid, or an antiandrogen.

In some embodiments, which may be used or combined with any of the above or below mentioned embodiments, the estrogens and/or progestin is norethindrone acetate-ethinyl estradiol, or norgestimate-ethinyl estradiol.

In some embodiments, which may be used or combined with any of the above or below mentioned embodiments, the glucocorticoid is prednisone.

In some embodiments, which may be used or combined with any of the above or below mentioned embodiments, the antiandrogen is spironolactone or flutamide.

In some embodiments, which may be used or combined with any of the above or below mentioned embodiments, the topical antibiotic is clindamycin, zithromycin, erythromycin, minocycline, or tetracycline.

In some embodiments, which may be used or combined with any of the above or below mentioned embodiments, the oral antibiotic is tetracycline, erythromycin, azithromycin, doxycycline, minocycline, clindamycin, ampicillin, amoxicillin, cephalosporin, or trimethoprim/sulfamethoxazole.

The present disclosure also provides uses of an anti-IL-1β antibody or binding fragment thereof which has a lower IC₅₀ than an IL-1 receptor antagonist in a human whole blood IL-1β inhibition assay that measures IL-1β induced production of IL-8, in the manufacture of a composition for use in the treatment of acne that is not responsive to one or more antimicrobial agents such as antibiotics, including topical or systemic (*e.g.,* oral) antibiotics.

In some embodiments, which may be used or combined with any of the above or below mentioned embodiments, the IL-1 receptor antagonist is anakinra.

In some embodiments, which may be used or combined with any of the above or below mentioned embodiments, one or more active agents used and/or approved for the treatment of acne are administered in conjunction with the anti-IL-1β antibody or binding fragment thereof.

In some embodiments, which may be used or combined with any of the above or below mentioned embodiments, the one or more active agents used and/or approved for the treatment of acne are administered as a topical treatment, including, for example, benzoyl peroxide, topical retinoids (*e.g.,* tretinoin, adapalene, isotretinoin, tazarotene), and/or topical antibiotics (*e.g.,* clindamycin, erythromycin, tetracycline). Such topical treatment may include other topical therapies (*e.g.,* salicylic acid, sulphur, resorcinol, sodium sulfacetamide, aluminium chloride, zinc, nicotinamide, triethyl citrate/ethyl linoleate, dapsone, taurine bromamine, azelaic acid, sodium sulfacetamide 10%/sulfur 5%) as well as combination topicals (*e.g.,* combinations of topical treatments with different mechanisms of action).

In some embodiments, which may be used or combined with any of the above or below mentioned embodiments, the one or more active agents used and/or approved for the treatment of acne are administered as an oral treatment, including, for example, oral antibiotics (*e.g.,* tetracyclines such as tetracycline, oxytetracycline, doxycycline, lymecycline or less preferably minocycline; cotrimoxazole; quinolones such as ciprofloxacin; aminoglycosides; chloramphenicol; clindamycin; macrolides such as erythromycin and azitromycin; trimethoprim), oral contraceptives (*e.g.,* combined oral contraceptives that contain an estrogen such as ethinylestradiol and a progestogen; progestogen; estrogen), and/or oral isotretinoin (*e.g*., ACCUTANE™).

In some embodiments, which may be used or combined with any of the above or below mentioned embodiments, the one or more active agents used and/or approved for the treatment of acne are administered as a combination treatment, including, for example, retinoid +/- benzoyl peroxide +/- topical antibiotic; retinoid +/- topical antibiotic or benzoyl peroxide; sulfacetamide/sulfur or topical antibiotic + benzoyl peroxide +/- oral antibiotic(s); sulfacetamide/sulfur or topical antibiotic + benzoyl peroxide +/- oral antibiotic(s) + retinoid.

In some embodiments, which may be used or combined with any of the above or below mentioned embodiments, the one or more active agents are selected from the group consisting of: benzoyl peroxide, a retinoid, isotretinoin, a corticosteroid, a hormone therapy, UV light, azelaic acid, a topical antibiotic, and an oral antibiotic.

In some embodiments, which may be used or combined with any of the above or below mentioned embodiments, the retinoid is retinol, retinal, tretinoin, isotretinoin, adapalene, or tazarotene.

In some embodiments, which may be used or combined with any of the above or below mentioned embodiments, the corticosteroid is prednisone.

In some embodiments, which may be used or combined with any of the above or below mentioned embodiments, the hormone therapy is an estrogen and/or progestin, a glucocorticoid, or an antiandrogen.

In some embodiments, which may be used or combined with any of the above or below mentioned embodiments, the estrogens and/or progestin is norethindrone acetate-ethinyl estradiol, or norgestimate-ethinyl estradiol.

In some embodiments, which may be used or combined with any of the above or below mentioned embodiments, the glucocorticoid is prednisone.

In some embodiments, which may be used or combined with any of the above or below mentioned embodiments, the antiandrogen is spironolactone or flutamide.

In some embodiments, which may be used or combined with any of the above or below mentioned embodiments, the topical antibiotic is clindamycin, zithromycin, erythromycin, minocycline, or tetracycline.

In some embodiments, which may be used or combined with any of the above or below mentioned embodiments, the oral antibiotic is tetracycline, erythromycin, azithromycin, doxycycline, minocycline, clindamycin, ampicillin, amoxicillin, cephalosporin, or trimethoprim/sulfamethoxazole.

The present disclosure also provides uses of treating a subject with acne by scoring the acne at a first time; administering to the subject conventional therapy; scoring the acne at a second time; determining that the subject is not responsive to treatment with the conventional therapy where there is not an improvement in a score assessed to the acne between the scoring at the first time and the scoring at the second time; and administering to the subject an effective amount of anti-IL-1[3 antibody or binding fragment thereof.

The present disclosure also provides uses of treating a subject with acne by scoring the acne at a first time; administering to the subject one or more anti-microbial agents for treatment of the acne; scoring the acne at a second time; determining that the subject is not responsive to treatment with the anti-microbial agent where there is not an improvement in a score assessed to the acne between the scoring at the first time and the scoring at the second time; and administering to the subject an effective amount of anti-IL-1β antibody or binding fragment thereof.

In some embodiments, which may be used or combined with any of the above or below mentioned embodiments, the use further comprise diagnosing acne in the subject.

In some embodiments, which may be used or combined with any of the above or below mentioned embodiments, the anti-microbial agent is an antibiotic.

In some embodiments, which may be used or combined with any of the above or below mentioned embodiments, the antibiotic is an oral antibiotic.

In some embodiments, which may be used or combined with any of the above or below mentioned embodiments, the acne is acne vulgaris.

In some embodiments, which may be used or combined with any of the above or below mentioned embodiments, the acne is moderate, severe, or moderate to severe acne vulgaris.

In some embodiments, which may be used or combined with any of the above or below mentioned embodiments, the use results in a reduction (*e.g*., decrease) in inflammatory lesion count including, for example, where prior to treatment the subject had an inflammatory lesion count of at least 20.

In some embodiments, which may be used or combined with any of the above or below mentioned embodiments, the use results in an improvement in facial Investigator's Global Assessment (IGA) grade including, for example, where prior to treatment the subject had an IGA grade of 3 or 4.

In some embodiments, which may be used or combined with any of the above or below mentioned embodiments, the use results in a reduction in total lesion count.

In some embodiments, which may be used or combined with any of the above or below mentioned embodiments, the use results in a reduction in number of facial or non-facial (*e.g.,* back, neck, shoulders, and/or chest) acne lesions in the subject.

In some embodiments, which may be used or combined with any of the above or below mentioned embodiments, the use results in a reduction in severity (*e.g*., size and/or number) of acne lesions in the subject.

In some embodiments, which may be used or combined with any of the above or below mentioned embodiments, the acne includes acne lesions that are inflammatory lesions.

In some embodiments, which may be used or combined with any of the above or below mentioned embodiments, the inflammatory lesions include lesions that are facial lesions.

In some embodiments, which may be used or combined with any of the above or below mentioned embodiments, the inflammatory lesions include lesions that are paples, pustules, or nodules/cysts.

In some embodiments, which may be used or combined with any of the above or below mentioned embodiments, the acne lesions include lesions that are non-inflammatory lesions.

In some embodiments, which may be used or combined with any of the above or below mentioned embodiments, the non-inflammatory acne lesions include lesions that are facial lesions.

In some embodiments, which may be used or combined with any of the above or below mentioned embodiments, the non-inflammatory acne lesions include lesions that are open or closed comedones.

In some embodiments, which may be used or combined with any of the above or below mentioned embodiments, the reduction in number of acne lesions is a reduction in facial acne lesion count (*e.g*., total facial acne lesion count).

In some embodiments, which may be used or combined with any of the above or below mentioned embodiments, the use results in an improvement in Investigator's Global Assessment (IGA) severity grade for facial acne.

In some embodiments, which may be used or combined with any of the above or below mentioned embodiments, the use results in an improvement in Investigator's Global Assessment (IGA) severity grade for non-facial acne.

In some embodiments, which may be used or combined with any of the above or below mentioned embodiments, the use results in an improvement in a quality of life assessment.

In some embodiments, which may be used or combined with any of the above or below mentioned embodiments, the use results in an improvement in a Cardiff Acne Disability Index (CADI) score.

In some embodiments, which may be used or combined with any of the above or below mentioned embodiments, the use results in a reduction of sebum production, a reduction in hyperkeratinization, a reduction in colonization by *P. acnes,* or a reduction in release of inflammatory mediators into the skin.

In some embodiments, which may be used or combined with any of the above or below mentioned embodiments, the use is effective to reduce a symptom of skin inflammation including, for example, redness, swelling, leukocyte infiltration, and/or lesion development.

In some embodiments, which may be used or combined with any of the above or below mentioned embodiments, the use is effective to reduce the severity of a condition of the skin associated with acne including, for example, scaling, erythema, itching, burning, and/or stinging.

In some embodiments, which may be used or combined with any of the above or below mentioned embodiments, the use is effective to improve one or more acne parameters including, for example, scaling, erythema, itching, burning, and/or stinging.

In some embodiments, which may be used or combined with any of the above or below mentioned embodiments, the use is effective to reduce the size of acne lesions, redness of acne lesions, and/or itchiness of acne lesions.

In some embodiments, which may be used or combined with any of the above or below mentioned embodiments, the use results in a delay in the recurrence of an acute acne outbreak in the subject.

In some embodiments, which may be used or combined with any of the above or below mentioned embodiments, the use results in a reduction in the severity of a recurrence of an acute acne outbreak in the subject.

In some embodiments, which may be used or combined with any of the above or below mentioned embodiments, the antibiotic is selected from the group consisting of: a penicillin, a polyketide antibiotic, a cephalosporin, a lincosamide, a quinolone, a folic acid synthesis inhibitor, a tetracycline, a rifamycin, a sulfonamide, an aminoglycoside, fusidic acid, a polypeptide antibiotic, a lipopeptide antibiotic, chloramphenicol and mupirocin.

In some embodiments, which may be used or combined with any of the above or below mentioned embodiments, the antibiotic is an oral antibiotic.

In some embodiments, which may be used or combined with any of the above or below mentioned embodiments, the antibiotic is a topical antibiotic.

In some embodiments, which may be used or combined with any of the above or below mentioned embodiments, the penicillin is penicillin, amoxicillin, benzylpenicillin, ampicillin, or augmentin.

In some embodiments, which may be used or combined with any of the above or below mentioned embodiments, the polyketide antibiotic is macrolide, azithromycin, erythromycin, or clarithromycin.

In some embodiments, which may be used or combined with any of the above or below mentioned embodiments, the cephalosporin is cefadroxil, cefixime, or cephalexin.

In some embodiments, which may be used or combined with any of the above or below mentioned embodiments, the lincosamide is clindamycin.

In some embodiments, which may be used or combined with any of the above or below mentioned embodiments, the quinolone is ciprofloxacin, levofloxacin, or moxifloxacin.

In some embodiments, which may be used or combined with any of the above or below mentioned embodiments, the folic acid synthesis inhibitor is a dihydrofolate reductase inhibitor, trimethoprim, dapsone, or co-trimoxazole.

In some embodiments, which may be used or combined with any of the above or below mentioned embodiments, the tetracycline is tetracycline, minocycline, doxycycline, demeclocycline, or oxytetracycline.

In some embodiments, which may be used or combined with any of the above or below mentioned embodiments, the rifamycin is rifampicin, rifabutin, or rifapentine.

In some embodiments, which may be used or combined with any of the above or below mentioned embodiments, the sulfonamide is sulfamethoxazole, or sulfacetamide.

In some embodiments, which may be used or combined with any of the above or below mentioned embodiments, the aminoglycoside is neomycin, amikacin, or tobramycin.

In some embodiments, which may be used or combined with any of the above or below mentioned embodiments, the polypeptide antibiotic is bacitracin, or polymixin B.

In some embodiments, which may be used or combined with any of the above or below mentioned embodiments, the lipopeptide antibiotic is daptomycin.

In some embodiments, which may be used or combined with any of the above or below mentioned embodiments, the acne is associated with *P. acnes* or *S. aureus.*

In some embodiments, which may be used or combined with any of the above or below mentioned embodiments, the antibody or binding fragment thereof binds to human IL-1β with a dissociation constant of about 1 nM or less, about 250 pM or less, about 50 pM or less, about 10 pM or less, about 1 pM or less, or about 0.3 pM or less.

In some embodiments, which may be used or combined with any of the above or below mentioned embodiments, the anti-IL-1β antibody or binding fragment thereof is a neutralizing antibody.

In some embodiments, which may be used or combined with any of the above or below mentioned embodiments, the anti-IL-1β antibody or binding fragment thereof binds to an IL-1β epitope such that the bound antibody or fragment substantially permits the binding of IL-1β to IL-1 receptor I (IL-1RI).

In some embodiments, which may be used or combined with any of the above or below mentioned embodiments, the antibody or binding fragment thereof does not detectably bind to IL-1α, IL-1R or IL-1Ra.

In some embodiments, which may be used or combined with any of the above or below mentioned embodiments, the anti-IL-1β antibody or binding fragment thereof comprises a heavy chain variable region comprising: a heavy chain complementarity determining region 1 (HCDR1) comprising TSGMGVG (SEQ ID NO: 13), a heavy chain complementarity determining region 2 (HCDR2) comprising HIWWDGDESYNPSLK (SEQ ID NO: 14), and/or a heavy chain complementarity determining region 3 (HCDR3) comprising NRYDPPWFVD (SEQ ID NO: 15); and/or a light chain variable region comprising: a light chain complementarity determining region 1 (LCDR1) comprising RASQDISNYLS (SEQ ID NO: 16), a light chain complementarity determining region 2 (LCDR2) comprising YTSKLHS (SEQ ID NO: 17), and/or a light chain complementarity determining region 3 (LCDR3) comprising LQGKMLPWT (SEQ ID NO: 18).

In some embodiments, which may be used or combined with any of the above or below mentioned embodiments, the antibody or binding fragment thereof comprises the light chain variable region of SEQ ID NO: 5 and the heavy chain variable region of SEQ ID NO: 6.

In some embodiments, which may be used or combined with any of the above or below mentioned embodiments, the antibody or binding fragment thereof competes with the binding of an antibody having the light chain variable region of SEQ ID NO: 5 and the heavy chain variable region of SEQ ID NO: 6.

In some embodiments, which may be used or combined with any of the above or below mentioned embodiments, the antibody or binding fragment thereof binds to an epitope of IL-1β that is substantially the same as the epitope bound by an antibody having the light chain variable region of SEQ ID NO: 5 and the heavy chain variable region of SEQ ID NO: 6.

In some embodiments, which may be used or combined with any of the above or below mentioned embodiments, the antibody or binding fragment thereof binds to an epitope contained in the amino acid sequence ESVDPKNYPKKKMEKRFVFNKIE (SEQ ID NO: 1) which corresponds to residues 83-105 of human IL-1β (SEQ ID NO: 35).

In some embodiments, which may be used or combined with any of the above or below mentioned embodiments, the antibody or binding fragment thereof binds to an epitope of human IL-1β that comprises: (a) a valine residue at a position corresponding to position 72 (Val72) of the human IL-1β sequence set forth in SEQ ID NO: 35, (b) a leucine residue at position 73 (Leu73) of the human IL-1β sequence set forth in SEQ ID NO: 35, (c) a lysine residue at a position corresponding to position 74 (Lys74) of the human IL-1β sequence set forth in SEQ ID NO: 35, (d) an aspartic acid residue at a position corresponding to position 75 (Asp75) of the human IL-1β sequence set forth in SEQ ID NO: 35, (e) a glutamine residue at a position corresponding to position 81 (Gln81) of the human IL-1β sequence set forth in SEQ ID NO: 35, (f) a glutamic acid residue at a position corresponding to position 83 (Glu83) of the human IL-1β sequence set forth in SEQ ID NO: 35, (g) a serine residue at a position corresponding to position 84 (Ser84) of the human IL-1β sequence set forth in SEQ ID NO: 35, (h) an asparagine residue at a position corresponding to position 89 (Asn89) of the human IL-1β sequence set forth in SEQ ID NO: 35, (i) a tyrosine residue at a position corresponding to position 90 (Tyr90) of the human IL-1β sequence set forth in SEQ ID NO: 35, (j) a lysine residue at a position corresponding to position 92 (Lys92) of the human IL-1β sequence set forth in SEQ ID NO: 35, (k) a lysine residue at a position corresponding to position 94 (Lys94) of the human IL-1β sequence set forth in SEQ ID NO: 35, (I) a glutamic acid residue at a position corresponding to position 96 (Glu96) of the human IL-1β sequence set forth in SEQ ID NO: 35, (m) a lysine residue at a position corresponding to position 97 (Lys97) of the human IL-1β sequence set forth in SEQ ID NO: 35, (n) an arginine residue at a position corresponding to position 98 (Arg98) of the human IL-1β sequence set forth in SEQ ID NO: 35, (o) an alanine residue at a position corresponding to position 115 (Ala115) of the human IL-1β sequence set forth in SEQ ID NO: 35, (p) a glutamine residue at a position corresponding to position 116 (GIn116) of the human IL-1β sequence set forth in SEQ ID NO: 35, and/or (q) a phenylalanine residue at a position corresponding to position 117 (Phe117) of the human IL-1β sequence set forth in SEQ ID NO: 35.

In some embodiments, which may be used or combined with any of the above or below mentioned embodiments, the antibody or binding fragment thereof is human or humanized.

In some embodiments, which may be used or combined with any of the above or below mentioned embodiments, the anti-IL-1β antibody or binding fragment thereof is administered by subcutaneous, intravenous, intradermal or intramuscular injection.

In some embodiments, which may be used or combined with any of the above or below mentioned embodiments, the anti-IL-1β antibody or binding fragment thereof is administered in a dose of 1 mg/kg or less or a dose less than or equal to 1 mg/kg (*e.g.,* 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg such as 0.2 mg/kg or 0.6 mg/kg). Such administration may be monthly, every two months, every three months, every four months, every five months, every six months, or on recurrance of the acne.

In some embodiments, which may be used or combined with any of the above or below mentioned embodiments, the anti-IL-1β antibody or binding fragment thereof is administered in a dose of less than 100 mg (*e.g.,* 10 mg, 20 mg, 30 mg, 40 mg, 50 mg, 60 mg, 70 mg, 80 mg, or 90 mg). Such administration may be monthly, every two months, every three months, every four months, every five months, every six months, or on recurrance of the acne.

. In some embodiments, which may be used or combined with any of the above or below mentioned embodiments, the anti-IL-1β antibody or binding fragment thereof is administered monthly, every two months, every three months, every four months, every five months, every six months, or on recurrance of the acne.

In some embodiments, which may be used or combined with any of the above or below mentioned embodiments, the uses may further comprise administering one or more active agents for the treatment of acne.

In some embodiments, which may be used or combined with any of the above or below mentioned embodiments, the one or more active agents are selected from the group consisting of: benzoyl peroxide, a retinoid, isotretinoin, a corticosteroid, a hormone therapy, UV light, azelaic acid, a topical antibiotic, and an oral antibiotic.

In some embodiments, which may be used or combined with any of the above or below mentioned embodiments, the retinoid is retinol, retinal, tretinoin, isotretinoin, adapalene, or tazarotene.

In some embodiments, which may be used or combined with any of the above or below mentioned embodiments, the corticosteroid is prednisone.

In some embodiments, which may be used or combined with any of the above or below mentioned embodiments, the hormone therapy is an estrogen and/or progestin, a glucocorticoid, or an antiandrogen.

In some embodiments, which may be used or combined with any of the above or below mentioned embodiments, the estrogens and/or progestin is norethindrone acetate-ethinyl estradiol, or norgestimate-ethinyl estradiol.

In some embodiments, which may be used or combined with any of the above or below mentioned embodiments, the glucocorticoid is prednisone.

In some embodiments, which may be used or combined with any of the above or below mentioned embodiments, the antiandrogen is spironolactone or flutamide.

In some embodiments, which may be used or combined with any of the above or below mentioned embodiments, the topical antibiotic is clindamycin, zithromycin, erythromycin, minocycline, or tetracycline.

In some embodiments, which may be used or combined with any of the above or below mentioned embodiments, the oral antibiotic is tetracycline, erythromycin, azithromycin, doxycycline, minocycline, clindamycin, ampicillin, amoxicillin, cephalosporin, or trimethoprim/sulfamethoxazole.

The present disclosure also provides an anti-IL-1β antibody or binding fragment thereof which has a lower IC₅₀ than an IL-1 receptor antagonist in a human whole blood IL-1β inhibition assay that measures IL-1β induced production of IL-8, for use in the treatment of acne that is not responsive to one or more antimicrobial agents such as antibiotics, including topical or systemic (*e.g.,* oral) antibiotics.

In some embodiments, which may be used or combined with any of the above or below mentioned embodiments, the IL-1 receptor antagonist is anakinra.

In some embodiments, which may be used or combined with any of the above or below mentioned embodiments, one or more active agents used and/or approved for the treatment of acne are administered in conjunction with the anti-IL-1β antibody or binding fragment thereof.

In some embodiments, which may be used or combined with any of the above or below mentioned embodiments, the one or more active agents used and/or approved for the treatment of acne are administered as a topical treatment, including, for example, benzoyl peroxide, topical retinoids (*e.g.,* tretinoin, adapalene, isotretinoin, tazarotene), and/or topical antibiotics (*e.g.,* clindamycin, erythromycin, tetracycline). Such topical treatment may include other topical therapies (*e.g.,* salicylic acid, sulphur, resorcinol, sodium sulfacetamide, aluminium chloride, zinc, nicotinamide, triethyl citrate/ethyl linoleate, dapsone, taurine bromamine, azelaic acid, sodium sulfacetamide 10%/sulfur 5%) as well as combination topicals (*e.g.,* combinations of topical treatments with different mechanisms of action).

In some embodiments, which may be used or combined with any of the above or below mentioned embodiments, the one or more active agents used and/or approved for the treatment of acne are administered as an oral treatment, including, for example, oral antibiotics (*e.g.,* tetracyclines such as tetracycline, oxytetracycline, doxycycline, lymecycline or less preferably minocycline; cotrimoxazole; quinolones such as ciprofloxacin; aminoglycosides; chloramphenicol; clindamycin; macrolides such as erythromycin and azitromycin; trimethoprim), oral contraceptives (*e.g.,* combined oral contraceptives that contain an estrogen such as ethinylestradiol and a progestogen; progestogen; estrogen), and/or oral isotretinoin (*e.g*., ACCUTANE™).

In some embodiments, which may be used or combined with any of the above or below mentioned embodiments, the one or more active agents used and/or approved for the treatment of acne are administered as a combination treatment, including, for example, retinoid +/- benzoyl peroxide +/- topical antibiotic; retinoid +/- topical antibiotic or benzoyl peroxide; sulfacetamide/sulfur or topical antibiotic + benzoyl peroxide +/- oral antibiotic(s); sulfacetamide/sulfur or topical antibiotic + benzoyl peroxide +/- oral antibiotic(s) + retinoid.

In some embodiments, which may be used or combined with any of the above or below mentioned embodiments, the one or more active agents are selected from the group consisting of: benzoyl peroxide, retinol, retinal, tretinoin, isotretinoin, adapalene, isotretinoin, prednisone, tretinoin, clindamycin, zithromycin, erythromycin, minocycline, and tetracycline.

The present disclosure also provides an amount of an anti-IL-1β antibody or binding fragment thereof comprising a heavy chain variable region of SEQ ID NO: 6 and a light chain variable region of SEQ ID NO: 5 for use in treating acne which is not responsive to treatment with one or more anti-microbial agents.

In some embodiments, which may be used or combined with any of the above or below mentioned embodiments, the uses comprise selecting a subject with acne that is not responsive to treatment with one or more anti-microbial agents.

In some embodiments, which may be used or combined with any of the above or below mentioned embodiments, the anti-microbial agent is an antibiotic.

In some embodiments, which may be used or combined with any of the above or below mentioned embodiments, the antibiotic is an oral antibiotic.

In some embodiments, which may be used or combined with any of the above or below mentioned embodiments, the acne is acne vulgaris.

In some embodiments, which may be used or combined with any of the above or below mentioned embodiments, the acne is moderate, severe, or moderate to severe acne vulgaris.

In some embodiments, which may be used or combined with any of the above or below mentioned embodiments, the use results in a reduction (*e.g*., decrease) in inflammatory lesion count including, for example, where prior to treatment the subject had an inflammatory lesion count of at least 20.

In some embodiments, which may be used or combined with any of the above or below mentioned embodiments, the use results in an improvement in facial Investigator's Global Assessment (IGA) grade including, for example, where prior to treatment the subject had an IGA grade of 3 or 4.

In some embodiments, which may be used or combined with any of the above or below mentioned embodiments, the use results in a reduction in total lesion count.

In some embodiments, which may be used or combined with any of the above or below mentioned embodiments, the use results in a reduction in number of facial or non-facial (*e.g.,* back, neck, shoulders, and/or chest) acne lesions in the subject.

In some embodiments, which may be used or combined with any of the above or below mentioned embodiments, the use results in a reduction in severity (*e.g*., size and/or number) of acne lesions in the subject.

In some embodiments, which may be used or combined with any of the above or below mentioned embodiments, the acne includes acne lesions that are inflammatory lesions.

In some embodiments, which may be used or combined with any of the above or below mentioned embodiments, the inflammatory lesions include lesions that are facial lesions.

In some embodiments, which may be used or combined with any of the above or below mentioned embodiments, the inflammatory lesions include lesions that are paples, pustules, or nodules/cysts.

In some embodiments, which may be used or combined with any of the above or below mentioned embodiments, the acne lesions include lesions that are non-inflammatory lesions.

In some embodiments, which may be used or combined with any of the above or below mentioned embodiments, the non-inflammatory acne lesions include lesions that are facial lesions.

In some embodiments, which may be used or combined with any of the above or below mentioned embodiments, the non-inflammatory acne lesions include lesions that are open or closed comedones.

In some embodiments, which may be used or combined with any of the above or below mentioned embodiments, the reduction in number of acne lesions is a reduction in facial acne lesion count (*e.g*., total facial acne lesion count).

In some embodiments, which may be used or combined with any of the above or below mentioned embodiments, the use results in an improvement in Investigator's Global Assessment (IGA) severity grade for facial acne.

In some embodiments, which may be used or combined with any of the above or below mentioned embodiments, the use results in an improvement in Investigator's Global Assessment (IGA) severity grade for non-facial acne.

In some embodiments, which may be used or combined with any of the above or below mentioned embodiments, the use results in an improvement in a quality of life assessment.

In some embodiments, which may be used or combined with any of the above or below mentioned embodiments, the use results in an improvement in a Cardiff Acne Disability Index (CADI) score.

In some embodiments, which may be used or combined with any of the above or below mentioned embodiments, the use results in a reduction of sebum production, a reduction in hyperkeratinization, a reduction in colonization by *P. acnes,* or a reduction in release of inflammatory mediators into the skin.

In some embodiments, which may be used or combined with any of the above or below mentioned embodiments, the use is effective to reduce a symptom of skin inflammation including, for example, redness, swelling, leukocyte infiltration, and/or lesion development.

In some embodiments, which may be used or combined with any of the above or below mentioned embodiments, the use is effective to reduce the severity of a condition of the skin associated with acne including, for example, scaling, erythema, itching, burning, and/or stinging.

In some embodiments, which may be used or combined with any of the above or below mentioned embodiments, the use is effective to improve one or more acne parameters including, for example, scaling, erythema, itching, burning, and/or stinging.

In some embodiments, which may be used or combined with any of the above or below mentioned embodiments, the use is effective to reduce the size of acne lesions, redness of acne lesions, and/or itchiness of acne lesions.

In some embodiments, which may be used or combined with any of the above or below mentioned embodiments, the use results in a delay in the recurrence of an acute acne outbreak in the subject.

In some embodiments, which may be used or combined with any of the above or below mentioned embodiments, the use results in a reduction in the severity of a recurrence of an acute acne outbreak in the subject.

In some embodiments, which may be used or combined with any of the above or below mentioned embodiments, the antibiotic is selected from the group consisting of: a penicillin, a polyketide antibiotic, a cephalosporin, a lincosamide, a quinolone, a folic acid synthesis inhibitor, a tetracycline, a rifamycin, a sulfonamide, an aminoglycoside, fusidic acid, a polypeptide antibiotic, a lipopeptide antibiotic, chloramphenicol and mupirocin.

In some embodiments, which may be used or combined with any of the above or below mentioned embodiments, the antibiotic is an oral antibiotic.

In some embodiments, which may be used or combined with any of the above or below mentioned embodiments, the antibiotic is a topical antibiotic.

In some embodiments, which may be used or combined with any of the above or below mentioned embodiments, the penicillin is penicillin, amoxicillin, benzylpenicillin, ampicillin, or augmentin.

In some embodiments, which may be used or combined with any of the above or below mentioned embodiments, the polyketide antibiotic is macrolide, azithromycin, erythromycin, or clarithromycin.

In some embodiments, which may be used or combined with any of the above or below mentioned embodiments, the cephalosporin is cefadroxil, cefixime, or cephalexin.

In some embodiments, which may be used or combined with any of the above or below mentioned embodiments, the lincosamide is clindamycin.

In some embodiments, which may be used or combined with any of the above or below mentioned embodiments, the quinolone is ciprofloxacin, levofloxacin, or moxifloxacin.

In some embodiments, which may be used or combined with any of the above or below mentioned embodiments, the folic acid synthesis inhibitor is a dihydrofolate reductase inhibitor, trimethoprim, dapsone, or co-trimoxazole.

In some embodiments, which may be used or combined with any of the above or below mentioned embodiments, the tetracycline is tetracycline, minocycline, doxycycline, demeclocycline, or oxytetracycline.

In some embodiments, which may be used or combined with any of the above or below mentioned embodiments, the rifamycin is rifampicin, rifabutin, or rifapentine.

In some embodiments, which may be used or combined with any of the above or below mentioned embodiments, the sulfonamide is sulfamethoxazole, or sulfacetamide.

In some embodiments, which may be used or combined with any of the above or below mentioned embodiments, the aminoglycoside is neomycin, amikacin, or tobramycin.

In some embodiments, which may be used or combined with any of the above or below mentioned embodiments, the polypeptide antibiotic is bacitracin, or polymixin B.

In some embodiments, which may be used or combined with any of the above or below mentioned embodiments, the lipopeptide antibiotic is daptomycin.

In some embodiments, which may be used or combined with any of the above or below mentioned embodiments, the acne is associated with *P. acnes* or *S. aureus.*

In some embodiments, which may be used or combined with any of the above or below mentioned embodiments, the anti-IL-1β antibody or binding fragment thereof is administered by subcutaneous, intravenous, intradermal or intramuscular injection.

In some embodiments, which may be used or combined with any of the above or below mentioned embodiments, the anti-IL-1β antibody or binding fragment thereof is administered in a dose of 1 mg/kg or less or a dose less than or equal to 1 mg/kg (*e.g.,* 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg such as 0.2 mg/kg or 0.6 mg/kg). Such administration may be monthly, every two months, every three months, every four months, every five months, every six months, or on recurrance of the acne.

In some embodiments, which may be used or combined with any of the above or below mentioned embodiments, the anti-IL-1β antibody or binding fragment thereof is administered in a dose of less than 100 mg (*e.g.,* 10 mg, 20 mg, 30 mg, 40 mg, 50 mg, 60 mg, 70 mg, 80 mg, or 90 mg). Such administration may be monthly, every two months, every three months, every four months, every five months, every six months, or on recurrance of the acne.

. In some embodiments, which may be used or combined with any of the above or below mentioned embodiments, the anti-IL-1β antibody or binding fragment thereof is administered monthly, every two months, every three months, every four months, every five months, every six months, or on recurrance of the acne.

In some embodiments, which may be used or combined with any of the above or below mentioned embodiments, the uses may further comprise administering one or more active agents for the treatment of acne.

In some embodiments, which may be used or combined with any of the above or below mentioned embodiments, the one or more active agents are selected from the group consisting of: benzoyl peroxide, a retinoid, isotretinoin, a corticosteroid, a hormone therapy, UV light, azelaic acid, a topical antibiotic, and an oral antibiotic.

In some embodiments, which may be used or combined with any of the above or below mentioned embodiments, the retinoid is retinol, retinal, tretinoin, isotretinoin, adapalene, or tazarotene.

In some embodiments, which may be used or combined with any of the above or below mentioned embodiments, the corticosteroid is prednisone.

In some embodiments, which may be used or combined with any of the above or below mentioned embodiments, the hormone therapy is an estrogen and/or progestin, a glucocorticoid, or an antiandrogen.

In some embodiments, which may be used or combined with any of the above or below mentioned embodiments, the estrogens and/or progestin is norethindrone acetate-ethinyl estradiol, or norgestimate-ethinyl estradiol.

In some embodiments, which may be used or combined with any of the above or below mentioned embodiments, the glucocorticoid is prednisone.

In some embodiments, which may be used or combined with any of the above or below mentioned embodiments, the antiandrogen is spironolactone or flutamide.

In some embodiments, which may be used or combined with any of the above or below mentioned embodiments, the topical antibiotic is clindamycin, zithromycin, erythromycin, minocycline, or tetracycline.

In some embodiments, which may be used or combined with any of the above or below mentioned embodiments, the oral antibiotic is tetracycline, erythromycin, azithromycin, doxycycline, minocycline, clindamycin, ampicillin, amoxicillin, cephalosporin, or trimethoprim/sulfamethoxazole.

The present disclosure also provides an amount of an anti-IL-1β antibody or binding fragment thereof, wherein the antibody or fragment thereof competes with the binding of an antibody having the light chain variable region of SEQ ID NO:5 and the heavy chain variable region of SEQ ID NO:6 for use in treating acne which is not responsive to treatment with one ore more-microbial agents.

In some embodiments, which may be used or combined with any of the above or below mentioned embodiments, the anti-microbial agent is an antibiotic.

In some embodiments, which may be used or combined with any of the above or below mentioned embodiments, the antibiotic is an oral antibiotic.

In some embodiments, which may be used or combined with any of the above or below mentioned embodiments, the acne is moderate, severe, or moderate to severe acne vulgaris.

In some embodiments, which may be used or combined with any of the above or below mentioned embodiments, the use results in a reduction (*e.g*., decrease) in inflammatory lesion count including, for example, where prior to treatment the subject had an inflammatory lesion count of at least 20.

In some embodiments, which may be used or combined with any of the above or below mentioned embodiments, the use results in an improvement in facial Investigator's Global Assessment (IGA) grade including, for example, where prior to treatment the subject had an IGA grade of 3 or 4.

In some embodiments, which may be used or combined with any of the above or below mentioned embodiments, the use results in a reduction in total lesion count.

In some embodiments, which may be used or combined with any of the above or below mentioned embodiments, the use results in a reduction in number of facial or non-facial (*e.g.,* back, neck, shoulders, and/or chest) acne lesions in the subject.

In some embodiments, which may be used or combined with any of the above or below mentioned embodiments, the use results in a reduction in severity (*e.g*., size and/or number) of acne lesions in the subject.

In some embodiments, which may be used or combined with any of the above or below mentioned embodiments, the acne includes acne lesions that are inflammatory lesions.

In some embodiments, which may be used or combined with any of the above or below mentioned embodiments, the inflammatory lesions include lesions that are facial lesions.

In some embodiments, which may be used or combined with any of the above or below mentioned embodiments, the inflammatory lesions include lesions that are paples, pustules, or nodules/cysts.

In some embodiments, which may be used or combined with any of the above or below mentioned embodiments, the acne lesions include lesions that are non-inflammatory lesions.

In some embodiments, which may be used or combined with any of the above or below mentioned embodiments, the non-inflammatory acne lesions include lesions that are facial lesions.

In some embodiments, which may be used or combined with any of the above or below mentioned embodiments, the non-inflammatory acne lesions include lesions that are open or closed comedones.

In some embodiments, which may be used or combined with any of the above or below mentioned embodiments, the reduction in number of acne lesions is a reduction in facial acne lesion count (*e.g*., total facial acne lesion count).

In some embodiments, which may be used or combined with any of the above or below mentioned embodiments, the use results in an improvement in Investigator's Global Assessment (IGA) severity grade for facial acne.

In some embodiments, which may be used or combined with any of the above or below mentioned embodiments, the use results in an improvement in Investigator's Global Assessment (IGA) severity grade for non-facial acne.

In some embodiments, which may be used or combined with any of the above or below mentioned embodiments, the use results in an improvement in a quality of life assessment.

In some embodiments, which may be used or combined with any of the above or below mentioned embodiments, the use results in an improvement in a Cardiff Acne Disability Index (CADI) score.

In some embodiments, which may be used or combined with any of the above or below mentioned embodiments, the use results in a reduction of sebum production, a reduction in hyperkeratinization, a reduction in colonization by *P. acnes,* or a reduction in release of inflammatory mediators into the skin.

In some embodiments, which may be used or combined with any of the above or below mentioned embodiments, the use is effective to reduce a symptom of skin inflammation including, for example, redness, swelling, leukocyte infiltration, and/or lesion development.

In some embodiments, which may be used or combined with any of the above or below mentioned embodiments, the use is effective to reduce the severity of a condition of the skin associated with acne including, for example, scaling, erythema, itching, burning, and/or stinging.

In some embodiments, which may be used or combined with any of the above or below mentioned embodiments, the use is effective to improve one or more acne parameters including, for example, scaling, erythema, itching, burning, and/or stinging.

In some embodiments, which may be used or combined with any of the above or below mentioned embodiments, the use is effective to reduce the size of acne lesions, redness of acne lesions, and/or itchiness of acne lesions.

In some embodiments, which may be used or combined with any of the above or below mentioned embodiments, the use results in a delay in the recurrence of an acute acne outbreak in the subject.

In some embodiments, which may be used or combined with any of the above or below mentioned embodiments, the use results in a reduction in the severity of a recurrence of an acute acne outbreak in the subject.

In some embodiments, which may be used or combined with any of the above or below mentioned embodiments, the antibiotic is selected from the group consisting of: a penicillin, a polyketide antibiotic, a cephalosporin, a lincosamide, a quinolone, a folic acid synthesis inhibitor, a tetracycline, a rifamycin, a sulfonamide, an aminoglycoside, fusidic acid, a polypeptide antibiotic, a lipopeptide antibiotic, chloramphenicol and mupirocin.

In some embodiments, which may be used or combined with any of the above or below mentioned embodiments, the antibiotic is an oral antibiotic.

In some embodiments, which may be used or combined with any of the above or below mentioned embodiments, the antibiotic is a topical antibiotic.

In some embodiments, which may be used or combined with any of the above or below mentioned embodiments, the penicillin is penicillin, amoxicillin, benzylpenicillin, ampicillin, or augmentin.

In some embodiments, which may be used or combined with any of the above or below mentioned embodiments, the polyketide antibiotic is macrolide, azithromycin, erythromycin, or clarithromycin.

In some embodiments, which may be used or combined with any of the above or below mentioned embodiments, the cephalosporin is cefadroxil, cefixime, or cephalexin.

In some embodiments, which may be used or combined with any of the above or below mentioned embodiments, the lincosamide is clindamycin.

In some embodiments, which may be used or combined with any of the above or below mentioned embodiments, the quinolone is ciprofloxacin, levofloxacin, or moxifloxacin.

In some embodiments, which may be used or combined with any of the above or below mentioned embodiments, the folic acid synthesis inhibitor is a dihydrofolate reductase inhibitor, trimethoprim, dapsone, or co-trimoxazole.

In some embodiments, which may be used or combined with any of the above or below mentioned embodiments, the tetracycline is tetracycline, minocycline, doxycycline, demeclocycline, or oxytetracycline.

In some embodiments, which may be used or combined with any of the above or below mentioned embodiments, the rifamycin is rifampicin, rifabutin, or rifapentine.

In some embodiments, which may be used or combined with any of the above or below mentioned embodiments, the sulfonamide is sulfamethoxazole, or sulfacetamide.

In some embodiments, which may be used or combined with any of the above or below mentioned embodiments, the aminoglycoside is neomycin, amikacin, or tobramycin.

In some embodiments, which may be used or combined with any of the above or below mentioned embodiments, the polypeptide antibiotic is bacitracin, or polymixin B.

In some embodiments, which may be used or combined with any of the above or below mentioned embodiments, the lipopeptide antibiotic is daptomycin.

In some embodiments, which may be used or combined with any of the above or below mentioned embodiments, the acne is associated with *P. acnes* or *S. aureus.*

In some embodiments, which may be used or combined with any of the above or below mentioned embodiments, the anti-IL-1β antibody or binding fragment thereof is administered by subcutaneous, intravenous, intradermal or intramuscular injection.

In some embodiments, which may be used or combined with any of the above or below mentioned embodiments, the anti-IL-1β antibody or binding fragment thereof is administered in a dose of 1 mg/kg or less or a dose less than or equal to 1 mg/kg (*e.g.,* 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg such as 0.2 mg/kg or 0.6 mg/kg). Such administration may be monthly, every two months, every three months, every four months, every five months, every six months, or on recurrance of the acne.

In some embodiments, which may be used or combined with any of the above or below mentioned embodiments, the anti-IL-1β antibody or binding fragment thereof is administered in a dose of less than 100 mg (*e.g.,* 10 mg, 20 mg, 30 mg, 40 mg, 50 mg, 60 mg, 70 mg, 80 mg, or 90 mg). Such administration may be monthly, every two months, every three months, every four months, every five months, every six months, or on recurrance of the acne.

In some embodiments, which may be used or combined with any of the above or below mentioned embodiments, the anti-IL-1β antibody or binding fragment thereof is administered monthly, every two months, every three months, every four months, every five months, every six months, or on recurrance of the acne.

In some embodiments, which may be used or combined with any of the above or below mentioned embodiments, the uses may further comprise administering one or more active agents for the treatment of acne.

In some embodiments, which may be used or combined with any of the above or below mentioned embodiments, the one or more active agents are selected from the group consisting of: benzoyl peroxide, a retinoid, isotretinoin, a corticosteroid, a hormone therapy, UV light, azelaic acid, a topical antibiotic, and an oral antibiotic.

In some embodiments, which may be used or combined with any of the above or below mentioned embodiments, the retinoid is retinol, retinal, tretinoin, isotretinoin, adapalene, or tazarotene.

In some embodiments, which may be used or combined with any of the above or below mentioned embodiments, the corticosteroid is prednisone.

In some embodiments, which may be used or combined with any of the above or below mentioned embodiments, the hormone therapy is an estrogen and/or progestin, a glucocorticoid, or an antiandrogen.

In some embodiments, which may be used or combined with any of the above or below mentioned embodiments, the estrogens and/or progestin is norethindrone acetate-ethinyl estradiol, or norgestimate-ethinyl estradiol.

In some embodiments, which may be used or combined with any of the above or below mentioned embodiments, the glucocorticoid is prednisone.

In some embodiments, which may be used or combined with any of the above or below mentioned embodiments, the antiandrogen is spironolactone or flutamide.

In some embodiments, which may be used or combined with any of the above or below mentioned embodiments, the topical antibiotic is clindamycin, zithromycin, erythromycin, minocycline, or tetracycline.

In some embodiments, which may be used or combined with any of the above or below mentioned embodiments, the oral antibiotic is tetracycline, erythromycin, azithromycin, doxycycline, minocycline, clindamycin, ampicillin, amoxicillin, cephalosporin, or trimethoprim/sulfamethoxazole.

The present disclosure also provides an amount of an anti-IL-1β antibody or binding fragment thereof, wherein the antibody or fragment binds to human IL-1β (e.g.,with a dissociation constant of about 1 pM or less), and wherein the antibody or fragment binds to substantially the same epitope that an antibody comprising a heavy chain variable region of SEQ ID NO: 6 and a light chain variable region of SEQ ID NO: 5 binds to for use in treating acne which is not responsive to treatment with one ore more-microbial agents.

In some embodiments, which may be used or combined with any of the above or below mentioned embodiments, the use further comprise selecting a subject with acne that is not responsive to treatment with one or more anti-microbial agents.

In some embodiments, which may be used or combined with any of the above or below mentioned embodiments, the anti-microbial agent is an antibiotic.

In some embodiments, which may be used or combined with any of the above or below mentioned embodiments, the antibiotic is an oral antibiotic.

In some embodiments, which may be used or combined with any of the above or below mentioned embodiments, the acne is acne vulgaris.

In some embodiments, which may be used or combined with any of the above or below mentioned embodiments, the acne is moderate, severe, or moderate to severe acne vulgaris.

In some embodiments, which may be used or combined with any of the above or below mentioned embodiments, the use results in a reduction (*e.g*., decrease) in inflammatory lesion count including, for example, where prior to treatment the subject had an inflammatory lesion count of at least 20.

In some embodiments, which may be used or combined with any of the above or below mentioned embodiments, the use results in an improvement in facial Investigator's Global Assessment (IGA) grade including, for example, where prior to treatment the subject had an IGA grade of 3 or 4.

In some embodiments, which may be used or combined with any of the above or below mentioned embodiments, the use results in a reduction in total lesion count.

In some embodiments, which may be used or combined with any of the above or below mentioned embodiments, the use results in a reduction in number of facial or non-facial (*e.g.,* back, neck, shoulders, and/or chest) acne lesions in the subject.

In some embodiments, which may be used or combined with any of the above or below mentioned embodiments, the use results in a reduction in severity (*e.g*., size and/or number) of acne lesions in the subject.

In some embodiments, which may be used or combined with any of the above or below mentioned embodiments, the acne includes acne lesions that are inflammatory lesions.

In some embodiments, which may be used or combined with any of the above or below mentioned embodiments, the inflammatory lesions include lesions that are facial lesions.

In some embodiments, which may be used or combined with any of the above or below mentioned embodiments, the inflammatory lesions include lesions that are paples, pustules, or nodules/cysts.

In some embodiments, which may be used or combined with any of the above or below mentioned embodiments, the acne lesions include lesions that are non-inflammatory lesions.

In some embodiments, which may be used or combined with any of the above or below mentioned embodiments, the non-inflammatory acne lesions include lesions that are facial lesions.

In some embodiments, which may be used or combined with any of the above or below mentioned embodiments, the non-inflammatory acne lesions include lesions that are open or closed comedones.

In some embodiments, which may be used or combined with any of the above or below mentioned embodiments, the reduction in number of acne lesions is a reduction in facial acne lesion count (*e.g*., total facial acne lesion count).

In some embodiments, which may be used or combined with any of the above or below mentioned embodiments, the use results in an improvement in Investigator's Global Assessment (IGA) severity grade for facial acne.

In some embodiments, which may be used or combined with any of the above or below mentioned embodiments, the use results in an improvement in Investigator's Global Assessment (IGA) severity grade for non-facial acne.

In some embodiments, which may be used or combined with any of the above or below mentioned embodiments, the use results in an improvement in a quality of life assessment.

In some embodiments, which may be used or combined with any of the above or below mentioned embodiments, the use results in an improvement in a Cardiff Acne Disability Index (CADI) score.

In some embodiments, which may be used or combined with any of the above or below mentioned embodiments, the use results in a reduction of sebum production, a reduction in hyperkeratinization, a reduction in colonization by *P. acnes,* or a reduction in release of inflammatory mediators into the skin.

In some embodiments, which may be used or combined with any of the above or below mentioned embodiments, the use is effective to reduce a symptom of skin inflammation including, for example, redness, swelling, leukocyte infiltration, and/or lesion development.

In some embodiments, which may be used or combined with any of the above or below mentioned embodiments, the use is effective to reduce the severity of a condition of the skin associated with acne including, for example, scaling, erythema, itching, burning, and/or stinging.

In some embodiments, which may be used or combined with any of the above or below mentioned embodiments, the use is effective to improve one or more acne parameters including, for example, scaling, erythema, itching, burning, and/or stinging.

In some embodiments, which may be used or combined with any of the above or below mentioned embodiments, the use is effective to reduce the size of acne lesions, redness of acne lesions, and/or itchiness of acne lesions.

In some embodiments, which may be used or combined with any of the above or below mentioned embodiments, the use results in a delay in the recurrence of an acute acne outbreak in the subject.

In some embodiments, which may be used or combined with any of the above or below mentioned embodiments, the use results in a reduction in the severity of a recurrence of an acute acne outbreak in the subject.

In some embodiments, which may be used or combined with any of the above or below mentioned embodiments, the antibiotic is selected from the group consisting of: a penicillin, a polyketide antibiotic, a cephalosporin, a lincosamide, a quinolone, a folic acid synthesis inhibitor, a tetracycline, a rifamycin, a sulfonamide, an aminoglycoside, fusidic acid, a polypeptide antibiotic, a lipopeptide antibiotic, chloramphenicol and mupirocin.

In some embodiments, which may be used or combined with any of the above or below mentioned embodiments, the antibiotic is an oral antibiotic.

In some embodiments, which may be used or combined with any of the above or below mentioned embodiments, the antibiotic is a topical antibiotic.

In some embodiments, which may be used or combined with any of the above or below mentioned embodiments, the penicillin is penicillin, amoxicillin, benzylpenicillin, ampicillin, or augmentin.

In some embodiments, which may be used or combined with any of the above or below mentioned embodiments, the polyketide antibiotic is macrolide, azithromycin, erythromycin, or clarithromycin.

In some embodiments, which may be used or combined with any of the above or below mentioned embodiments, the cephalosporin is cefadroxil, cefixime, or cephalexin.

In some embodiments, which may be used or combined with any of the above or below mentioned embodiments, the lincosamide is clindamycin.

In some embodiments, which may be used or combined with any of the above or below mentioned embodiments, the quinolone is ciprofloxacin, levofloxacin, or moxifloxacin.

In some embodiments, which may be used or combined with any of the above or below mentioned embodiments, the folic acid synthesis inhibitor is a dihydrofolate reductase inhibitor, trimethoprim, dapsone, or co-trimoxazole.

In some embodiments, which may be used or combined with any of the above or below mentioned embodiments, the tetracycline is tetracycline, minocycline, doxycycline, demeclocycline, or oxytetracycline.

In some embodiments, which may be used or combined with any of the above or below mentioned embodiments, the rifamycin is rifampicin, rifabutin, or rifapentine.

In some embodiments, which may be used or combined with any of the above or below mentioned embodiments, the sulfonamide is sulfamethoxazole, or sulfacetamide.

In some embodiments, which may be used or combined with any of the above or below mentioned embodiments, the aminoglycoside is neomycin, amikacin, or tobramycin.

In some embodiments, which may be used or combined with any of the above or below mentioned embodiments, the polypeptide antibiotic is bacitracin, or polymixin B.

In some embodiments, which may be used or combined with any of the above or below mentioned embodiments, the lipopeptide antibiotic is daptomycin.

In some embodiments, which may be used or combined with any of the above or below mentioned embodiments, the acne is associated with *P. acnes* or *S. aureus.*

In some embodiments, which may be used or combined with any of the above or below mentioned embodiments, one or more active agents used and/or approved for the treatment of acne are administered in conjunction with the anti-IL-1β antibody or binding fragment thereof.

In some embodiments, which may be used or combined with any of the above or below mentioned embodiments, the one or more active agents used and/or approved for the treatment of acne are administered as a topical treatment, including, for example, benzoyl peroxide, topical retinoids (*e.g.,* tretinoin, adapalene, isotretinoin, tazarotene), and/or topical antibiotics (*e.g.,* clindamycin, erythromycin, tetracycline). Such topical treatment may include other topical therapies *(e.g.,* salicylic acid, sulphur, resorcinol, sodium sulfacetamide, aluminium chloride, zinc, nicotinamide, triethyl citrate/ethyl linoleate, dapsone, taurine bromamine, azelaic acid, sodium sulfacetamide 10%/sulfur 5%) as well as combination topicals *(e.g.,* combinations of topical treatments with different mechanisms of action).

In some embodiments, which may be used or combined with any of the above or below mentioned embodiments, the one or more active agents used and/or approved for the treatment of acne are administered as an oral treatment, including, for example, oral antibiotics (*e.g.,* tetracyclines such as tetracycline, oxytetracycline, doxycycline, lymecycline or less preferably minocycline; cotrimoxazole; quinolones such as ciprofloxacin; aminoglycosides; chloramphenicol; clindamycin; macrolides such as erythromycin and azitromycin; trimethoprim), oral contraceptives (*e.g.,* combined oral contraceptives that contain an estrogen such as ethinylestradiol and a progestogen; progestogen; estrogen), and/or oral isotretinoin (*e.g*., ACCUTANE™ ).

In some embodiments, which may be used or combined with any of the above or below mentioned embodiments, the one or more active agents used and/or approved for the treatment of acne are administered as a combination treatment, including, for example, retinoid +/- benzoyl peroxide +/- topical antibiotic; retinoid +/- topical antibiotic or benzoyl peroxide; sulfacetamide/sulfur or topical antibiotic + benzoyl peroxide +/- oral antibiotic(s); sulfacetamide/sulfur or topical antibiotic + benzoyl peroxide +/- oral antibiotic(s) + retinoid.

In some embodiments, which may be used or combined with any of the above or below mentioned embodiments, the one or more active agents are selected from the group consisting of: benzoyl peroxide, retinol, retinal, tretinoin, isotretinoin, adapalene, isotretinoin, prednisone, tretinoin, clindamycin, zithromycin, erythromycin, minocycline, and tetracycline.

In some embodiments, which may be used or combined with any of the above or below mentioned embodiments, the anti-IL-1β antibody or binding fragment thereof is a neutralizing antibody.

In some embodiments, which may be used or combined with any of the above or below mentioned embodiments, the anti-IL-1β antibody or binding fragment thereof binds to an IL-1β epitope such that the bound antibody or fragment substantially permits the binding of IL-1β to IL-1 receptor I (IL-1RI).

In some embodiments, which may be used or combined with any of the above or below mentioned embodiments, the antibody or binding fragment thereof does not detectably bind to IL-1α, IL-1R or IL-1Ra.

In some embodiments, which may be used or combined with any of the above or below mentioned embodiments, the anti-IL-1β antibody or binding fragment thereof comprises a heavy chain variable region comprising: a heavy chain complementarity determining region 1 (HCDR1) comprising TSGMGVG (SEQ ID NO: 13), a heavy chain complementarity determining region 2 (HCDR2) comprising HIWWDGDESYNPSLK (SEQ ID NO: 14), and/or a heavy chain complementarity determining region 3 (HCDR3) comprising NRYDPPWFVD (SEQ ID NO: 15); and/or a light chain variable region comprising: a light chain complementarity determining region 1 (LCDR1) comprising RASQDISNYLS (SEQ ID NO: 16), a light chain complementarity determining region 2 (LCDR2) comprising YTSKLHS (SEQ ID NO: 17), and/or a light chain complementarity determining region 3 (LCDR3) comprising LQGKMLPWT (SEQ ID NO: 18).

In some embodiments, which may be used or combined with any of the above or below mentioned embodiments, the antibody or binding fragment thereof is human or humanized.

In some embodiments, which may be used or combined with any of the above or below mentioned embodiments, the anti-IL-1β antibody or binding fragment thereof is administered by subcutaneous, intravenous, intradermal or intramuscular injection.

In some embodiments, which may be used or combined with any of the above or below mentioned embodiments, the anti-IL-1β antibody or binding fragment thereof is administered in a dose of 1 mg/kg or less or a dose less than or equal to 1 mg/kg (*e.g.,* 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg such as 0.2 mg/kg or 0.6 mg/kg). Such administration may be monthly, every two months, every three months, every four months, every five months, every six months, or on recurrance of the acne.

In some embodiments, which may be used or combined with any of the above or below mentioned embodiments, the anti-IL-1β antibody or binding fragment thereof is administered in a dose of less than 100 mg (*e.g.,* 10 mg, 20 mg, 30 mg, 40 mg, 50 mg, 60 mg, 70 mg, 80 mg, or 90 mg). Such administration may be monthly, every two months, every three months, every four months, every five months, every six months, or on recurrance of the acne.

. In some embodiments, which may be used or combined with any of the above or below mentioned embodiments, the anti-IL-1β antibody or binding fragment thereof is administered monthly, every two months, every three months, every four months, every five months, every six months, or on recurrance of the acne.

In some embodiments, which may be used or combined with any of the above or below mentioned embodiments, the uses may further comprise administering one or more active agents for the treatment of acne.

In some embodiments, which may be used or combined with any of the above or below mentioned embodiments, the one or more active agents are selected from the group consisting of: benzoyl peroxide, a retinoid, isotretinoin, a corticosteroid, a hormone therapy, UV light, azelaic acid, a topical antibiotic, and an oral antibiotic.

In some embodiments, which may be used or combined with any of the above or below mentioned embodiments, the retinoid is retinol, retinal, tretinoin, isotretinoin, adapalene, or tazarotene.

In some embodiments, which may be used or combined with any of the above or below mentioned embodiments, the corticosteroid is prednisone.

In some embodiments, which may be used or combined with any of the above or below mentioned embodiments, the hormone therapy is an estrogen and/or progestin, a glucocorticoid, or an antiandrogen.

In some embodiments, which may be used or combined with any of the above or below mentioned embodiments, the estrogens and/or progestin is norethindrone acetate-ethinyl estradiol, or norgestimate-ethinyl estradiol.

In some embodiments, which may be used or combined with any of the above or below mentioned embodiments, the glucocorticoid is prednisone.

In some embodiments, which may be used or combined with any of the above or below mentioned embodiments, the antiandrogen is spironolactone or flutamide.

In some embodiments, which may be used or combined with any of the above or below mentioned embodiments, the topical antibiotic is clindamycin, zithromycin, erythromycin, minocycline, or tetracycline.

In some embodiments, which may be used or combined with any of the above or below mentioned embodiments, the oral antibiotic is tetracycline, erythromycin, azithromycin, doxycycline, minocycline, clindamycin, ampicillin, amoxicillin, cephalosporin, or trimethoprim/sulfamethoxazole.

The present disclosure also provides an amount of an anti-IL-1β antibody or binding fragment thereof, wherein the antibody or fragment thereof binds to human IL-1β (*e.g.,* with a dissociation constant of about 1 pM or less), and wherein the antibody or fragment competes with the binding of an antibody having the light chain variable region of SEQ ID NO: 5 and the heavy chain variable region of SEQ ID NO: 6 for use in treating acne which is not responsive to treatment with one ore more-microbial agents.

In some embodiments, which may be used or combined with any of the above or below mentioned embodiments, the uses further comprise selecting a subject with acne that is not responsive to treatment with one or more anti-microbial agents.

In some embodiments, which may be used or combined with any of the above or below mentioned embodiments, the anti-microbial agent is an antibiotic.

In some embodiments, which may be used or combined with any of the above or below mentioned embodiments, the antibiotic is an oral antibiotic.

In some embodiments, which may be used or combined with any of the above or below mentioned embodiments, the acne is acne vulgaris.

In some embodiments, which may be used or combined with any of the above or below mentioned embodiments, the acne is moderate, severe, or moderate to severe acne vulgaris.

In some embodiments, which may be used or combined with any of the above or below mentioned embodiments, the use results in a reduction (*e.g*., decrease) in inflammatory lesion count including, for example, where prior to treatment the subject had an inflammatory lesion count of at least 20.

In some embodiments, which may be used or combined with any of the above or below mentioned embodiments, the use results in an improvement in facial Investigator's Global Assessment (IGA) grade including, for example, where prior to treatment the subject had an IGA grade of 3 or 4.

In some embodiments, which may be used or combined with any of the above or below mentioned embodiments, the use results in a reduction in total lesion count.

In some embodiments, which may be used or combined with any of the above or below mentioned embodiments, the use results in a reduction in number of facial or non-facial (*e.g.,* back, neck, shoulders, and/or chest) acne lesions in the subject.

In some embodiments, which may be used or combined with any of the above or below mentioned embodiments, the use results in a reduction in severity (*e.g*., size and/or number) of acne lesions in the subject.

In some embodiments, which may be used or combined with any of the above or below mentioned embodiments, the acne includes acne lesions that are inflammatory lesions.

In some embodiments, which may be used or combined with any of the above or below mentioned embodiments, the inflammatory lesions include lesions that are facial lesions.

In some embodiments, which may be used or combined with any of the above or below mentioned embodiments, the inflammatory lesions include lesions that are paples, pustules, or nodules/cysts.

In some embodiments, which may be used or combined with any of the above or below mentioned embodiments, the acne lesions include lesions that are non-inflammatory lesions.

In some embodiments, which may be used or combined with any of the above or below mentioned embodiments, the non-inflammatory acne lesions include lesions that are facial lesions.

In some embodiments, which may be used or combined with any of the above or below mentioned embodiments, the non-inflammatory acne lesions include lesions that are open or closed comedones.

In some embodiments, which may be used or combined with any of the above or below mentioned embodiments, the reduction in number of acne lesions is a reduction in facial acne lesion count (*e.g.,* total facial acne lesion count).

In some embodiments, which may be used or combined with any of the above or below mentioned embodiments, the use results in an improvement in Investigator's Global Assessment (IGA) severity grade for facial acne.

In some embodiments, which may be used or combined with any of the above or below mentioned embodiments, the use results in an improvement in Investigator's Global Assessment (IGA) severity grade for non-facial acne.

In some embodiments, which may be used or combined with any of the above or below mentioned embodiments, the use results in an improvement in a quality of life assessment.

In some embodiments, which may be used or combined with any of the above or below mentioned embodiments, the use results in an improvement in a Cardiff Acne Disability Index (CADI) score.

In some embodiments, which may be used or combined with any of the above or below mentioned embodiments, the use results in a reduction of sebum production, a reduction in hyperkeratinization, a reduction in colonization by *P. acnes,* or a reduction in release of inflammatory mediators into the skin.

In some embodiments, which may be used or combined with any of the above or below mentioned embodiments, the use is effective to reduce a symptom of skin inflammation including, for example, redness, swelling, leukocyte infiltration, and/or lesion development.

In some embodiments, which may be used or combined with any of the above or below mentioned embodiments, the use is effective to reduce the severity of a condition of the skin associated with acne including, for example, scaling, erythema, itching, burning, and/or stinging.

In some embodiments, which may be used or combined with any of the above or below mentioned embodiments, the use is effective to improve one or more acne parameters including, for example, scaling, erythema, itching, burning, and/or stinging.

In some embodiments, which may be used or combined with any of the above or below mentioned embodiments, the use is effective to reduce the size of acne lesions, redness of acne lesions, and/or itchiness of acne lesions.

In some embodiments, which may be used or combined with any of the above or below mentioned embodiments, the use results in a delay in the recurrence of an acute acne outbreak in the subject.

In some embodiments, which may be used or combined with any of the above or below mentioned embodiments, the use results in a reduction in the severity of a recurrence of an acute acne outbreak in the subject.

In some embodiments, which may be used or combined with any of the above or below mentioned embodiments, the antibiotic is selected from the group consisting of: a penicillin, a polyketide antibiotic, a cephalosporin, a lincosamide, a quinolone, a folic acid synthesis inhibitor, a tetracycline, a rifamycin, a sulfonamide, an aminoglycoside, fusidic acid, a polypeptide antibiotic, a lipopeptide antibiotic, chloramphenicol and mupirocin.

In some embodiments, which may be used or combined with any of the above or below mentioned embodiments, the antibiotic is an oral antibiotic.

In some embodiments, which may be used or combined with any of the above or below mentioned embodiments, the antibiotic is a topical antibiotic.

In some embodiments, which may be used or combined with any of the above or below mentioned embodiments, the anti-IL-1β antibody or binding fragment thereof is a neutralizing antibody.

In some embodiments, which may be used or combined with any of the above or below mentioned embodiments, the anti-IL-1β antibody or binding fragment thereof binds to an IL-1β epitope such that the bound antibody or binding fragment substantially permits the binding of IL-1β to IL-1 receptor I (IL-1RI).

In some embodiments, which may be used or combined with any of the above or below mentioned embodiments, the antibody or binding fragment thereof does not detectably bind to IL-1α, IL-1R or IL-1Ra.

In some embodiments, which may be used or combined with any of the above or below mentioned embodiments, the anti-IL-1β antibody or binding fragment thereof comprises a heavy chain variable region comprising: a heavy chain complementarity determining region 1 (HCDR1) comprising TSGMGVG (SEQ ID NO: 13), a heavy chain complementarity determining region 2 (HCDR2) comprising HIWWDGDESYNPSLK (SEQ ID NO: 14), and/or a heavy chain complementarity determining region 3 (HCDR3) comprising NRYDPPWFVD (SEQ ID NO: 15); and/or a light chain variable region comprising: a light chain complementarity determining region 1 (LCDR1) comprising RASQDISNYLS (SEQ ID NO: 16), a light chain complementarity determining region 2 (LCDR2) comprising YTSKLHS (SEQ ID NO: 17), and/or a light chain complementarity determining region 3 (LCDR3) comprising LQGKMLPWT (SEQ ID NO: 18)

In some embodiments, which may be used or combined with any of the above or below mentioned embodiments, the antibody or binding fragment thereof is human or humanized.

In some embodiments, which may be used or combined with any of the above or below mentioned embodiments, the anti-IL-1β antibody or binding fragment thereof is administered by subcutaneous, intravenous, intradermal or intramuscular injection.

In some embodiments, which may be used or combined with any of the above or below mentioned embodiments, the anti-IL-1β antibody or binding fragment thereof is administered in a dose of 1 mg/kg or less or a dose less than or equal to 1 mg/kg (*e.g.,* 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg such as 0.2 mg/kg or 0.6 mg/kg). Such administration may be monthly, every two months, every three months, every four months, every five months, every six months, or on recurrance of the acne.

In some embodiments, which may be used or combined with any of the above or below mentioned embodiments, the anti-IL-1β antibody or binding fragment thereof is administered in a dose of less than 100 mg (*e.g.,* 10 mg, 20 mg, 30 mg, 40 mg, 50 mg, 60 mg, 70 mg, 80 mg, or 90 mg). Such administration may be monthly, every two months, every three months, every four months, every five months, every six months, or on recurrance of the acne.

. In some embodiments, which may be used or combined with any of the above or below mentioned embodiments, the anti-IL-1β antibody or binding fragment thereof is administered monthly, every two months, every three months, every four months, every five months, every six months, or on recurrance of the acne.

In some embodiments, which may be used or combined with any of the above or below mentioned embodiments, the uses may further comprise administering one or more active agents for the treatment of acne.

In some embodiments, which may be used or combined with any of the above or below mentioned embodiments, the one or more active agents are selected from the group consisting of: benzoyl peroxide, a retinoid, isotretinoin, a corticosteroid, a hormone therapy, UV light, azelaic acid, a topical antibiotic, and an oral antibiotic.

In some embodiments, which may be used or combined with any of the above or below mentioned embodiments, the retinoid is retinol, retinal, tretinoin, isotretinoin, adapalene, or tazarotene.

In some embodiments, which may be used or combined with any of the above or below mentioned embodiments, the corticosteroid is prednisone.

In some embodiments, which may be used or combined with any of the above or below mentioned embodiments, the hormone therapy is an estrogen and/or progestin, a glucocorticoid, or an antiandrogen.

In some embodiments, which may be used or combined with any of the above or below mentioned embodiments, the estrogens and/or progestin is norethindrone acetate-ethinyl estradiol, or norgestimate-ethinyl estradiol.

In some embodiments, which may be used or combined with any of the above or below mentioned embodiments, the glucocorticoid is prednisone.

In some embodiments, which may be used or combined with any of the above or below mentioned embodiments, the antiandrogen is spironolactone or flutamide.

In some embodiments, which may be used or combined with any of the above or below mentioned embodiments, the topical antibiotic is clindamycin, zithromycin, erythromycin, minocycline, or tetracycline.

In some embodiments, which may be used or combined with any of the above or below mentioned embodiments, the oral antibiotic is tetracycline, erythromycin, azithromycin, doxycycline, minocycline, clindamycin, ampicillin, amoxicillin, cephalosporin, or trimethoprim/sulfamethoxazole.

It is to be understood that where the present specification mentions methods of treatments making use of antibodies or binding fragments thereof with certain properties (such as Kd values or IC50 values), this also means to embody the use of such antibodies or fragments thereof in the manufacture of a medicament for use in these methods. Further, the invention also encompasses antibodies or binding fragments thereof having these properties as well as pharmaceutical compositions comprising these antibodies or binding fragments thereof for use in the methods of treatment discussed hereinafter.

### DETAILED DESCRIPTION

Effective therapies for use in treating acne have remained an important medical need. The present disclosure provides materials, and related articles of manufacture, and their use for treating acne, which is not responsive to one or more antimicrobial agents such as antibiotics, including topical or systemic (*e.g.,* oral) antibiotics. Such acne may include acne presented on facial and/or non-facial skin (*e.g.,* skin of the neck, back, shoulders, and/or chest). Such uses may include selecting a subject with acne that exhibits moderate or severe acne and is not responsive to to one or more antimicrobial agents (*e.g.,* antibiotics), and administering to the subject an effective amount of anti-IL-1β antibody or binding fragment thereof. The anti-IL-1β antibodies or binding fragments thereof disclosed herein are advantageous for use in that they bind IL-1β and do not cross react with IL-1α and/or IL-1Ra. Additionally, the anti-IL-1β antibodies or binding fragments thereof disclosed herein are advantageous for use because they provide an unexpected therapeutic benefit (*e.g.,* a reduction of a clinical symptom or manifestation of acne) to be achieved in a shorter time and/or maintained over a longer time after administration than conventional therapy used to treat acne. The disclosed materials and uses may be used to replace or complement other pharmaceutical approaches as provided herein.

The present disclosure provides uses of treating acne (*e.g.,* moderate to severe acne vulgaris) in a subject, comprising: administering to the subject an amount of an anti-IL-1β antibody or binding fragment thereof including, for example, wherein (i) the acne is moderate and/or severe acne, and/or (ii) the acne is not responsive to conventional therapy (*e.g.,* not responsive to one or more antimicrobial agents such as antibiotics, including topical or systemic (*e.g.,* oral) antibiotics).

The present disclosure also an amount of an anti-IL-1β antibody or binding fragment thereof for use in treating acne that is not responsiveto treatment with one or more anti-microbial agents (*e.g.,* not responsive to one or more antimicrobial agents such as antibiotics, including topical or systemic (*e.g.,* oral) antibiotics), wherein the anti-IL-1β antibody or binding fragment thereof comprises a heavy chain variable region comprising: a heavy chain complementarity determining region 1 (HCDR1) comprising TSGMGVG (SEQ ID NO: 13), a heavy chain complementarity determining region 2 (HCDR2) comprising HIWWDGDESYNPSLK (SEQ ID NO: 14), and/or a heavy chain complementarity determining region 3 (HCDR3) comprising NRYDPPWFVD (SEQ ID NO: 15); and/or a light chain variable region comprising: a light chain complementarity determining region 1 (LCDR1) comprising RASQDISNYLS (SEQ ID NO: 16), a light chain complementarity determining region 2 (LCDR2) comprising YTSKLHS (SEQ ID NO: 17), and/or a light chain complementarity determining region 3 (LCDR3) comprising LQGKMLPWT (SEQ ID NO: 18), including, for example, wherein the acne is moderate and/or severe acne (*e.g.,* not responsive to one or more antimicrobial agents such as antibiotics, including topical or systemic (*e.g.,* oral) antibiotics).

The present disclosure also provides an amount (*e.g.,* a therapeutically effective amount) of an anti-IL-1β antibody or binding fragment thereof (*e.g.,* an antibody comprising a heavy chain variable region of SEQ ID NO: 6 and a light chain variable region of SEQ ID NO: 5) for use in treating acne (*e.g.,* moderate to severe acne vulgaris) that is not responsive to treatment with one or more anti-microbial agents (*e.g.,* not responsive to one or more antimicrobial agents such as antibiotics, including topical or systemic (*e.g.,* oral) antibiotics), including, for example, wherein the acne is moderate and/or severe acne.

The present disclosure also provides an amount (*e.g.,* a therapeutically effective amount) of an anti-IL-1β antibody or binding fragment thereof for use in treating acne (*e.g.,* moderate to severe acne vulgaris) that is not responsiveto treatment with one or more anti-microbial agents (*e.g.,* not responsive to one or more antimicrobial agents such as antibiotics, including topical or systemic (*e.g.,* oral) antibiotics), wherein the antibody or fragment thereof competes with the binding of an antibody having the light chain variable region of SEQ ID NO: 5 and the heavy chain variable region of SEQ ID NO: 6, including, for example, wherein the acne is moderate and/or severe acne.

The present disclosure also provides an amount (*e.g.,* a therapeutically effective amount) of an anti-IL-1β antibody or binding fragment thereof for use in treating acne (*e.g.,* moderate to severe acne vulgaris) that is not responsiveto treatment with one or more anti-microbial agents (*e.g.,* not responsive to one or more antimicrobial agents such as antibiotics, including topical or systemic (*e.g.,* oral) antibiotics), wherein the antibody or fragment binds to human IL-1β (*e.g.,* with a dissociation constant of about 1 pM or less), and wherein the antibody or fragment binds to substantially the same (*e.g.,* the same) epitope that an antibody comprising a heavy chain variable region of SEQ ID NO: 6 and a light chain variable region of SEQ ID NO: 5 binds to, including, for example, wherein the acne is moderate and/or severe acne).

The present disclosure also provides an amount (*e.g.,* a therapeutically effective amount) of an anti-IL-1β antibody or binding fragment thereof for use in treating acne (*e.g.,* moderate to severe acne vulgaris) that is not responsiveto treatment with one or more anti-microbial agents (*e.g.,* not responsive to one or more antimicrobial agents such as antibiotics, including topical or systemic (*e.g.,* oral) antibiotics), wherein the antibody or fragment binds to human IL-1β (*e.g.,* with a dissociation constant of about 1 pM or less), and wherein the antibody or fragment thereof competes with the binding of an antibody having the light chain variable region of SEQ ID NO: 5 and the heavy chain variable region of SEQ ID NO: 6, including, for example, wherein the acne is moderate and/or severe acne.

The present disclosure also provides uses of treating a subject with acne (*e.g.,* moderate to severe acne vulgaris) by scoring the acne at a first time; administering to the subject a conventional therapy (*e.g.,* an anti-microbial agent); scoring the acne at a second time; determining that the subject is not responsive to treatment with the conventional therapy where there is not an improvement in a score assessed to the acne between the scoring at the first time and the scoring at the second time; and administering to the subject an amount (*e.g.,* a therapeutically effective amount) of anti-IL-1β antibody or binding fragment thereof (*e.g.,* an antibody comprising a heavy chain variable region of SEQ ID NO: 6 and a light chain variable region of SEQ ID NO: 5).

### Antibodies, Humanized Antibodies, and Human Engineered Antibodies

The IL-1β binding antibodies of the present disclosure may be provided as polyclonal antibodies, monoclonal antibodies (mAbs), recombinant antibodies, chimeric antibodies, CDR-grafted antibodies, fully human antibodies, single chain antibodies, and/or bispecific antibodies, as well as fragments, including variants and derivatives thereof, provided by known techniques, including, but not limited to enzymatic cleavage, peptide synthesis or recombinant techniques.

Antibodies generally comprise two heavy chain polypeptides and two light chain polypeptides, though single domain antibodies having one heavy chain and one light chain, and heavy chain antibodies devoid of light chains are also contemplated. There are five types of heavy chains, called alpha, delta, epsilon, gamma and mu, based on the amino acid sequence of the heavy chain constant domain. These different types of heavy chains give rise to five classes of antibodies, IgA (including IgA1 and IgA2), IgD, IgE, IgG and IgM, respectively, including four subclasses of IgG, namely IgG1, IgG2, IgG3 and IgG4. There are also two types of light chains, called kappa (κ) or lambda (λ) based on the amino acid sequence of the constant domains. A full-length antibody includes a constant domain and a variable domain. The constant region need not be present in an antigen binding fragment of an antibody. Antigen binding fragments of an antibody disclosed herein can include Fab, Fab', F(ab')2, and F(v) antibody fragments. As discussed in more detail below, IL-1β binding fragments encompass antibody fragments and antigen-binding polypeptides that will bind IL-1β.

Each of the heavy chain and light chain sequences of an antibody, or antigen binding fragment thereof, includes a variable region with three complementarity determining regions (CDRs) as well as non-CDR framework regions (FRs). The terms "heavy chain" and "light chain," as used herein, mean the heavy chain variable region and the light chain variable region, respectively, unless otherwise noted. Heavy chain CDRs are referred to herein as CDR-H1, CDR-H2, and CDR-H3. Light chain CDRs are referred to herein as CDR-L1, CDR-L2, and CDR-L3. Variable regions and CDRs in an antibody sequence can be identified (i) according to general rules that have been developed in the art or (ii) by aligning the sequences against a database of known variable regions. Methods for identifying these regions are described in Kontermann and Dubel, eds., Antibody Engineering, Springer, New York, NY, 2001, and Dinarello et al., Current Protocols in Immunology, John Wiley and Sons Inc., Hoboken, NJ, 2000. Databases of antibody sequences are described in and can be accessed through "The Kabatman" database at www.bioinf.org.uk/abs (maintained by A.C. Martin in the Department of Biochemistry & Molecular Biology University College London, London, England) and VBASE2 at www.vbase2.org, as described in Retter et al., Nucl. Acids Res., 33(Database issue): D671-D674 (2005). The "Kabatman" database web site also includes general rules of thumb for identifying CDRs.

Polyclonal antibodies are preferably raised in animals by multiple subcutaneous (sc) or intraperitoneal (ip) injections of the relevant antigen and an adjuvant. An improved antibody response may be obtained by conjugating the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride or other agents known in the art.

Animals are immunized against the antigen, immunogenic conjugates, or derivatives by combining, e.g., 100 µg or 5 µg of the protein or conjugate (for rabbits or mice, respectively) with 3 volumes of Freund's complete adjuvant and injecting the solution intradermally at multiple sites. One month later, the animals are boosted with 1/5 to {fraction (1/10)} the original amount of peptide or conjugate in Freund's complete adjuvant by subcutaneous injection at multiple sites. At 7-14 days post-booster injection, the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

Monoclonal antibody refers to an antibody obtained from a population of substantially homogeneous antibodies. Monoclonal antibodies are generally highly specific, and may be directed against a single antigenic site, in contrast to conventional (polyclonal) antibody preparations that typically include different antibodies directed against different determinants (epitopes). In addition to their specificity, the monoclonal antibodies are advantageous in that they are synthesized by the homogeneous culture, uncontaminated by other immunoglobulins with different specificities and characteristics.

Monoclonal antibodies may be made by the hybridoma method first described by Kohler et al., (Nature, 256:495-7, 1975), or may be made by recombinant DNA methods (see, *e.g.,* U.S. Patent No. 4,816,567). The monoclonal antibodies may also be isolated from display libraries (*e.g.,* yeast libraries, phage antibody libraries) using the techniques described in, for example, Clackson et al., (Nature 352:624-628, 1991), Marks et al., (J. Mol. Biol. 222:581-597, 1991) Hoogenboom (Nat Biotechnol. 23:1105-16, 2005) and Mondon et al., (Front Biosci., 13:1117-1129, 2008).

In the hybridoma method, a mouse or other appropriate host animal, such as a hamster or macaque monkey, is immunized as herein described to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp. 59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium. Human myeloma and mouse-human heteromyeloma cell lines also have been described for the production of human monoclonal antibodies (Kozbor, J. Immunol., 133: 3001 (1984); Brodeur et al., Monoclonal Antibody Production Techniques and Applications, pp. 51-63 (Marcel Dekker, Inc., New York, 1987)). Exemplary murine myeloma lines include those derived from MOP-21 and M.C.-11 mouse tumors available from the Salk Institute Cell Distribution Center, San Diego, Calif. USA, and SP-2 or X63-Ag8-653 cells available from the American Type Culture Collection, Rockville, Md. USA.

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA). The binding affinity of the monoclonal antibody can, for example, be determined by Scatchard analysis (Munson et al., Anal. Biochem., 107:220 (1980)) or using a KINEXA™ device (from Sapidyne Instruments Inc., Boise, ID).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp. 59-103 (Academic Press, 1986)). Suitable culture media for this purpose include, for example, DMEM or RPMI-1640 medium. In addition, the hybridoma cells may be grown in vivo as ascites tumors in an animal. The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

It is further contemplated that antibodies may be used as smaller antigen binding fragments of the antibody well-known in the art and described herein.

The present disclosure encompasses IL-1β binding antibodies that include two full length heavy chains and two full length light chains. Alternatively, the anti-IL-1β antibodies can be constructs such as single chain antibodies or "mini" antibodies that retain binding activity to IL-1β. Such constructs can be prepared by methods known in the art such as, for example, the PCR mediated cloning and assembly of single chain antibodies for expression in E. coli (as described in Antibody Engineering, The practical approach series, J. McCafferty, H. R. Hoogenboom, and D. J. Chiswell, editors, Oxford University Press, 1996). In this type of construct, the variable portions of the heavy and light chains of an antibody molecule are PCR amplified from cDNA. The resulting amplicons are then assembled, for example, in a second PCR step, through a linker DNA that encodes a flexible protein linker composed of the amino acids Gly and Ser. This linker allows the variable heavy and light chain portions to fold in such a way that the antigen binding pocket is regenerated and antigen is bound with affinities often comparable to the parent full-length dimeric immunoglobulin molecule.

The IL-1β binding antibodies and binding fragments of the present disclosure encompass variants of the exemplary antibodies, fragments and sequences disclosed herein. Variants include peptides and polypeptides comprising one or more amino acid sequence substitutions, deletions, and/or additions that have the same or substantially the same affinity and specificity of epitope binding as one or more of the exemplary antibodies, fragments and sequences disclosed herein. Thus, variants include peptides and polypeptides comprising one or more amino acid sequence substitutions, deletions, and/or additions to the exemplary antibodies, fragments and sequences disclosed herein where such substitutions, deletions and/or additions do not cause substantial changes in affinity and specificity of epitope binding. For example, a variant of an antibody or fragment may result from one or more changes to an antibody or fragment, where the changed antibody or fragment has the same or substantially the same affinity and specificity of epitope binding as the starting sequence. Variants may be naturally occurring, such as allelic or splice variants, or may be artificially constructed. Variants may be prepared from the corresponding nucleic acid molecules encoding said variants. Variants of the present antibodies and anti-IL-1β binding fragments may have changes in light and/or heavy chain amino acid sequences that are naturally occurring or are introduced by in vitro engineering of native sequences using recombinant DNA techniques. Naturally occurring variants include "somatic" variants which are generated in vivo in the corresponding germ line nucleotide sequences during the generation of an antibody response to a foreign antigen.

Variants of IL-1β binding antibodies and binding fragments may also be prepared by mutagenesis techniques. For example, amino acid changes may be introduced at random throughout an antibody coding region and the resulting variants may be screened for binding affinity for IL-1β or for another property. Alternatively, amino acid changes may be introduced in selected regions of an IL-1β antibody, such as in the light and/or heavy chain CDRs, and/or in the framework regions, and the resulting antibodies may be screened for binding to IL-1β or some other activity. Amino acid changes encompass one or more amino acid substitutions in a CDR, ranging from a single amino acid difference to the introduction of multiple permutations of amino acids within a given CDR, such as CDR3. In another method, the contribution of each residue within a CDR to IL-1β binding may be assessed by substituting at least one residue within the CDR with alanine. Lewis et al. (1995), Mol. Immunol. 32: 1065-72. Residues which are not optimal for binding to IL-1β may then be changed in order to determine a more optimum sequence. Also encompassed are variants generated by insertion of amino acids to increase the size of a CDR, such as CDR3. For example, most light chain CDR3 sequences are nine amino acids in length. Light chain sequences in an antibody which are shorter than nine residues may be optimized for binding to IL-1 β by insertion of appropriate amino acids to increase the length of the CDR.

Variants may also be prepared by "chain shuffling" of light or heavy chains. Marks et al. (1992), Biotechnology 10: 779-83. A single light (or heavy) chain can be combined with a library having a repertoire of heavy (or light) chains and the resulting population is screened for a desired activity, such as binding to IL-1β. This permits screening of a greater sample of different heavy (or light) chains in combination with a single light (or heavy) chain than is possible with libraries comprising repertoires of both heavy and light chains.

The IL-1β binding antibodies and binding fragments of the present disclosure encompass derivatives of the exemplary antibodies, fragments and sequences disclosed herein. Derivatives include polypeptides or peptides, or variants, fragments or derivatives thereof, which have been chemically modified. Examples include covalent attachment of one or more polymers, such as water soluble polymers, N-linked, or O-linked carbohydrates, sugars, phosphates, and/or other such molecules. The derivatives are modified in a manner that is different from naturally occurring or starting peptide or polypeptides, either in the type or location of the molecules attached. Derivatives further include deletion of one or more chemical groups which are naturally present on the peptide or polypeptide.

The anti-IL-1β antibodies and binding fragments thereof can be bispecific. Bispecific antibodies or fragments can be of several configurations. For example, bispecific antibodies may resemble single antibodies (or antibody fragments) but have two different antigen binding sites (variable regions). Bispecific antibodies can be produced by chemical techniques (Kranz et al. (1981), Proc. Natl. Acad. Sci. USA, 78: 5807), by "polydoma" techniques (U.S. Pat. No. 4,474,893) or by recombinant DNA techniques. Bispecific antibodies can have binding specificities for at least two different epitopes, at least one of which is an epitope of IL-1β The anti-IL-1β antibodies and binding fragments thereof can also be heteroantibodies. Heteroantibodies are two or more antibodies, or antibody binding fragments (Fab) linked together, each antibody or fragment having a different specificity.

Techniques for creating recombinant DNA versions of the antigen-binding regions of antibody molecules which bypass the generation of monoclonal antibodies are contemplated for the present IL-1β binding antibodies and binding fragments. DNA is cloned into a bacterial expression system. One example of such a technique suitable for the practice of this invention uses a bacteriophage lambda vector system having a leader sequence that causes the expressed Fab protein to migrate to the periplasmic space (between the bacterial cell membrane and the cell wall) or to be secreted. One can rapidly generate and screen great numbers of functional Fab fragments for those which bind IL-1β. Such anti-IL-1β binding agents (Fab fragments with specificity for an IL-1β polypeptide) are specifically encompassed within the anti-IL-1β antibodies or binding fragments thereof of the present disclosure.

The present IL-1β binding antibodies and binding fragments can be humanized or human engineered antibodies. As used herein, a humanized antibody, or antigen binding fragment thereof, is a recombinant polypeptide that comprises a portion of an antigen binding site from a non-human antibody and a portion of the framework and/or constant regions of a human antibody. A human engineered antibody or antibody fragment is a non-human (*e.g.,* mouse) antibody that has been engineered by modifying (*e.g.,* deleting, inserting, or substituting) amino acids at specific positions so as to reduce or eliminate any detectable immunogenicity of the modified antibody in a human.

Humanized antibodies include chimeric antibodies and CDR-grafted antibodies. Chimeric antibodies are antibodies that include a non-human antibody variable region linked to a human constant region. Thus, in chimeric antibodies, the variable region is mostly non-human, and the constant region is human. Chimeric antibodies and methods for making them are described in Morrison, et al., Proc. Natl. Acad. Sci. USA, 81: 6841-6855 (1984), Boulianne, et al., Nature, 312: 643-646 (1984), and PCT Application Publication WO 86/01533. Although, they can be less immunogenic than a mouse monoclonal antibody, administrations of chimeric antibodies have been associated with human anti-mouse antibody responses (HAMA) to the non-human portion of the antibodies. Chimeric antibodies can also be produced by splicing the genes from a mouse antibody molecule of appropriate antigen-binding specificity together with genes from a human antibody molecule of appropriate biological activity, such as the ability to activate human complement and mediate ADCC. Morrison et al. (1984), Proc. Natl. Acad. Sci., 81: 6851; Neuberger et al. (1984), Nature, 312: 604. One example is the replacement of a Fc region with that of a different isotype.

CDR-grafted antibodies are antibodies that include the CDRs from a non-human "donor" antibody linked to the framework region from a human "recipient" antibody. Generally, CDR-grafted antibodies include more human antibody sequences than chimeric antibodies because they include both constant region sequences and variable region (framework) sequences from human antibodies. Thus, for example, a CDR-grafted humanized antibody of the invention can comprise a heavy chain that comprises a contiguous amino acid sequence (*e.g.,* about 5 or more, 10 or more, or even 15 or more contiguous amino acid residues) from the framework region of a human antibody (*e.g.,* FR-1, FR-2, or FR-3 of a human antibody) or, optionally, most or all of the entire framework region of a human antibody. CDR-grafted antibodies and methods for making them are described in, Jones et al., Nature, 321: 522-525 (1986), Riechmann et al., Nature, 332: 323-327 (1988), and Verhoeyen et al., Science, 239: 1534-1536 (1988)). Methods that can be used to produce humanized antibodies also are described in U.S. Patents 4,816,567, 5,721,367, 5,837,243, and 6,180,377. CDR-grafted antibodies are considered less likely than chimeric antibodies to induce an immune reaction against non-human antibody portions. However, it has been reported that framework sequences from the donor antibodies are required for the binding affinity and/or specificity of the donor antibody, presumably because these framework sequences affect the folding of the antigen-binding portion of the donor antibody. Therefore, when donor, non-human CDR sequences are grafted onto unaltered human framework sequences, the resulting CDR-grafted antibody can exhibit, in some cases, loss of binding avidity relative to the original non-human donor antibody. See, *e.g.,* Riechmann et al., Nature, 332: 323-327 (1988), and Verhoeyen et al., Science, 239: 1534-1536 (1988).

Human engineered antibodies include for example "veneered" antibodies and antibodies prepared using Human Engineering™ technology (see for example, U.S. Patents 5,766,886 and 5,869,619). Human Engineering™ technology is commercially available, and involves altering an non-human antibody or antibody fragment, such as a mouse or chimeric antibody or antibody fragment, by making specific changes to the amino acid sequence of the antibody so as to produce a modified antibody with reduced immunogenicity in a human that nonetheless retains the desirable binding properties of the original non-human antibodies. Generally, the technique involves classifying amino acid residues of a non-human (*e.g*., mouse) antibody as "low risk", "moderate risk", or "high risk" residues. The classification is performed using a global risk/reward calculation that evaluates the predicted benefits of making particular substitution (*e.g*., for immunogenicity in humans) against the risk that the substitution will affect the resulting antibody's folding and/or antigen-binding properties. Thus, a low risk position is one for which a substitution is predicted to be beneficial because it is predicted to reduce immunogenicity without significantly affecting antigen binding properties. A moderate risk position is one for which a substitution is predicted to reduce immunogenicity, but is more likely to affect protein folding and/or antigen binding. High risk positions contain residues most likely to be involved in proper folding or antigen binding. Generally, low risk positions in a non-human antibody are substituted with human residues, high risk positions are rarely substituted, and humanizing substitutions at moderate risk positions are sometimes made, although not indiscriminately. Positions with prolines in the non-human antibody variable region sequence are usually classified as at least moderate risk positions.

The particular human amino acid residue to be substituted at a given low or moderate risk position of a non-human (*e.g.,* mouse) antibody sequence can be selected by aligning an amino acid sequence from the non-human antibody's variable regions with the corresponding region of a specific or consensus human antibody sequence. The amino acid residues at low or moderate risk positions in the non-human sequence can be substituted for the corresponding residues in the human antibody sequence according to the alignment. Techniques for making human engineered proteins are described in greater detail in Studnicka et al., Protein Engineering, 7: 805-814 (1994), U.S. Patents 5,766,886, 5,770,196, 5,821,123, and 5,869,619, and PCT Application Publication WO 93/11794.

"Veneered" antibodies are non-human or humanized (*e.g.,* chimeric or CDR-grafted antibodies) antibodies that have been engineered to replace certain solvent-exposed amino acid residues so as to further reduce their immunogenicity or enhance their function. As surface residues of a chimeric antibody are presumed to be less likely to affect proper antibody folding and more likely to elicit an immune reaction, veneering of a chimeric antibody can include, for instance, identifying solvent-exposed residues in the non-human framework region of a chimeric antibody and replacing at least one of them with the corresponding surface residues from a human framework region. Veneering can be accomplished by any suitable engineering technique, including the use of the above-described Human Engineering™ technology.

In a different approach, a recovery of binding avidity can be achieved by "de-humanizing" a CDR-grafted antibody. De-humanizing can include restoring residues from the donor antibody's framework regions to the CDR grafted antibody, thereby restoring proper folding. Similar "de-humanization" can be achieved by (i) including portions of the "donor" framework region in the "recipient" antibody or (ii) grafting portions of the "donor" antibody framework region into the recipient antibody (along with the grafted donor CDRs).

For a further discussion of antibodies, humanized antibodies, human engineered, and methods for their preparation, see Kontermann and Dubel, eds., Antibody Engineering, Springer, New York, NY, 2001.

Exemplary humanized or human engineered antibodies include IgG, IgM, IgE, IgA, and IgD antibodies. The present antibodies can be of any class (IgG, IgA, IgM, IgE, IgD, *etc*.) or isotype and can comprise a kappa or lambda light chain. For example, a human antibody can comprise an IgG heavy chain or defined fragment, such as at least one of isotypes, IgG1, IgG2, IgG3 or IgG4. As a further example, the present antibodies or fragments can comprise an IgG1 heavy chain and an IgG1 light chain.

The present antibodies and fragments can be human antibodies, such as antibodies which bind IL-1β polypeptides and are encoded by nucleic acid sequences which are naturally occurring somatic variants of human germline immunoglobulin nucleic acid sequence, and fragments, synthetic variants, derivatives and fusions thereof. Such antibodies may be produced by any method known in the art, such as through the use of transgenic mammals (such as transgenic mice) in which the native immunoglobulin repertoire has been replaced with human V-genes in the mammal chromosome. Such mammals appear to carry out VDJ recombination and somatic hypermutation of the human germline antibody genes in a normal fashion, thus producing high affinity antibodies with completely human sequences.

Human antibodies to target protein can also be produced using transgenic animals that have no endogenous immunoglobulin production and are engineered to contain human immunoglobulin loci. For example, WO 98/24893 discloses transgenic animals having a human Ig locus wherein the animals do not produce functional endogenous immunoglobulins due to the inactivation of endogenous heavy and light chain loci. WO 91/00906 also discloses transgenic non-primate mammalian hosts capable of mounting an immune response to an immunogen, wherein the antibodies have primate constant and/or variable regions, and wherein the endogenous immunoglobulin encoding loci are substituted or inactivated. WO 96/30498 and US Patent No. 6,091,001 disclose the use of the Cre/Lox system to modify the immunoglobulin locus in a mammal, such as to replace all or a portion of the constant or variable region to form a modified antibody molecule. WO 94/02602 discloses non-human mammalian hosts having inactivated endogenous Ig loci and functional human Ig loci. U.S. Patent No. 5,939,598 discloses methods of making transgenic mice in which the mice lack endogenous heavy chains, and express an exogenous immunoglobulin locus comprising one or more xenogeneic constant regions. See also, U.S. Patent Nos. 6,114,598 6,657,103 and 6,833,268.

Using a transgenic animal described above, an immune response can be produced to a selected antigenic molecule, and antibody producing cells can be removed from the animal and used to produce hybridomas that secrete human monoclonal antibodies. Immunization protocols, adjuvants, and the like are known in the art, and are used in immunization of, for example, a transgenic mouse as described in WO 96/33735. This publication discloses monoclonal antibodies against a variety of antigenic molecules including IL-6, IL-8, TNFa, human CD4, L selectin, gp39, and tetanus toxin. The monoclonal antibodies can be tested for the ability to inhibit or neutralize the biological activity or physiological effect of the corresponding protein. WO 96/33735 discloses that monoclonal antibodies against IL-8, derived from immune cells of transgenic mice immunized with IL-8, blocked IL-8 induced functions of neutrophils. Human monoclonal antibodies with specificity for the antigen used to immunize transgenic animals are also disclosed in WO 96/34096 and U.S. patent application no. 20030194404; and U.S. patent application no. 20030031667.

Additional transgenic animals useful to make monoclonal antibodies include the Medarex HuMAb-MOUSE®, described in U.S. Pat. No. 5,770,429 and Fishwild, et al. (Nat. Biotechnol. 14:845-851, 1996), which contains gene sequences from unrearranged human antibody genes that code for the heavy and light chains of human antibodies. Immunization of a HuMAb-MOUSE® enables the production of fully human monoclonal antibodies to the target protein.

Also, Ishida et al. (Cloning Stem Cells 4:91-102, 2002) describes the TransChromo Mouse (TCMOUSE™) which comprises megabase-sized segments of human DNA and which incorporates the entire human immunoglobulin (hIg) loci. The TCMOUSE™ has a fully diverse repertoire of hlgs, including all the subclasses of IgGs (IgG1-G4). Immunization of the TC MOUSE™ with various human antigens produces antibody responses comprising human antibodies. Alternatively, Poueymirou et al. (Nature Biotechnology 25:91-99, 2007) describe the VelocImmune™ mouse (Regeneron Pharamceuticals, Inc., Tarrytown, NY) that is capable of producing fully human antibodies comprising human variable regions but preserving the mouse constant regions.

See also Jakobovits et al., Proc. Natl. Acad. Sci. USA, 90:2551 (1993); Jakobovits et al., Nature, 362:255-258 (1993); Bruggermann et al., Year in Immunol., 7:33 (1993); and U.S. Pat. No. 5,591,669, U.S. Patent No. 5,589,369, U.S. Patent No. 5,545,807; and U.S Patent Publication No. 20020199213. U.S. Patent Publication No. 20030092125 describes methods for biasing the immune response of an animal to the desired epitope. Human antibodies may also be generated by in vitro activated B cells (see, U.S. Pat. Nos. 5,567,610 and 5,229,275).

Human antibodies can also be generated through the *in vitro* screening of antibody display libraries. See Hoogenboom et al. (1991), J. Mol. Biol. 227: 381; and Marks et al. (1991), J. Mol. Biol. 222: 581. Various antibody-containing phage display libraries have been described and may be readily prepared. Libraries may contain a diversity of human antibody sequences, such as human Fab, Fv, and scFv fragments, that may be screened against an appropriate target. Phage display libraries may comprise peptides or proteins other than antibodies which may be screened to identify selective binding agents of IL-1β.

The development of technologies for making repertoires of recombinant human antibody genes, and the display of the encoded antibody fragments on the surface of filamentous bacteriophage, has provided a means for making human antibodies directly. The antibodies produced by phage technology are produced as antigen binding fragments-usually Fv or Fab fragments-in bacteria and thus lack effector functions. Effector functions can be introduced by one of two strategies: The fragments can be engineered either into complete antibodies for expression in mammalian cells, or into bispecific antibody fragments with a second binding site capable of triggering an effector function.

The disclosure contemplates a method for producing target-specific antibody or antigen-binding portion thereof comprising the steps of synthesizing a library of human antibodies on phage, screening the library with target protein or a portion thereof, isolating phage that bind target, and obtaining the antibody from the phage. By way of example, one method for preparing the library of antibodies for use in phage display techniques comprises the steps of immunizing a non-human animal comprising human immunoglobulin loci with target antigen or an antigenic portion thereof to create an immune response, extracting antibody producing cells from the immunized animal; isolating RNA from the extracted cells, reverse transcribing the RNA to produce cDNA, amplifying the cDNA using a primer, and inserting the cDNA into a phage display vector such that antibodies are expressed on the phage. Recombinant target-specific antibodies of the invention may be obtained in this way.

Phage-display processes mimic immune selection through the display of antibody repertoires on the surface of filamentous bacteriophage, and subsequent selection of phage by their binding to an antigen of choice. One such technique is described in WO 99/10494, which describes the isolation of high affinity and functional agonistic antibodies for MPL and msk receptors using such an approach. Antibodies of the invention can be isolated by screening of a recombinant combinatorial antibody library, preferably a scFv phage display library, prepared using human VL and VH cDNAs prepared from mRNA derived from human lymphocytes. Methodologies for preparing and screening such libraries are known in the art. See *e.g*., U.S. Patent No. 5,969,108. There are commercially available kits for generating phage display libraries (*e.g.*, the Pharmacia Recombinant Phage Antibody System, catalog no. 27-9400-01; and the Stratagene SurfZAP.TM. phage display kit, catalog no. 240612). There are also other methods and reagents that can be used in generating and screening antibody display libraries (see, *e.g.,* Ladner et al. U.S. Pat. No. 5,223,409; Kang et al. PCT Publication No. WO 92/18619; Dower et al. PCT Publication No. WO 91/17271; Winter et al. PCT Publication No. WO 92/20791; Markland et al. PCT Publication No. WO 92/15679; Breitling et al. PCT Publication No. WO 93/01288; McCafferty et al. PCT Publication No. WO 92/01047; Garrard et al. PCT Publication No. WO 92/09690; Fuchs et al. (1991) Bio/Technology 9:1370-1372; Hay et al. (1992) Hum. Antibod. Hybridomas 3:81-85; Huse et al. (1989) Science 246:1275-1281; McCafferty et al., Nature (1990) 348:552-554; Griffiths et al. (1993) EMBO J 12:725-734; Hawkins et al. (1992) J. Mol. Biol. 226:889-896; Clackson et al. (1991) Nature 352:624-628; Gram et al. (1992) Proc. Natl. Acad. Sci. USA 89:3576-3580; Garrad et al. (1991) Bio/Technology 9:1373-1377; Hoogenboom et al. (1991) Nuc Acid Res 19:4133-4137; and Barbas et al. (1991) Proc. Natl. Acad. Sci. USA 88:7978-7982.

In one embodiment, to isolate human antibodies specific for the target antigen with the desired characteristics, a human VH and VL library are screened to select for antibody fragments having the desired specificity. The antibody libraries used in this method are preferably scFv libraries prepared and screened as described herein and in the art (McCafferty et al., PCT Publication No. WO 92/01047, McCafferty et al., (Nature 348:552-554, 1990); and Griffiths et al., (EMBO J 12:725-734, 1993). The scFv antibody libraries preferably are screened using target protein as the antigen.

Alternatively, the Fd fragment (VH-CH1) and light chain (VL-CL) of antibodies are separately cloned by PCR and recombined randomly in combinatorial phage display libraries, which can then be selected for binding to a particular antigen. The Fab fragments are expressed on the phage surface, *e.g.,* physically linked to the genes that encode them. Thus, selection of Fab by antigen binding co-selects for the Fab encoding sequences, which can be amplified subsequently. Through several rounds of antigen binding and re-amplification, a procedure termed panning, Fab specific for the antigen are enriched and finally isolated.

In 1994, an approach for the humanization of antibodies, called "guided selection", was described. Guided selection utilizes the power of the phage display technique for the humanization of mouse monoclonal antibody (See Jespers, L. S., et al., Bio/Technology 12, 899-903 (1994)). For this, the Fd fragment of the mouse monoclonal antibody can be displayed in combination with a human light chain library, and the resulting hybrid Fab library may then be selected with antigen. The mouse Fd fragment thereby provides a template to guide the selection. Subsequently, the selected human light chains are combined with a human Fd fragment library. Selection of the resulting library yields entirely human Fab.

A variety of procedures have been described for deriving human antibodies from phage-display libraries (see, for example, Hoogenboom et al., J. Mol. Biol., 227:381 (1991); Marks et al., J. Mol. Biol, 222:581-597 (1991); U.S. Pat. Nos. 5,565,332 and 5,573,905; Clackson, T., and Wells, J. A., TIBTECH 12, 173-184 (1994)). In particular, *in vitro* selection and evolution of antibodies derived from phage display libraries has become a powerful tool (see, Burton, D. R., and Barbas III, C. F., Adv. Immunol. 57, 191-280 (1994); Winter, G., et al., Annu. Rev. Immunol. 12, 433-455 (1994); U.S. patent publication no. 20020004215 and WO 92/01047; U.S. patent publication no. 20030190317; and U.S. Patent Nos. 6,054,287 and 5,877,293.

Watkins, "Screening of Phage-Expressed Antibody Libraries by Capture Lift," Methods in Molecular Biology, Antibody Phage Display: Methods and Protocols 178: 187-193 (2002), and U.S. patent publication no. 20030044772, published March 6, 2003, describe methods for screening phage-expressed antibody libraries or other binding molecules by capture lift, a method involving immobilization of the candidate binding molecules on a solid support. Alternatively, antibody libraries including, for example, antibody libraries displayed on the surface of yeast may be screened as described in U.S. Patent No. 7,700,302.

Fv fragments are displayed on the surface of phage, by the association of one chain expressed as a phage protein fusion (*e.g.,* with M13 gene III) with the complementary chain expressed as a soluble fragment. It is contemplated that the phage may be a filamentous phage such as one of the class I phages: fd, M13, f1, If1, Ike, ZJ/Z, Ff and one of the class II phages Xf, Pf1 and Pf3. The phage may be M13, or fd or a derivative thereof.

Once initial human VL and VH segments are selected, "mix and match" experiments, in which different pairs of the initially selected VL and VH segments are screened for target binding, are performed to select preferred VL/VH pair combinations. Additionally, to further improve the quality of the antibody, the VL and VH segments of the preferred VL/VH pair(s) can be randomly mutated, preferably within the any of the CDR1, CDR2 or CDR3 region of VH and/or VL, in a process analogous to the in vivo somatic mutation process responsible for affinity maturation of antibodies during a natural immune response. This in vitro affinity maturation can be accomplished by amplifying VL and VH regions using PCR primers complimentary to the VH CDR1, CDR2, and CDR3, or VL CDR1, CDR2, and CDR3, respectively, which primers have been "spiked" with a random mixture of the four nucleotide bases at certain positions such that the resultant PCR products encode VL and VH segments into which random mutations have been introduced into the VH and/or VL CDR3 regions. These randomly mutated VL and VH segments can be rescreened for binding to target antigen.

Following screening and isolation of an target specific antibody from a recombinant immunoglobulin display library, nucleic acid encoding the selected antibody can be recovered from the display package (*e.g*., from the phage genome) and subcloned into other expression vectors by standard recombinant DNA techniques. If desired, the nucleic acid can be further manipulated to create other antibody forms of the invention, as described below. To express a recombinant human antibody isolated by screening of a combinatorial library, the DNA encoding the antibody is cloned into a recombinant expression vector and introduced into a mammalian host cell, as described herein.

It is contemplated that the phage display method may be carried out in a mutator strain of bacteria or host cell. A mutator strain is a host cell which has a genetic defect which causes DNA replicated within it to be mutated with respect to its parent DNA. Example mutator strains are NR9046mutD5 and NR9046 mut T1.

It is also contemplated that the phage display method may be carried out using a helper phage. This is a phage which is used to infect cells containing a defective phage genome and which functions to complement the defect. The defective phage genome can be a phagemid or a phage with some function encoding gene sequences removed. Examples of helper phages are M13K07, M13K07 gene III no. 3; and phage displaying or encoding a binding molecule fused to a capsid protein.

Antibodies are also generated via phage display screening methods using the hierarchical dual combinatorial approach as disclosed in WO 92/01047 in which an individual colony containing either an H or L chain clone is used to infect a complete library of clones encoding the other chain (L or H) and the resulting two-chain specific binding member is selected in accordance with phage display techniques such as those described therein. This technique is also disclosed in Marks et al., (Bio/Technology, 10:779-783, 1992).

Methods for display of peptides on the surface of yeast and microbial cells have also been used to identify antigen specific antibodies. See, for example, U.S. Patent No. 6,699,658. Antibody libraries may be attached to yeast proteins, such as agglutinin, effectively mimicking the cell surface display of antibodies by B cells in the immune system. Alternatively, antibody libraries may be displayed on the surface of eukaryotic cells (*e.g*., yeast and mammalian cells) or prokaryotic cells via an interaction of protein-PDZ-binding peptide fusions to PDZ Domain-cell surface protein fusions (*see, e.g.,* WO 2012/092323).

In addition to phage display methods, antibodies may be isolated using ribosome mRNA display methods and microbial cell display methods. Selection of polypeptide using ribosome display is described in Hanes et al., (Proc. Natl Acad Sci USA, 94:4937-4942, 1997) and U.S. Pat. Nos. 5,643,768 and 5,658,754 issued to Kawasaki. Ribosome display is also useful for rapid large scale mutational analysis of antibodies. The selective mutagenesis approach also provides a method of producing antibodies with improved activities that can be selected using ribosomal display techniques.

IL-1β binding antibodies and binding fragments may comprise one or more portions that do not bind IL-1β but instead are responsible for other functions, such as circulating half-life, direct cytotoxic effect, detectable labeling, or activation of the recipient's endogenous complement cascade or endogenous cellular cytotoxicity. The antibodies or fragments may comprise all or a portion of the constant region and may be of any isotype, including IgA (*e.g.,* IgA1 or IgA2), IgD, IgE, IgG (*e.g.* IgG1, IgG2, IgG3 or IgG4), or IgM. In addition to, or instead of, comprising a constant region, antigen-binding compounds of the invention may include an epitope tag, a salvage receptor epitope, a label moiety for diagnostic or purification purposes, or a cytotoxic moiety such as a radionuclide or toxin.

The constant region (when present) of the present antibodies and fragments may be of the γ1, γ2, γ3, γ4, µ, β2, or δ or ε type, preferably of the γ type, more preferably of the y, type, whereas the constant part of a human light chain may be of the κ or λ type (which includes the λ1, λ2 and λ3 subtypes) but is preferably of the κ type.

Variants also include antibodies or fragments comprising a modified Fc region, wherein the modified Fc region comprises at least one amino acid modification relative to a wild-type Fc region. The variant Fc region may be designed, relative to a comparable molecule comprising the wild-type Fc region, so as to bind Fc receptors with a greater or lesser affinity.

For example, the present anti-IL-1β antibodies or binding fragments thereof may comprise a modified Fc region. Fc region refers to naturally-occurring or synthetic polypeptides homologous to the IgG C-terminal domain that is produced upon papain digestion of IgG. IgG Fc has a molecular weight of approximately 50 kD. In the present antibodies and fragments, an entire Fc region can be used, or only a half-life enhancing portion. In addition, many modifications in amino acid sequence are acceptable, as native activity is not in all cases necessary or desired.

The Fc region can be mutated, if desired, to inhibit its ability to fix complement and bind the Fc receptor with high affinity. For murine IgG Fc, substitution of Ala residues for Glu 318, Lys 320, and Lys 322 renders the protein unable to direct ADCC. Substitution of Glu for Leu 235 inhibits the ability of the protein to bind the Fc receptor with high affinity. Various mutations for human IgG also are known (see, *e.g*., Morrison et al., 1994, The Immunologist 2: 119 124 and Brekke et al., 1994, The Immunologist 2: 125).

In some embodiments, the present antibodies or fragments are provided with a modified Fc region where a naturally-occurring Fc region is modified to increase the half-life of the antibody or fragment in a biological environment, for example, the serum half-life or a half-life measured by an in vitro assay. Methods for altering the original form of a Fc region of an IgG also are described in U.S. Patent No. 6,998,253.

In certain embodiments, it may be desirable to modify the antibody or fragment in order to increase its serum half-life, for example, adding molecules such as PEG or other water soluble polymers, including polysaccharide polymers, to antibody fragments to increase the half-life. This may also be achieved, for example, by incorporation of a salvage receptor binding epitope into the antibody fragment (*e.g.,* by mutation of the appropriate region in the antibody fragment or by incorporating the epitope into a peptide tag that is then fused to the antibody fragment at either end or in the middle, *e.g.,* by DNA or peptide synthesis) (see, International Publication No. WO96/32478). Salvage receptor binding epitope refers to an epitope of the Fc region of an IgG molecule (*e.g*.*,* IgG1, IgG2, IgG3, or IgG4) that is responsible for increasing the in vivo serum half-life of the IgG molecule.

A salvage receptor binding epitope can include a region wherein any one or more amino acid residues from one or two loops of a Fc domain are transferred to an analogous position of the antibody fragment. Even more preferably, three or more residues from one or two loops of the Fc domain are transferred. Still more preferred, the epitope is taken from the CH2 domain of the Fc region (*e.g.,* of an IgG) and transferred to the CH1, CH3, or VH region, or more than one such region, of the antibody. Alternatively, the epitope is taken from the CH2 domain of the Fc region and transferred to the CL region or VL region, or both, of the antibody fragment. See also International applications WO 97/34631 and WO 96/32478 which describe Fc variants and their interaction with the salvage receptor.

Mutation of residues within Fc receptor binding sites can result in altered effector function, such as altered ADCC or CDC activity, or altered half-life. Potential mutations include insertion, deletion or substitution of one or more residues, including substitution with alanine, a conservative substitution, a non-conservative substitution, or replacement with a corresponding amino acid residue at the same position from a different IgG subclass (*e.g.* replacing an IgG1 residue with a corresponding IgG2 residue at that position). For example it has been reported that mutating the serine at amino acid position 241 in IgG4 to proline (found at that position in IgG1 and IgG2) led to the production of a homogeneous antibody, as well as extending serum half-life and improving tissue distribution compared to the original chimeric IgG4. (Angal et al., Mol Immunol. 30:105-8, 1993).

Antibody fragments are portions of an intact full length antibody, such as an antigen binding or variable region of the intact antibody. Examples of antibody fragments include Fab, Fab', F(ab')2, and Fv fragments; diabodies; linear antibodies; single-chain antibody molecules (*e.g.,* scFv); multispecific antibody fragments such as bispecific, trispecific, and multispecific antibodies (*e.g.,* diabodies, triabodies, tetrabodies); minibodies; chelating recombinant antibodies; tribodies or bibodies; intrabodies; nanobodies; small modular immunopharmaceuticals (SMIP), adnectins, binding-domain immunoglobulin fusion proteins; camelized antibodies; VHH containing antibodies; and any other polypeptides formed from antibody fragments.

The present disclosure includes IL-1β binding fragments comprising any of the foregoing heavy or light chain sequences and which bind IL-1β. The term fragments as used herein refers to any 3 or more contiguous amino acids (*e.g.,* 4 or more, 5 or more 6 or more, 8 or more, or even 10 or more contiguous amino acids) of the antibody and encompasses Fab, Fab', F(ab')2, and F(v) fragments, or the individual light or heavy chain variable regions or portion thereof. IL-1β binding fragments include, for example, Fab, Fab', F(ab')2, Fv and scFv. These fragments lack the Fc fragment of an intact antibody, clear more rapidly from the circulation, and can have less non-specific tissue binding than an intact antibody. See Wahl et al. (1983), J. Nucl. Med., 24: 316-25. These fragments can be produced from intact antibodies using well known methods, for example by proteolytic cleavage with enzymes such as papain (to produce Fab fragments) or pepsin (to produce F(ab')2 fragments).

In vitro and cell based assays are well described in the art for use in determining binding of IL-1β to IL-1 receptor type I (IL-1R1), including assays that determining in the presence of molecules (such as antibodies, antagonists, or other inhibitors) that bind to IL-1β or IL-1RI. (see for example Evans et al., (1995), J. Biol. Chem. 270:11477-11483; Vigers et al., (2000), J. Biol. Chem. 275:36927-36933; Yanofsky et al., (1996), Proc. Natl. Acad. Sci. USA 93:7381-7386; Fredericks et al., (2004), Protein Eng. Des. Sel. 17:95-106; Slack et al., (1993), J. Biol. Chem. 268:2513-2524; Smith et al., (2003), Immunity 18:87-96; Vigers et al., (1997), Nature 386:190-194; Ruggiero et al., (1997), J. Immunol. 158:3881-3887; Guo et al., (1995), J. Biol. Chem. 270:27562-27568; Svenson et al., (1995), Eur. J. Immunol. 25:2842-2850; Arend et al., (1994), J. Immunol. 153:4766-4774). Recombinant IL-1 receptor type I, including human IL-1 receptor type I, for such assays is readily available from a variety of commercial sources (see for example R&D Systems, Sigma). IL-1 receptor type I also can be expressed from an expression construct or vector introduced into an appropriate host cell using standard molecular biology and transfection techniques known in the art. The expressed IL-1 receptor type I may then be isolated and purified for use in binding assays, or alternatively used directly in a cell associated form.

For example, the binding of IL-1β to IL-1 receptor type I may be determined by immobilizing an anti-IL-1β antibody or binding fragment thereof, contacting IL-1β with the immobilized antibody and determining whether the IL-1β was bound to the antibody, and contacting a soluble form of IL-1RI with the bound IL-1β/antibody complex and determining whether the soluble IL-1RI was bound to the complex. The protocol may also include contacting the soluble IL-1RI with the immobilized antibody before the contact with It-1β, to confirm that the soluble IL-1RI does not bind to the immobilized antibody. This protocol can be performed using a Biacore® instrument for kinetic analysis of binding interactions. Such a protocol can also be employed to determine whether an antibody or other molecule permits or blocks the binding of IL-1β to IL-1 receptor type I.

For other IL-1β / IL-1RI binding assays, the permitting or blocking of IL-1β binding to IL-1 receptor type I may be determined by comparing the binding of IL-1β to IL-1RI in the presence or absence of anti-IL-1β antibodies or binding fragments thereof. Blocking is identified in the assay readout as a designated reduction of IL-1β binding to IL-1 receptor type I in the presence of anti-IL-1β antibodies or binding fragments thereof, as compared to a control sample that contains the corresponding buffer or diluent but not an anti-IL-1β antibody or binding fragment thereof. The assay readout may be qualitatively viewed as indicating the presence or absence of blocking, or may be quantitatively viewed as indicating a percent or fold reduction in binding due to the presence of the antibody or fragment.

Alternatively or additionally, when an anti-IL-1β antibodies or binding fragments thereof substantially blocks IL-1β binding to IL-1RI, the IL-1β binding to IL-1RI is reduced by at least 10-fold, alternatively at least about 20-fold, alternatively at least about 50-fold, alternatively at least about 100-fold, alternatively at least about 1000-fold, alternatively at least about 10000-fold, or more, compared to binding of the same concentrations of IL-1β and IL-1RI in the absence of the antibody or fragment. As another example, when an anti-IL-1β antibody or binding fragment thereof substantially permits IL-1β binding to IL-1RI, the IL-1β binding to IL-1RI is at least about 90%, alternatively at least about 95%, alternatively at least about 99%, alternatively at least about 99.9%, alternatively at least about 99.99%, alternatively at least about 99.999%, alternatively at least about 99.9999%, alternatively substantially identical to binding of the same concentrations of IL-1β and IL-1RI in the absence of the antibody or fragment.

The present disclosure may in certain embodiments encompass anti-IL-1β antibodies or binding fragments thereof that bind to the same epitope or substantially the same epitope as one or more of the exemplary antibodies described herein. Alternatively or additionally, the anti-IL-1β antibodies or binding fragments thereof compete with the binding of an antibody having variable region sequences of AB7, described in US Patent 7,531,166 (sequences shown below). As an example, when an anti-IL-1β antibody or binding fragment thereof competes with the binding of an antibody having the light chain variable region of SEQ ID NO: 5 and the heavy chain variable region of SEQ ID NO: 6, binding of an antibody having the light chain variable region of SEQ ID NO: 5 and the heavy chain variable region of SEQ ID NO: 6 to IL-1β may be reduced by at least about 2-fold, alternatively at least about 5-fold, alternatively at least about 10-fold, alternatively at least about 20-fold, alternatively at least about 50-fold, alternatively at least about 100-fold, alternatively at least about 1000-fold, alternatively at least about 10000-fold, or more, if the binding is measured in the presence of the anti-IL-1β antibody or binding fragment thereof. The anti-IL-1β antibody or binding fragment thereof may be present in excess of the antibody having the light chain variable region of SEQ ID NO: 5 and the heavy chain variable region of SEQ ID NO: 6, for example an excess of least about 2-fold, alternatively at least about 5-fold, alternatively at least about 10-fold, alternatively at least about 20-fold, alternatively at least about 50-fold, alternatively at least about 100-fold, alternatively at least about 1000-fold, alternatively at least about 10000-fold. Alternatively or additionally, the present disclosure encompasses anti-IL-1β antibodies or binding fragments thereof that bind to an epitope contained in the amino acid sequence ESVDPKNYPKKKMEKRFVFNKIE (SEQ ID NO: 1) (US Patent 7,531,166) which corresponds to residues 83-105 of the mature IL-1β protein. As contemplated herein, one can readily determine if an anti-IL-1β antibody or binding fragment thereof binds to the same epitope or substantially the same epitope as one or more of the exemplary antibodies, such as for example the antibody designated AB7, using any of several known methods in the art.

For example, the key amino acid residues (epitope) bound by an anti-IL-1β antibody or binding fragment thereof may be determined using a peptide array, such as for example, a PepSpot™ peptide array (JPT Peptide Technologies, Berlin, Germany), wherein a scan of twelve amino-acid peptides, spanning the entire IL-1β amino acid sequence, each peptide overlapping by 11 amino acid to the previous one, is synthesized directly on a membrane. The membrane carrying the peptides is then probed with the antibody for which epitope binding information is sought, for example at a concentration of 2 µg/ml, for 2 hr at room temperature. Binding of antibody to membrane bound peptides may be detected using a secondary HRP-conjugated goat anti-human (or mouse, when appropriate) antibody, followed by enhanced chemiluminescence (ECL). The peptides spot(s) corresponding to particular amino acid residues or sequences of the mature IL-1β protein, and which score positive for antibody binding, are indicative of the epitope bound by the particular antibody.

Alternatively or additionally, the present disclosure encompasses anti-IL-1β antibodies or binding fragments thereof that bind an epitope of human IL-1β that comprises (a) a valine residue at a position corresponding to position 72 (Val72) of the human IL-1β sequence set forth in SEQ ID NO: 35, (b) a leucine residue at position 73 (Leu73) of the human IL-1β sequence set forth in SEQ ID NO: 35, (c) a lysine residue at a position corresponding to position 74 (Lys74) of the human IL-1β sequence set forth in SEQ ID NO: 35, (d) an aspartic acid residue at a position corresponding to position 75 (Asp75) of the human IL-1β sequence set forth in SEQ ID NO: 35, (e) a glutamine residue at a position corresponding to position 81 (Gln81) of the human IL-1β sequence set forth in SEQ ID NO: 35, (f) a glutamic acid residue at a position corresponding to position 83 (Glu83) of the human IL-1β sequence set forth in SEQ ID NO: 35, (g) a serine residue at a position corresponding to position 84 (Ser84) of the human IL-1β sequence set forth in SEQ ID NO: 35, (h) an asparagine residue at a position corresponding to position 89 (Asn89) of the human IL-1β sequence set forth in SEQ ID NO: 35, (i) a tyrosine residue at a position corresponding to position 90 (Tyr90) of the human IL-1β sequence set forth in SEQ ID NO: 35, (j) a lysine residue at a position corresponding to position 92 (Lys92) of the human IL-1β sequence set forth in SEQ ID NO: 35, (k) a lysine residue at a position corresponding to position 94 (Lys94) of the human IL-1β sequence set forth in SEQ ID NO: 35, (I) a glutamic acid residue at a position corresponding to position 96 (Glu96) of the human IL-1β sequence set forth in SEQ ID NO: 35, (m) a lysine residue at a position corresponding to position 97 (Lys97) of the human IL-1β sequence set forth in SEQ ID NO: 35, (n) an arginine residue at a position corresponding to position 98 (Arg98) of the human IL-1β sequence set forth in SEQ ID NO: 35, (o) an alanine residue at a position corresponding to position 115 (Ala115) of the human IL-1β sequence set forth in SEQ ID NO: 35, (p) a glutamine residue at a position corresponding to position 116 (Gln116) of the human IL-1β sequence set forth in SEQ ID NO: 35, and/or (q) a phenylalanine residue at a position corresponding to position 117 (Phe117) of the human IL-1β sequence set forth in SEQ ID NO: 35. The IL-1β antibodies or binding fragments thereof may bind to an epitope of human IL-1β that comprises one or more of amino acid residues (a) - (q), including, all of the amino acid residues (a) - (q). Residues that comprise the epitope of human IL-1β bound by IL-1β antibodies or binding fragments thereof may include those residues (*e.g*., residues (a) - (q) above) identified by both X-ray crystallography and NMR spectroscopy.

Alternatively or additionally, the present disclosure encompasses anti-IL-1β antibodies or binding fragments thereof that bind an epitope of human IL-1β that comprises (a) a valine residue at a position corresponding to position 72 (Val72) of the human IL-1β sequence set forth in SEQ ID NO: 35, (b) a leucine residue at position 73 (Leu73) of the human IL-1β sequence set forth in SEQ ID NO: 35, (c) a lysine residue at a position corresponding to position 74 (Lys74) of the human IL-1β sequence set forth in SEQ ID NO: 35, (d) an aspartic acid residue at a position corresponding to position 75 (Asp75) of the human IL-1β sequence set forth in SEQ ID NO: 35, (e) a glutamine residue at a position corresponding to position 81 (Gln81) of the human IL-1β sequence set forth in SEQ ID NO: 35, (f) a glutamic acid residue at a position corresponding to position 83 (Glu83) of the human IL-1β sequence set forth in SEQ ID NO: 35, (g) a serine residue at a position corresponding to position 84 (Ser84) of the human IL-1β sequence set forth in SEQ ID NO: 35, (h) an aspartic acid residue at a position corresponding to position 86 (Asp86) of the human IL-1β sequence set forth in SEQ ID NO: 35, (i) an asparagine residue at a position corresponding to position 89 (Asn89) of the human IL-1β sequence set forth in SEQ ID NO: 35, (j) a tyrosine residue at a position corresponding to position 90 (Tyr90) of the human IL-1β sequence set forth in SEQ ID NO: 35, (k) a lysine residue at a position corresponding to position 92 (Lys92) of the human IL-1β sequence set forth in SEQ ID NO: 35, (I) a lysine residue at a position corresponding to position 94 (Lys94) of the human IL-1β sequence set forth in SEQ ID NO: 35, (m) a glutamic acid residue at a position corresponding to position 96 (Glu96) of the human IL-1β sequence set forth in SEQ ID NO: 35, (n) a lysine residue at a position corresponding to position 97 (Lys97) of the human IL-1β sequence set forth in SEQ ID NO: 35, (o) an arginine residue at a position corresponding to position 98 (Arg98) of the human IL-1β sequence set forth in SEQ ID NO: 35, (p) an alanine residue at a position corresponding to position 115 (Ala115) of the human IL-1β sequence set forth in SEQ ID NO: 35, (q) a glutamine residue at a position corresponding to position 116 (Gln116) of the human IL-1β sequence set forth in SEQ ID NO: 35, (r) a phenylalanine residue at a position corresponding to position 117 (Phe117) of the human IL-1β sequence set forth in SEQ ID NO: 35, and/or (s) a proline residue at a position corresponding to position 118 (Pro118) of the human IL-1β sequence set forth in SEQ ID NO: 35. The IL-1β antibodies or binding fragments thereof may bind to an epitope of human IL-1β that comprises one or more of amino acid residues (a) - (s), including, all of the amino acid residues (a) - (s). Residues that comprise the epitope of human IL-1β bound by IL-1β antibodies or binding fragments thereof may include those residues (*e.g*., residues (a) - (s) above) identified by X-ray crystallography.

Alternatively or additionally, the present disclosure encompasses anti-IL-1β antibodies or binding fragments thereof that bind an epitope of human IL-1β that comprises (a) a glycine residue at a position corresponding to position 49 (Gly49) of the human IL-1β sequence set forth in SEQ ID NO: 35, (b) a valine residue at a position corresponding to position 72 (Val72) of the human IL-1β sequence set forth in SEQ ID NO: 35, (c) a leucine residue at position 73 (Leu73) of the human IL-1β sequence set forth in SEQ ID NO: 35, (d) a lysine residue at a position corresponding to position 74 (Lys74) of the human IL-1β sequence set forth in SEQ ID NO: 35, (e) an aspartic acid residue at a position corresponding to position 75 (Asp75) of the human IL-1β sequence set forth in SEQ ID NO: 35, (f) a glutamine residue at a position corresponding to position 81 (Gln81) of the human IL-1β sequence set forth in SEQ ID NO: 35, (g) a leucine residue at a position corresponding to position 82 (Leu82) of the human IL-1β sequence set forth in SEQ ID NO: 35, (h) a glutamic acid residue at a position corresponding to position 83 (Glu83) of the human IL-1β sequence set forth in SEQ ID NO: 35, (i) a serine residue at a position corresponding to position 84 (Ser84) of the human IL-1β sequence set forth in SEQ ID NO: 35, (j) an asparagine residue at a position corresponding to position 89 (Asn89) of the human IL-1β sequence set forth in SEQ ID NO: 35, (k) a tyrosine residue at a position corresponding to position 90 (Tyr90) of the human IL-1β sequence set forth in SEQ ID NO: 35, (I) a lysine residue at a position corresponding to position 92 (Lys92) of the human IL-1β sequence set forth in SEQ ID NO: 35, (m) a lysine residue at a position corresponding to position 93 (Lys93) of the human IL-1β sequence set forth in SEQ ID NO: 35, (n) a lysine residue at a position corresponding to position 94 (Lys94) of the human IL-1β sequence set forth in SEQ ID NO: 35, (o) a methionine residue at a position corresponding to position 95 (Met95) of the human IL-1β sequence set forth in SEQ ID NO: 35, (p) a glutamic acid residue at a position corresponding to position 96 (Glu96) of the human IL-1β sequence set forth in SEQ ID NO: 35, (q) a lysine residue at a position corresponding to position 97 (Lys97) of the human IL-1β sequence set forth in SEQ ID NO: 35, (r) an arginine residue at a position corresponding to position 98 (Arg98) of the human IL-1β sequence set forth in SEQ ID NO: 35, (s) a valine residue at a position corresponding to position 100 (Val100) of the human IL-1β sequence set forth in SEQ ID NO: 35, (t) an alanine residue at a position corresponding to position 115 (Ala115) of the human IL-1β sequence set forth in SEQ ID NO: 35, (u) a glutamine residue at a position corresponding to position 116 (Gln116) of the human IL-1β sequence set forth in SEQ ID NO: 35, (v) a phenylalanine residue at a position corresponding to position 117 (Phe117) of the human IL-1β sequence set forth in SEQ ID NO: 35, and/or (w) a tyrosine residue at a position corresponding to position 121 (Tyr121) of the human IL-1β sequence set forth in SEQ ID NO: 35. The IL-1β antibodies or binding fragments thereof may bind to an epitope of human IL-1β that comprises one or more of amino acid residues (a) - (w), including, all of the amino acid residues (a) - (w). Residues that comprise the epitope of human IL-1β bound by IL-1β antibodies or binding fragments thereof may include those residues (*e.g*., residues (a) - (w) above) identified by NMR spectroscopy.

Alternatively or in addition, antibody competition experiments may be performed and such assays are well known in the art. For example, an antibody of unknown specificity may be compared with any of the exemplary of antibodies (*e.g.*, AB7) of the present disclosure. Binding competition assays may be performed, for example, using a Biacore® instrument for kinetic analysis of binding interactions or by ELISA. In such an assay, the antibody of unknown epitope specificity is evaluated for its ability to compete for binding against the known comparator antibody (*e.g*., AB7). Competition for binding to a particular epitope is determined by a reduction in binding to the IL-1β epitope of at least about 50%, or at least about 70%, or at least about 80%, or at least about 90%, or at least about 95%, or at least about 99% or about 100% for the known comparator antibody (*e.g*., AB7) and is indicative of binding to substantially the same epitope.

In view of the identification in this disclosure of IL-1β binding regions in exemplary antibodies and/or epitopes recognized by the disclosed antibodies, it is contemplated that additional antibodies with similar binding characteristics and therapeutic or diagnostic utility can be generated that parallel the embodiments of this disclosure.

Antigen-binding fragments of an antibody include fragments that retain the ability to specifically bind to an antigen, generally by retaining the antigen-binding portion of the antibody. It is well established that the antigen-binding function of an antibody can be performed by fragments of a full-length antibody. Examples of antigen-binding portions include (i) a Fab fragment, which is a monovalent fragment consisting of the VL, VH, CL and CH1 domains; (ii) a F(ab')2 fragment, which is a bivalent fragment comprising two Fab fragments linked by a disulfide bridge at the hinge region; (iii) a Fd fragment which is the VH and CH1 domains; (iv) a Fv fragment which is the VL and VH domains of a single arm of an antibody, (v) a dAb fragment (Ward et al., (1989) Nature 341:544-546), which is a VH domain; and (vi) an isolated complementarity determining region (CDR). Single chain antibodies are also encompassed within the term antigen-binding portion of an antibody. The anti-IL-1β antibodies or binding fragments thereof of the present invention also encompass monovalent or multivalent, or monomeric or multimeric (e.g. tetrameric), CDR-derived binding domains with or without a scaffold (for example, protein or carbohydrate scaffolding).

The present anti-IL-1β antibodies or binding fragments thereof may be part of larger immunoadhesion molecules, formed by covalent or non-covalent association of the antibody or antibody portion with one or more other proteins or peptides. Examples of such immunoadhesion molecules include use of the streptavidin core region to make a tetrameric scFv molecule (Kipriyanov, S. M., et al. (1995) Human Antibodies and Hybridomas 6:93-101) and use of a cysteine residue, a marker peptide and a C-terminal polyhistidine tag to make bivalent and biotinylated scFv molecules (Kipriyanov, S. M., et al. (1994) Mol. Immunol. 31:1047-1058). Antibodies and fragments comprising immunoadhesion molecules can be obtained using standard recombinant DNA techniques, as described herein. Preferred antigen binding portions are complete domains or pairs of complete domains.

The anti-IL-1β antibodies and binding fragments thereof may also encompass domain antibody (dAb) fragments (Ward et al., Nature 341:544-546, 1989) which consist of a VH domain. The anti-IL-1β antibodies or binding fragments thereof of the present invention also encompass diabodies, which are bivalent antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* EP 404,097; WO 93/11161; Holliger et al., Proc. Natl. Acad. Sci. USA 90:6444-6448, 1993, and Poljak et al., Structure 2:1121-1123, 1994). Diabodies can be bispecific or monospecific.

The anti-IL-1β antibodies or binding fragments thereof of the present disclosure also encompass single-chain antibody fragments (scFv) that bind to IL-1β. An scFv comprises an antibody heavy chain variable region (VH) operably linked to an antibody light chain variable region (VL) wherein the heavy chain variable region and the light chain variable region, together or individually, form a binding site that binds IL-1β. An scFv may comprise a VH region at the amino-terminal end and a VL region at the carboxy-terminal end. Alternatively, scFv may comprise a VL region at the amino-terminal end and a VH region at the carboxy-terminal end. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. (1988) Science 242:423-426; and Huston et al. (1988) Proc. Natl. Acad. Sci. USA 85:5879-5883).

An scFv may optionally further comprise a polypeptide linker between the heavy chain variable region and the light chain variable region. Such polypeptide linkers generally comprise between 1 and 50 amino acids, alternatively between 3 and 12 amino acids, alternatively 2 amino acids. An example of a linker peptide for linking heavy and light chains in an scFv comprises the 5 amino acid sequence Gly-Gly-Gly-Gly-Ser (SEQ ID NO: 2). Other examples comprise one or more tandem repeats of this sequence (for example, a polypeptide comprising two to four repeats of Gly-Gly-Gly-Gly-Ser (SEQ ID NO: 2) to create linkers.

The IL anti-IL-1β antibodies or binding fragments thereof of the present invention also encompass heavy chain antibodies (HCAb). Exceptions to the H2L2 structure of conventional antibodies occur in some isotypes of the found in camelids (camels, dromedaries and llamas; Hamers-Casterman et al., 1993 Nature 363: 446; Nguyen et al., 1998 J. Mol. Biol. 275: 413), wobbegong sharks (Nuttall et al., Mol Immunol. 38:313-26, 2001), nurse sharks (Greenberg et al., Nature 374:168-73, 1995; Roux et al., 1998 Proc. Nat. Acad. Sci. USA 95: 11804), and in the spotted ratfish (Nguyen, et al., "Heavy-chain antibodies in Camelidae; a case of evolutionary innovation," 2002 Immunogenetics 54(1): 39-47). These antibodies can apparently form antigen-binding regions using only heavy chain variable regions, in that these functional antibodies are dimers of heavy chains only (referred to as "heavy-chain antibodies" or "HCAbs"). Accordingly, some embodiments of the present anti-IL-1β antibodies or binding fragments thereof may be heavy chain antibodies that specifically bind to IL-1β. For example, heavy chain antibodies that are a class of IgG and devoid of light chains are produced by animals of the genus Camelidae which includes camels, dromedaries and llamas (Hamers-Casterman et al., Nature 363:446-448 (1993)). HCAbs have a molecular weight of about 95 kDa instead of the about 160 kDa molecular weight of conventional IgG antibodies. Their binding domains consist only of the heavy-chain variable domains, often referred to as VHH to distinguish them from conventional VH. Muyldermans et al., J. Mol. Recognit. 12:131-140 (1999). The variable domain of the heavy-chain antibodies is sometimes referred to as a nanobody (Cortez-Retamozo et al., Cancer Research 64:2853-57, 2004). A nanobody library may be generated from an immunized dromedary as described in Conrath et al., (Antimicrob Agents Chemother 45: 2807-12, 2001) or using recombinant methods.

Since the first constant domain (CH1) is absent (spliced out during mRNA processing due to loss of a splice consensus signal), the variable domain (VHH) is immediately followed by the hinge region, the CH2 and the CH3 domains (Nguyen et al., Mol. Immunol. 36:515-524 (1999); Woolven et al., Immunogenetics 50:98-101 (1999)). Camelid VHH reportedly recombines with IgG2 and IgG3 constant regions that contain hinge, CH2, and CH3 domains and lack a CH1 domain (Hamers-Casterman *et al*., supra). For example, llama IgG1 is a conventional (H2L2) antibody isotype in which VH recombines with a constant region that contains hinge, CH1, CH2 and CH3 domains, whereas the llama IgG2 and IgG3 are heavy chain-only isotypes that lack CH1 domains and that contain no light chains.

Although the HCAbs are devoid of light chains, they have an antigen-binding repertoire. The genetic generation mechanism of HCAbs is reviewed in Nguyen et al. Adv. Immunol 79:261-296 (2001) and Nguyen et al., Immunogenetics 54:39-47 (2002). Sharks, including the nurse shark, display similar antigen receptor-containing single monomeric V-domains. Irving et al., J. Immunol. Methods 248:31-45 (2001); Roux et al., Proc. Natl. Acad. Sci. USA 95:11804 (1998).

VHHs comprise small intact antigen-binding fragments (for example, fragments that are about 15 kDa, 118-136 residues). Camelid VHH domains have been found to bind to antigen with high affinity (Desmyter et al., J. Biol. Chem. 276:26285-90, 2001), with VHH affinities typically in the nanomolar range and comparable with those of Fab and scFv fragments. VHHs are highly soluble and more stable than the corresponding derivatives of scFv and Fab fragments. VH fragments have been relatively difficult to produce in soluble form, but improvements in solubility and specific binding can be obtained when framework residues are altered to be more VHH-like. (See, for example, Reichman et al., J Immunol Methods 1999, 231:25-38.) VHHs carry amino acid substitutions that make them more hydrophilic and prevent prolonged interaction with BiP (immunoglobulin heavy-chain binding protein), which normally binds to the H-chain in the Endoplasmic Reticulum (ER) during folding and assembly, until it is displaced by the L-chain. Because of the VHHs' increased hydrophilicity, secretion from the ER is improved.

Functional VHHs may be obtained by proteolytic cleavage of HCAb of an immunized camelid, by direct cloning of VHH genes from B-cells of an immunized camelid resulting in recombinant VHHs, or from naive or synthetic libraries. VHHs with desired antigen specificity may also be obtained through phage display methodology. Using VHHs in phage display is much simpler and more efficient compared to Fabs or scFvs, since only one domain needs to be cloned and expressed to obtain a functional antigen-binding fragment. Muyldermans, Biotechnol. 74:277-302 (2001); Ghahroudi et al., FEBS Lett. 414:521-526 (1997); and van der Linden et al., J. Biotechnol. 80:261-270 (2000). Methods for generating antibodies having camelid heavy chains are also described in U.S. Patent Publication Nos. 20050136049 and 20050037421.

Ribosome display methods may be used to identify and isolate scFv and/or VHH molecules having the desired binding activity and affinity. Irving et al., J. Immunol. Methods 248:31-45 (2001). Ribosome display and selection has the potential to generate and display large libraries (10¹⁴).

Other embodiments provide V_{HH}-like molecules generated through the process of camelisation, by modifying non-Camelidae V_{H}s, such as human V_{HH}s, to improve their solubility and prevent non-specific binding. This is achieved by replacing residues on the V_{L}s side of V_{H}s with V_{HH}-like residues, thereby mimicking the more soluble V_{HH} fragments. Camelised V_{H} fragments, particularly those based on the human framework, are expected to exhibit a greatly reduced immune response when administered *in vivo* to a patient and, accordingly, are expected to have significant advantages for therapeutic applications. Davies et al., FEBS Lett. 339:285-290 (1994); Davies et al., Protein Eng. 9:531-537 (1996); Tanha et al., J. Biol. Chem. 276:24774-24780 (2001); and Riechmann et al., Immunol. Methods 231:25-38 (1999).

A wide variety of expression systems are available for the production of IL-1β binding fragments including Fab fragments, scFv, and VHHs. For example, expression systems of both prokaryotic and eukaryotic origin may be used for the large-scale production of antibody fragments and antibody fusion proteins. Particularly advantageous are expression systems that permit the secretion of large amounts of antibody fragments into the culture medium.

Production of bispecific Fab-scFv ("bibody") and trispecific Fab-(scFv)(2) ("tribody") are described in Schoonjans et al. (J Immunol. 165:7050-57, 2000) and Willems et al. (J Chromatogr B Analyt Technol Biomed Life Sci. 786:161-76, 2003). For bibodies or tribodies, a scFv molecule is fused to one or both of the VL-CL (L) and VH-CH1 (Fd) chains, e.g., to produce a tribody two scFvs are fused to C-term of Fab while in a bibody one scFv is fused to C-term of Fab. A "minibody" consisting of scFv fused to CH3 via a peptide linker (hingeless) or via an IgG hinge has been described in Olafsen, et al., Protein Eng Des Scl. 2004 Apr;17(4):315-23.

Intrabodies are single chain antibodies which demonstrate intracellular expression and can manipulate intracellular protein function (Biocca, et al., EMBO J. 9:101-108, 1990; Colby et al., Proc Natl Acad Sci USA 101:17616-21, 2004). Intrabodies, which comprise cell signal sequences which retain the antibody construct in intracellular regions, may be produced as described in Mhashilkar et al (EMBO J 14:1542-51, 1995) and Wheeler et al. (FASEB J. 17:1733-5. 2003). Transbodies are cell-permeable antibodies in which a protein transduction domains (PTD) is fused with single chain variable fragment (scFv) antibodies Heng et al., (Med Hypotheses. 64:1105-8, 2005).

The anti-IL-1β antibodies or binding fragments thereof also encompass antibodies that are SMIPs or binding domain immunoglobulin fusion proteins specific for target protein. These constructs are single-chain polypeptides comprising antigen binding domains fused to immunoglobulin domains necessary to carry out antibody effector functions. See *e.g.,* WO 03/041600, U.S. Patent Publication 2003/0133939 and U.S. Patent Publication 2003/0118592.

The anti-IL-1β antibodies or binding fragments thereof of the present disclosure also encompass immunoadhesins. One or more CDRs may be incorporated into a molecule either covalently or noncovalently to make it an immunoadhesin. An immunoadhesin may incorporate the CDR(s) as part of a larger polypeptide chain, may covalently link the CDR(s) to another polypeptide chain, or may incorporate the CDR(s) noncovalently. The CDRs disclosed herein permit the immunoadhesin to specifically bind to IL-1β.

The anti-IL-1β antibodies or binding fragments thereof also encompass antibody mimics comprising one or more IL-1β binding portions built on an organic or molecular scaffold (such as a protein or carbohydrate scaffold). Proteins having relatively defined three-dimensional structures, commonly referred to as protein scaffolds, may be used as reagents for the design of antibody mimics. These scaffolds typically contain one or more regions which are amenable to specific or random sequence variation, and such sequence randomization is often carried out to produce libraries of proteins from which desired products may be selected. For example, an antibody mimic can comprise a chimeric non-immunoglobulin binding polypeptide having an immunoglobulin-like domain containing scaffold having two or more solvent exposed loops containing a different CDR from a parent antibody inserted into each of the loops and exhibiting selective binding activity toward a ligand bound by the parent antibody. Non-immunoglobulin protein scaffolds have been proposed for obtaining proteins with novel binding properties. (Tramontano et al., J. Mol. Recognit. 7:9, 1994; McConnell and Hoess, J. Mol. Biol. 250:460, 1995). Other proteins have been tested as frameworks and have been used to display randomized residues on alpha helical surfaces (Nord et al., Nat. Biotechnol. 15:772, 1997; Nord et al., Protein Eng. 8:601, 1995), loops between alpha helices in alpha helix bundles (Ku and Schultz, Proc. Natl. Acad. Sci. USA 92:6552, 1995), and loops constrained by disulfide bridges, such as those of the small protease inhibitors (Markland et al., Biochemistry 35:8045, 1996; Markland et al., Biochemistry 35:8058, 1996; Rottgen and Collins, Gene 164:243, 1995; Wang et al., J. Biol. Chem. 270:12250, 1995). Methods for employing scaffolds for antibody mimics are disclosed in US Patent 5,770,380 and US Patent Publications 2004/0171116, 2004/0266993, and 2005/0038229.

Preferred IL-1β antibodies or antibody fragments for use in accordance with the invention generally bind to human IL-1β with high affinity (*e.g.,* as determined with BIACORE, as determined by KinExA), such as for example with an equilibrium binding dissociation constant (KD) for IL-1β of about 1 nM or less, about 500 pM or less, about 250 pM or less, about 100 pM or less, about 50 pM or less, about 25 pM or less, about 10 pM or less, about 5 pM or less, about 3 pM or less about 1 pM or less, about 0.75 pM or less, about 0.5 pM or less, or about 0.3 pM or less. The dissociation constant may be measured, for example, using Biacore (GE Healthcare), and measurement using Biacore may be preferred when the dissociation constant is greater than about 10 pM. Alternatively or in addition, the dissociation constant may be measured using KinExA (Sapidyne Instruments, Inc), and measurement using KinExA may be preferred when the dissociation constant is less than about 10 pM.

Antibodies or fragments of the present invention may, for example, bind to IL-1β with an IC50 of about 10 nM or less, about 5 nM or less, about 2 nM or less, about 1 nM or less, about 0.75 nM or less, about 0.5 nM or less, about 0.4 nM or less, about 0.3 nM or less, or even about 0.2 nM or less, as determined by enzyme linked immunosorbent assay (ELISA). The present antibodies and fragments may bind to IL-1β, but do not detectably bind to IL-1α, or have at least about 100 times (*e.g*., at least about 150 times, at least about 200 times, or even at least about 250 times) greater selectivity in its binding of IL-1β relative to its binding of IL-1α. Antibodies or fragments used according to the invention may, in certain embodiments, inhibit IL-1β induced expression of serum IL-6 in an animal by at least 50% (*e.g.,* at least 60%, at least 70%, or even at least 80%) as compared to the level of serum IL-6 in an IL-1β stimulated animal that has not been administered an antibody or fragment of the invention. Antibodies may bind IL-1β but permit or substantially permit the binding of the bound IL-1β ligand to IL-1 receptor type I (IL-1RI). In contrast to many known anti-IL-1β antibodies or binding fragments thereof that block or substantially interfere with binding of IL-1β to IL-1RI, the antibodies designated AB5 and AB7 (U.S. 7,531,166) selectively bind to the IL-1β ligand, but permit the binding of the bound IL-1β ligand to IL-1RI. For example, the antibody designated AB7 binds to an IL-1β epitope but still permits the bound IL-1β to bind to IL-1RI. In certain embodiments, the antibody may decrease the affinity of interaction of bound IL-1β to bind to IL-1RI. Accordingly, the disclosure provides, in a related aspect, use of an anti-IL-1β antibody or binding fragment thereof that has at least one of the aforementioned characteristics. Any of the foregoing antibodies, antibody fragments, or polypeptides of the invention can be humanized or human engineered, as described herein.

A variety of IL-1β antibodies and fragments known in the art may be used as provided by the disclosure herein, including for example antibodies and antibody binding fragments (*e.g.*, V-region sequences) described in, or derived using methods described in the following patents and patent applications: US 4,935,343; US 2003/0026806; US 2003/0124617 (*e.g.,* antibody AAL160); U.S. 7,566,772 (*e.g.,* antibody 9.5.2); WO 03/034984; WO 95/01997 (*e.g.,* antibody SK48-E26 VTKY); U.S. 7,446,175 (*e.g.,* antibody ACZ 885); WO 03/010282 (*e.g.,* antibody Hu007); WO 03/073982 (*e.g.,* antibody N55S), U.S. 7,541,033 (*e.g*., W17, U43, W13, W18, W20), U.S. 7,491,392, WO 2004/072116, WO 2004/067568, EP 0 267 611 B1, EP 0 364 778 B1, and US application number 11/472813. As a non-limiting example, antibodies AB5 and AB7 (U.S. 7,531,166) may be used in accordance with the invention. Variable region sequences of AB5 and AB7 (also referred to as XOMA 052 or gevokizumab) are as follows:

### AB5

### LIGHT CHAIN

The underlined sequences depict (from left to right) CDR1, 2 and 3.

### HEAVY CHAIN

The underlined sequences depict (from left to right) CDR1, 2 and 3.

### AB7

### LIGHT CHAIN

The underlined sequences depict (from left to right) CDR1, 2 and 3.

### HEAVY CHAIN

The underlined sequences depict (from left to right) CDR1, 2 and 3.

The anti-IL-1β antibodies or binding fragments thereof of the present disclosure include anti-IL-1β antibodies or fragments thereof that are non-competitive (*e.g.*, allosteric) IL-1β antagonists.

The anti-IL-1β antibodies and fragments thereof of the present disclosure are well-tolerated as shown by lack or reduction of drug related serious adverse events (*e.g.,* may be considered safe or may be said to have a favorable safety profile).

The anti-IL-1β antibodies or binding fragments thereof of the present disclosure include anti-IL-1β antibodies or fragments thereof which have high affinity (for example, less than 3 pM, alternatively about 1 pM or less) compared to known anti-IL-1β antibodies. Exemplary antibodies include the antibodies designated AB5, AB6, AB7, AB8, and AB9 herein. AB5 comprises a heavy chain variable region comprising the amino acid sequence of SEQ ID NO: 4 and a light chain variable region comprising the amino acid sequence of SEQ ID NO: 3. AB6 comprises a heavy chain variable region comprising the amino acid sequence of SEQ ID NO: 8 and a light chain variable region comprising the amino acid sequence of SEQ ID NO: 7. AB7 comprises a heavy chain variable region comprising the amino acid sequence of SEQ ID NO: 6 and a light chain variable region comprising the amino acid sequence of SEQ ID NO: 5. AB8 comprises a heavy chain variable region comprising the amino acid sequence of SEQ ID NO: 10 and a light chain variable region comprising the amino acid sequence of SEQ ID NO: 9. AB9 comprises a heavy chain variable region comprising the amino acid sequence of SEQ ID NO: 12 and a light chain variable region comprising the amino acid sequence of SEQ ID NO: 11.

Additional exemplary antibodies include the antibodies designated AB5, AB6, AB7, AB8, and AB9 herein including, such AB5, AB6, AB7, AB8, and AB9 antibodies with one or more conservative amino acid substitutions. AB5 may comprise a heavy chain variable region comprising the amino acid sequence of SEQ ID NO: 4 optionally with one or more conservative amino acid substitutions and a light chain variable region comprising the amino acid sequence of SEQ ID NO: 3 optionally with one or more conservative amino acid substitutions. AB6 may comprise a heavy chain variable region comprising the amino acid sequence of SEQ ID NO: 8 optionally with one or more conservative amino acid substitutions and a light chain variable region comprising the amino acid sequence of SEQ ID NO: 7 optionally with one or more conservative amino acid substitutions. AB7 may comprise a heavy chain variable region comprising the amino acid sequence of SEQ ID NO: 6 optionally with one or more conservative amino acid substitutions and a light chain variable region comprising the amino acid sequence of SEQ ID NO: 5 optionally with one or more conservative amino acid substitutions. AB8 may comprise a heavy chain variable region comprising the amino acid sequence of SEQ ID NO: 10 optionally with one or more conservative amino acid substitutions and a light chain variable region comprising the amino acid sequence of SEQ ID NO: 9 optionally with one or more conservative amino acid substitutions. AB9 may comprise a heavy chain variable region comprising the amino acid sequence of SEQ ID NO: 12 optionally with one or more conservative amino acid substitutions and a light chain variable region comprising the amino acid sequence of SEQ ID NO: 11 optionally with one or more conservative amino acid substitutions.

Anti-IL-1β antibodies or binding fragments thereof may comprise one of the heavy chain variable regions of the sequences set forth in SEQ ID NO: 4, 6, 8, 10, or 12, and one of the light chain variable regions of the sequences set forth in SEQ ID NO: 3, 5, 7, 9, or 11.

Furthermore, the IL-1β antibodies and fragments of the present disclosure encompass any of the foregoing amino acid sequences of the light or heavy chains with one or more conservative substitutions (*e.g.,* 1, 2, 3, 4, 5, 6, 7, 8, 9, 10, 11, 12, 13, 14, or 15 conservative substitutions). In light of the present disclosure, one can determine the positions of an amino acid sequence that are candidates for conservative substitutions, and one can select synthetic and naturally-occurring amino acids that effect conservative substitutions for any particular amino acids. Consideration for selecting conservative substitutions include the context in which any particular amino acid substitution is made, the hydrophobicity or polarity of the side-chain, the general size of the side chain, and the pK value of side-chains with acidic or basic character under physiological conditions. For example, lysine, arginine, and histidine are often suitably substituted for each other. As is known in the art, this is because all three amino acids have basic side chains, whereas the pK value for the side-chains of lysine and arginine are much closer to each other (about 10 and 12) than to histidine (about 6). Similarly, glycine, alanine, valine, leucine, and isoleucine are often suitably substituted for each other, with the proviso that glycine is frequently not suitably substituted for the other members of the group. This is because each of these amino acids are relatively hydrophobic when incorporated into a polypeptide, but glycine's lack of an α-carbon allows the phi and psi angles of rotation (around the α-carbon) so much conformational freedom that glycinyl residues can trigger changes in conformation or secondary structure that do not often occur when the other amino acids are substituted for each other. Other groups of amino acids frequently suitably substituted for each other include, but are not limited to, the group consisting of glutamic and aspartic acids; the group consisting of phenylalanine, tyrosine, and tryptophan; and the group consisting of serine, threonine, and, optionally, tyrosine.

By making conservative modifications to the amino acid sequence or corresponding modifications to the encoding nucleotides, one can produce anti-IL-1β antibodies or binding fragments thereof having functional and chemical characteristics similar to those of the exemplary antibodies and fragments disclosed herein. In contrast, substantial modifications in the functional and/or chemical characteristics of anti-IL-1β antibodies or binding fragments thereof may be accomplished by selecting substitutions that differ significantly in their effect on maintaining (a) the structure of the molecular backbone in the area of the substitution, for example, as a sheet or helical conformation, (b) the charge or hydrophobicity of the molecule at the target site, or (c) the bulk of the side chain.

Conservative modifications include modifications made to an amino acid sequence (or its corresponding DNA) that do not significantly affect or alter the binding characteristics of the antibody containing the amino acid sequence. Such conservative modifications include amino acid substitutions, additions and deletions. Modifications can be introduced into an anti-IL-1β antibody or binding fragment thereof by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (*e.g*., lysine, arginine, histidine), acidic side chains (*e.g.,* aspartic acid, glutamic acid), uncharged polar side chains (*e.g.,* glycine, asparagine, glutamine, serine, threonine, tyrosine, cysteine, tryptophan), nonpolar side chains (*e.g.*, alanine, valine, leucine, isoleucine, proline, phenylalanine, methionine), beta-branched side chains (*e.g.,* threonine, valine, isoleucine) and aromatic side chains (*e.g.,* tyrosine, phenylalanine, tryptophan, histidine). Thus, one or more amino acid residues within the CDR and/or frameworks regions of an anti-IL-1β antibody or binding fragment thereof can be replaced with other amino acid residues from the same side chain family and the altered antibody can be tested for retained function.

Anti-IL-1β antibodies or binding fragments thereof may comprise a heavy chain variable region that comprises a heavy chain complementarity determining region 1 (HCDR1) comprising TSGMGVG (SEQ ID NO: 13), a heavy chain complementarity determining region 2 (HCDR2) comprising HIWWDGDESYNPSLK (SEQ ID NO: 14), and/or a heavy chain complementarity determining region 3 (HCDR3) comprising NRYDPPWFVD (SEQ ID NO: 15); and/or a light chain complementarity determining region 1 (LCDR1) comprising RASQDISNYLS (SEQ ID NO: 16), a light chain complementarity determining region 2 (LCDR2) comprising YTSKLHS (SEQ ID NO: 17), and/or a light chain complementarity determining region 3 (LCDR3) comprising LQGKMLPWT (SEQ ID NO: 18).

In some embodiments, the anti-IL-1β antibodies or binding fragments thereof disclosed herein may comprises a heavy chain framework 1 region (HFW1) comprising QVTLKESGPGILKPSOTLSLTCSFSGFSLS (SEQ ID NO: 19), a heavy chain framework 2 region (HFW2) comprising WIRQPSGKGLEWLA (SEQ ID NO: 20), a heavy chain framework 3 region (HFW3) comprising TQLTISKDTSRNQVFLKITSVDTVDTATYFCAR (SEQ ID NO: 21), a heavy chain framework 4 region (HFW4) comprising WGQGTLVTVSS (SEQ ID NO: 22); and/or a light chain framework 1 region (LFW1) comprising DIQMTQTTSSLSASLGDRVTISC (SEQ ID NO: 23), a light chain framework 2 region (LFW2) comprising WYQQKPDGTVKLLIY (SEQ ID NO: 24), a light chain framework 3 region (LFW3) comprising GVPSRFSGSGSGTDYSLTISNLEQEDIATYFC (SEQ ID NO: 25), and/or a light chain framework 4 region (LFW4) comprising FGGGTKLEIK (SEQ ID NO: 26). In alternative embodiments, the anti-IL-1β antibodies or binding fragments thereof disclosed herein may comprises a heavy chain framework 1 region (HFW1) comprising QVQLQESGPGLVKPSQTLSLTCSFSGFSLS (SEQ ID NO: 27), a heavy chain framework 2 region WIRQPSGKGLEWLA (HFW2) comprising (SEQ ID NO: 28), a heavy chain framework 3 region (HFW3) comprising SRLTISKDTSKNQVSLKITSVTAADTAVYFCAR (SEQ ID NO: 29), a heavy chain framework 4 region (HFW4) comprising WGQGTLVTVSS (SEQ ID NO: 30); and/or a light chain framework 1 region (LFW1) comprising DIQMTQSTSSLSASVGDRVTITC (SEQ ID NO: 31), a light chain framework 2 region (LFW2) comprising WYQQKPGKAVKLLIY (SEQ ID NO: 32), a light chain framework 3 region (LFW3) comprising GVPSRFSGSGSGTDYTLTISSLQQEDFATYFC (SEQ ID NO: 33), and/or a light chain framework 4 region (LFW4) comprising FGQGTKLEIK (SEQ ID NO: 34).

Anti-IL-1β antibodies or binding fragments thereof may comprise a heavy chain variable region that comprises a heavy chain complementarity determining region 1 (HCDR1) comprising TSGMGVG (SEQ ID NO: 13), a heavy chain complementarity determining region 2 (HCDR2) comprising HIWWDGDESYNPSLK (SEQ ID NO: 14), and/or a heavy chain complementarity determining region 3 (HCDR3) comprising NRYDPPWFVD (SEQ ID NO: 15); and/or a light chain complementarity determining region 1 (LCDR1) comprising RASQDISNYLS (SEQ ID NO: 16), a light chain complementarity determining region 2 (LCDR2) comprising YTSKLHS (SEQ ID NO: 17), and/or a light chain complementarity determining region 3 (LCDR3) comprising LQGKMLPWT (SEQ ID NO: 18); and/or a heavy chain framework 1 region (HFW1) comprising QVTLKESGPGILKPSQTLSLTCSFSGFSLS (SEQ ID NO: 19) or QVQLQESGPGLVKPSQTLSLTCSFSGFSLS (SEQ ID NO: 27), a heavy chain framework 2 region (HFW2) comprising WIRQPSGKGLEWLA (SEQ ID NO: 20) or WIRQPSGKGLEWLA (SEQ ID NO: 28), a heavy chain framework 3 region (HFW3) comprising TQLTISKDTSRNQVFLKITSVDTVDTATYFCAR (SEQ ID NO: 21) or SRLTISKDTSKNQVSLKITSVTAADTAVYFCAR (SEQ ID NO: 29), a heavy chain framework 4 region (HFW4) comprising WGQGTLVTVSS (SEQ ID NO: 22) or WGQGTLVTVSS (SEQ ID NO: 30); and/or a light chain framework 1 region (LFW1) comprising DIQMTQTTSSLSASLGDRVTISC (SEQ ID NO: 23) or DIQMTQSTSSLSASVGDRVTITC (SEQ ID NO: 31), a light chain framework 2 region (LFW2) comprising WYQQKPDGTVKLLIY (SEQ ID NO: 24) or WYQQKPGKAVKLLIY (SEQ ID NO: 32), a light chain framework 3 region (LFW3) comprising GVPSRFSGSGSGTDYSLTISNLEQEDIATYFC (SEQ ID NO: 25) or GVPSRFSGSGSGTDYTLTISSLQQEDFATYFC (SEQ ID NO: 33), and/or a light chain framework 4 region (LFW4) comprising FGGGTKLEIK (SEQ ID NO: 26) or FGQGTKLEIK (SEQ ID NO: 34).

Anti-IL-1β antibodies or binding fragments thereof may comprise a heavy chain variable region that comprises a HCDR1, a HCDR2, and a HCDR3, wherein i) the HCDR2 comprises an aspartic acid residue at a position corresponding to position 56 (Asp56) of the AB7 heavy chain variable region sequence set forth in SEQ ID NO: 6, an aspartic acid residue at a position corresponding to position 58 (Asp58) of the AB7 heavy chain variable region sequence set forth in SEQ ID NO: 6, a glutamic acid residue at a position corresponding to position 59 (Glu59) of the AB7 heavy chain variable region sequence set forth in SEQ ID NO: 6, and/or a serine residue at a position corresponding to position 60 (Ser60) of the AB7 heavy chain variable region sequence set forth in SEQ ID NO: 6, and/or ii) the HCDR3 comprises an aspartic acid residue at a position corresponding to position 100 (Asp100) of the AB7 heavy chain variable region sequence set forth in SEQ ID NO: 6.

Anti-IL-1β antibodies or binding fragments thereof may comprise a light chain variable region that comprises a LCDR1, a LCDR2, and a LCDR3, wherein i) the LCDR1 comprises a glutamine residue at a position corresponding to position 27 (Gln27) of the AB7 light chain variable region sequence set forth in SEQ ID NO: 5, a tyrosine residue at a position corresponding to position 32 (Tyr32) of the AB7 light chain variable region sequence set forth in SEQ ID NO: 5, ii) the LCDR2 comprises a tyrosine residue at a position corresponding to position 50 (Tyr50) of the AB7 light chain variable region sequence set forth in SEQ ID NO: 5, and/or iii) the LCDR3 comprises a glycine residue at a position corresponding to position 91 (Gly91) of the AB7 light chain variable region sequence set forth in SEQ ID NO: 5, a lysine residue at a position corresponding to position 92 (Lys92) of the AB7 light chain variable region sequence set forth in SEQ ID NO: 5, and/or a leucine residue at a position corresponding to position 94 (Leu94) of the AB7 light chain variable region sequence set forth in SEQ ID NO: 5.

Anti-IL-1β antibodies or binding fragments thereof may comprise a heavy chain variable region that comprises a HCDR1, a HCDR2, and a HCDR3, wherein i) the HCDR2 comprises an aspartic acid residue at a position corresponding to position 56 (Asp56) of the AB7 heavy chain variable region sequence set forth in SEQ ID NO: 6, an aspartic acid residue at a position corresponding to position 58 (Asp58) of the AB7 heavy chain variable region sequence set forth in SEQ ID NO: 6, a glutamic acid residue at a position corresponding to position 59 (Glu59) of the AB7 heavy chain variable region sequence set forth in SEQ ID NO: 6, and/or a serine residue at a position corresponding to position 60 (Ser60) of the AB7 heavy chain variable region sequence set forth in SEQ ID NO: 6, and/or ii) the HCDR3 comprises an aspartic acid residue at a position corresponding to position 100 (Asp100) of the AB7 heavy chain variable region sequence set forth in SEQ ID NO: 6; and/or a light chain variable region that comprises a LCDR1, a LCDR2, and a LCDR3, wherein i) the LCDR1 comprises a glutamine residue at a position corresponding to position 27 (Gln27) of the AB7 light chain variable region sequence set forth in SEQ ID NO: 5, a tyrosine residue at a position corresponding to position 32 (Tyr32) of the AB7 light chain variable region sequence set forth in SEQ ID NO: 5, ii) the LCDR2 comprises a tyrosine residue at a position corresponding to position 50 (Tyr50) of the AB7 light chain variable region sequence set forth in SEQ ID NO: 5, and/or iii) the LCDR3 comprises a glycine residue at a position corresponding to position 91 (Gly91) of the AB7 light chain variable region sequence set forth in SEQ ID NO: 5, a lysine residue at a position corresponding to position 92 (Lys92) of the AB7 light chain variable region sequence set forth in SEQ ID NO: 5, and/or a leucine residue at a position corresponding to position 94 (Leu94) of the AB7 light chain variable region sequence set forth in SEQ ID NO: 5.

The antibodies and antibody fragments described herein can be prepared by any suitable method. Suitable methods for preparing such antibodies and antibody fragments are known in the art. Other methods for preparing the antibodies and antibody fragments are as described herein as part of the invention. The antibody, antibody fragment, or polypeptide of the invention, as described herein, can be isolated or purified to any degree. As used herein, an isolated compound is a compound that has been removed from its natural environment. A purified compound is a compound that has been increased in purity, such that the compound exists in a form that is more pure than it exists (i) in its natural environment or (ii) when initially synthesized and/or amplified under laboratory conditions, wherein "purity" is a relative term and does not necessarily mean "absolute purity."

### Compositions

IL-1β binding antibodies and binding fragments can be formulated in compositions, especially pharmaceutical compositions, for use in the methods disclosed herein. Such compositions comprise an amount (*e.g*., a therapeutically or prophylactically effective amount) of an anti-IL-1β antibody or binding fragment thereof in admixture with a suitable carrier, *e.g*., a pharmaceutically acceptable agent. Typically, anti-IL-1β antibodies or binding fragments thereof are sufficiently purified for administration to an animal (*e.g.*, human) before formulation in a pharmaceutical composition.

Pharmaceutically acceptable agents include for example, carriers, excipients, diluents, antioxidants, preservatives, coloring, flavoring and diluting agents, emulsifying agents, suspending agents, solvents, fillers, bulking agents, buffers, delivery vehicles, tonicity agents, cosolvents, wetting agents, complexing agents, buffering agents, antimicrobials, and surfactants.

Neutral buffered saline or saline mixed with albumin are exemplary appropriate carriers. The pharmaceutical compositions can include antioxidants such as ascorbic acid; low molecular weight polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween, pluronics, or polyethylene glycol (PEG). Also by way of example, suitable tonicity enhancing agents include alkali metal halides (preferably sodium or potassium chloride), mannitol, sorbitol, and the like. Suitable preservatives include benzalkonium chloride, thimerosal, phenethyl alcohol, methylparaben, propylparaben, chlorhexidine, sorbic acid and the like. Hydrogen peroxide also can be used as preservative. Suitable cosolvents include glycerin, propylene glycol, and PEG. Suitable complexing agents include caffeine, polyvinylpyrrolidone, beta-cyclodextrin or hydroxy-propyl-beta-cyclodextrin. Suitable surfactants or wetting agents include sorbitan esters, polysorbates such as polysorbate 80, tromethamine, lecithin, cholesterol, tyloxapal, and the like. The buffers can be conventional buffers such as acetate, borate, citrate, phosphate, bicarbonate, or Tris-HCl. Acetate buffer may be about pH 4-5.5, and Tris buffer can be about pH 7-8.5. Additional pharmaceutical agents are set forth in Remington's Pharmaceutical Sciences, 18th Edition, A. R. Gennaro, ed., Mack Publishing Company, 1990.

The composition can be in liquid form or in a lyophilized or freeze-dried form and may include one or more lyoprotectants, excipients, surfactants, high molecular weight structural additives and/or bulking agents (see for example US Patents 6,685,940, 6,566,329, and 6,372,716). In one embodiment, a lyoprotectant is included, which is a non-reducing sugar such as sucrose, lactose or trehalose. The amount of lyoprotectant generally included is such that, upon reconstitution, the resulting formulation will be isotonic, although hypertonic or slightly hypotonic formulations also may be suitable. In addition, the amount of lyoprotectant should be sufficient to prevent an unacceptable amount of degradation and/or aggregation of the protein upon lyophilization. Exemplary lyoprotectant concentrations for sugars (*e.g.*, sucrose, lactose, trehalose) in the pre-lyophilized formulation are from about 10 mM to about 400 mM. In another embodiment, a surfactant is included, such as for example, nonionic surfactants and ionic surfactants such as polysorbates (*e.g.* polysorbate 20, polysorbate 80); poloxamers (*e.g.* poloxamer 188); poly (ethylene glycol) phenyl ethers (*e.g.* Triton); sodium dodecyl sulfate (SDS); sodium laurel sulfate; sodium octyl glycoside; lauryl-, myristyl-, linoleyl-, or stearyl-sulfobetaine; lauryl-, myristyl-, linoleyl- or stearyl-sarcosine; linoleyl-, myristyl-, or cetyl-betaine; lauroamidopropyl-, cocamidopropyl-, linoleamidopropyl-, myristamidopropyl-, palmidopropyl-, or isostearamidopropyl-betaine (*e.g.* lauroamidopropyl); myristarnidopropyl-, palmidopropyl-, or isostearamidopropyl-dimethylamine; sodium methyl cocoyl-, or disodium methyl ofeyl-taurate; and the MONAQUAT™ series (Mona Industries, Inc., Paterson, N.J.), polyethyl glycol, polypropyl glycol, and copolymers of ethylene and propylene glycol (*e.g.* Pluronics, PF68, *etc*). Exemplary amounts of surfactant that may be present in the pre-lyophilized formulation are from about 0.001-0.5%. High molecular weight structural additives (*e.g.* fillers, binders) may include for example, acacia, albumin, alginic acid, calcium phosphate (dibasic), cellulose, carboxymethylcellulose, carboxymethylcellulose sodium, hydroxyethylcellulose, hydroxypropylcellulose, hydroxypropylmethylcellulose, microcrystalline cellulose, dextran, dextrin, dextrates, sucrose, tylose, pregelatinized starch, calcium sulfate, amylose, glycine, bentonite, maltose, sorbitol, ethylcellulose, disodium hydrogen phosphate, disodium phosphate, disodium pyrosulfite, polyvinyl alcohol, gelatin, glucose, guar gum, liquid glucose, compressible sugar, magnesium aluminum silicate, maltodextrin, polyethylene oxide, polymethacrylates, povidone, sodium alginate, tragacanth microcrystalline cellulose, starch, and zein. Exemplary concentrations of high molecular weight structural additives are from 0.1% to 10% by weight. In other embodiments, a bulking agent (*e.g*., mannitol, glycine) may be included.

Compositions can be suitable for parenteral administration. Exemplary compositions are suitable for injection or infusion into an animal by any route available to the skilled worker, such as intraarticular, subcutaneous, intravenous, intramuscular, intraperitoneal, intracerebral (intraparenchymal), intracerebroventricular, intramuscular, intraocular, intraarterial, intralesional, intrarectal, transdermal, oral, and inhaled routes. A parenteral formulation typically will be a sterile, pyrogen-free, isotonic aqueous solution, optionally containing pharmaceutically acceptable preservatives.

Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringers' dextrose, dextrose and sodium chloride, lactated Ringer's, or fixed oils. Intravenous vehicles include fluid and nutrient replenishers, electrolyte replenishers, such as those based on Ringer's dextrose, and the like. Preservatives and other additives may also be present, such as, for example, anti-microbials, anti-oxidants, chelating agents, inert gases and the like. See generally, Remington's Pharmaceutical Science, 16th Ed., Mack Eds., 1980, which is incorporated herein by reference.

Pharmaceutical compositions described herein can be formulated for controlled or sustained delivery in a manner that provides local concentration of the product (*e.g.*, bolus, depot effect) sustained release and/or increased stability or half-life in a particular local environment. The invention contemplates that in certain embodiments such compositions may include a significantly larger amount of antibody or fragment in the initial deposit, while the effective amount of antibody or fragment actually released and available at any point in time for is in accordance with the disclosure herein an amount much lower than the initial deposit. The compositions can include the formulation of anti-IL-1β antibodies or binding fragments thereof,, nucleic acids, or vectors of the invention with particulate preparations of polymeric compounds such as polylactic acid, polyglycolic acid, *etc.,* as well as agents such as a biodegradable matrix, injectable microspheres, microcapsular particles, microcapsules, bioerodible particles beads, liposomes, and implantable delivery devices that provide for the controlled or sustained release of the active agent which then can be delivered as a depot injection. Techniques for formulating such sustained- or controlled-delivery means are known and a variety of polymers have been developed and used for the controlled release and delivery of drugs. Such polymers are typically biodegradable and biocompatible. Polymer hydrogels, including those formed by complexation of enantiomeric polymer or polypeptide segments, and hydrogels with temperature or pH sensitive properties, may be desirable for providing drug depot effect because of the mild and aqueous conditions involved in trapping bioactive protein agents (*e.g.*, antibodies). See, for example, the description of controlled release porous polymeric microparticles for the delivery of pharmaceutical compositions in PCT Application Publication WO 93/15722.

Suitable materials for this purpose include polylactides (see, *e.g*., U.S. Patent 3,773,919), polymers of poly-(a-hydroxycarboxylic acids), such as poly-D-(-)-3-hydroxybutyric acid (EP 133,988A), copolymers of L-glutamic acid and gamma ethyl-L-glutamate (Sidman et al., Biopolymers, 22: 547-556 (1983)), poly (2-hydroxyethylmethacrylate) (Langer et al., J. Biomed. Mater. Res., 15: 167-277 (1981), and Langer, Chem. Tech., 12: 98-105 (1982)), ethylene vinyl acetate, or poly-D(-)-3-hydroxybutyric acid. Other biodegradable polymers include poly(lactones), poly(acetals), poly(orthoesters), and poly(orthocarbonates). Sustained-release compositions also may include liposomes, which can be prepared by any of several methods known in the art (see, *e.g.,* Eppstein et al., Proc. Natl. Acad. Sci. USA, 82: 3688-92 (1985)). The carrier itself, or its degradation products, should be nontoxic in the target tissue and should not further aggravate the condition. This can be determined by routine screening in animal models of the target disorder or, if such models are unavailable, in normal animals.

Microencapsulation of recombinant proteins for sustained release has been performed successfully with human growth hormone (rhGH), interferon- (rhIFN--), interleukin-2, and MN rgp120. Johnson et al., Nat. Med., 2:795-799 (1996); Yasuda, Biomed. Ther., 27:1221-1223 (1993); Hora et al., Bio/Technology. 8:755-758 (1990); Cleland, "Design and Production of Single Immunization Vaccines Using Polylactide Polyglycolide Microsphere Systems," in Vaccine Design: The Subunit and Adjuvant Approach, Powell and Newman, eds, (Plenum Press: New York, 1995), pp. 439-462; WO 97/03692, WO 96/40072, WO 96/07399; and U.S. Pat. No. 5,654,010. The sustained-release formulations of these proteins were developed using poly-lactic-coglycolic acid (PLGA) polymer due to its biocompatibility and wide range of biodegradable properties. The degradation products of PLGA, lactic and glycolic acids can be cleared quickly within the human body. Moreover, the degradability of this polymer can be depending on its molecular weight and composition. Lewis, "Controlled release of bioactive agents from lactide/glycolide polymer," in: M. Chasin and R. Langer (Eds.), Biodegradable Polymers as Drug Delivery Systems (Marcel Dekker: New York, 1990), pp. 1-41. Additional examples of sustained release compositions include, for example, EP 58,481A, U.S. Pat. No. 3,887,699, EP 158,277A, Canadian Patent No. 1176565, U. Sidman et al., Biopolymers 22, 547 (1983), R. Langer et al., Chem. Tech. 12, 98 (1982), Sinha et al., J. Control. Release 90, 261 (2003), Zhu et al., Nat. Biotechnol. 18, 24 (2000), and Dai et al., Colloids Surf B Biointerfaces 41, 117 (2005).

Bioadhesive polymers are also contemplated for use in or with compositions of the present invention. Bioadhesives are synthetic and naturally occurring materials able to adhere to biological substrates for extended time periods. For example, Carbopol and polycarbophil are both synthetic cross-linked derivatives of poly(acrylic acid). Bioadhesive delivery systems based on naturally occurring substances include for example hyaluronic acid, also known as hyaluronan. Hyaluronic acid is a naturally occurring mucopolysaccharide consisting of residues of D-glucuronic and N-acetyl-D-glucosamine. Hyaluronic acid is found in the extracellular tissue matrix of vertebrates, including in connective tissues, as well as in synovial fluid and in the vitreous and aqueous humour of the eye. Esterified derivatives of hyaluronic acid have been used to produce microspheres for use in delivery that are biocompatible and biodegrable (see, for example, Cortivo et al., Biomaterials (1991) 12:727-730; European Publication No. 517,565; International Publication No. WO 96/29998; Illum et al., J. Controlled Rel. (1994) 29:133-141). Exemplary hyaluronic acid containing compositions of the present invention comprise a hyaluronic acid ester polymer in an amount of approximately 0.1% to about 40% (w/w) of an anti-IL-1β antibody or binding fragment thereof to hyaluronic acid polymer.

Both biodegradable and non-biodegradable polymeric matrices can be used to deliver compositions in accordance with the invention, and such polymeric matrices may comprise natural or synthetic polymers. Biodegradable matrices are preferred. The period of time over which release occurs is based on selection of the polymer. Typically, release over a period ranging from between a few hours and three to twelve months is most desirable. Exemplary synthetic polymers which can be used to form the biodegradable delivery system include: polymers of lactic acid and glycolic acid, polyamides, polycarbonates, polyalkylenes, polyalkylene glycols, polyalkylene oxides, polyalkylene terepthalates, polyvinyl alcohols, polyvinyl ethers, polyvinyl esters, poly-vinyl halides, polyvinylpyrrolidone, polyglycolides, polysiloxanes, polyanhydrides, polyurethanes and co-polymers thereof, poly(butic acid), poly(valeric acid), alkyl cellulose, hydroxyalkyl celluloses, cellulose ethers, cellulose esters, nitro celluloses, polymers of acrylic and methacrylic esters, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxylethyl cellulose, cellulose triacetate, cellulose sulphate sodium salt, poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), polyethylene, polypropylene, poly(ethylene glycol), poly(ethylene oxide), poly(ethylene terephthalate), poly(vinyl alcohols), polyvinyl acetate, poly vinyl chloride, polystyrene and polyvinylpyrrolidone. Exemplary natural polymers include alginate and other polysaccharides including dextran and cellulose, collagen, chemical derivatives thereof (substitutions, additions of chemical groups, for example, alkyl, alkylene, hydroxylations, oxidations, and other modifications routinely made by those skilled in the art), albumin and other hydrophilic proteins, zein and other prolamines and hydrophobic proteins, copolymers and mixtures thereof. In general, these materials degrade either by enzymatic hydrolysis or exposure to water in vivo, by surface or bulk erosion. The polymer optionally is in the form of a hydrogel (see for example WO 04/009664, WO 05/087201, Sawhney, et al., Macromolecules, 1993, 26, 581-587,) that can absorb up to about 90% of its weight in water and further, optionally is cross-linked with multi-valent ions or other polymers.

Delivery systems also include non-polymer systems that are lipids including sterols such as cholesterol, cholesterol esters and fatty acids or neutral fats such as mono- di- and tri-glycerides; hydrogel release systems; silastic systems; peptide based systems; wax coatings; compressed tablets using conventional binders and excipients; partially fused implants; and the like. Specific examples include, but are not limited to: (a) erosional systems in which the product is contained in a form within a matrix such as those described in U.S. Pat. Nos. 4,452,775, 4,675,189 and 5,736,152 and (b) diffusional systems in which a product permeates at a controlled rate from a polymer such as described in U.S. Pat. Nos. 3,854,480, 5,133,974 and 5,407,686. Liposomes containing the product may be prepared by methods known methods, such as for example (DE 3,218,121; Epstein et al., Proc. Natl. Acad. Sci. USA, 82: 3688-3692 (1985); Hwang et al., Proc. Natl. Acad. Sci. USA, 77: 4030-4034 (1980); EP 52,322; EP 36,676; EP 88,046; EP 143,949; EP 142,641; Japanese patent application 83-118008; U.S. Pat. Nos. 4,485,045 and 4,544,545; and EP 102,324).

A pharmaceutical composition comprising an anti-IL-1β antibody or binding fragment thereof can be formulated for inhalation, such as for example, as a dry powder. Inhalation solutions also can be formulated in a liquefied propellant for aerosol delivery. In yet another formulation, solutions may be nebulized. Additional pharmaceutical composition for pulmonary administration include, those described, for example, in PCT Application Publication WO 94/20069, which discloses pulmonary delivery of chemically modified proteins. For pulmonary delivery, the particle size should be suitable for delivery to the distal lung. For example, the particle size can be from 1 µm to 5 µm; however, larger particles may be used, for example, if each particle is fairly porous.

Certain formulations containing anti-IL-1β antibodies or binding fragments thereof can be administered orally. Formulations administered in this fashion can be formulated with or without those carriers customarily used in the compounding of solid dosage forms such as tablets and capsules. For example, a capsule can be designed to release the active portion of the formulation at the point in the gastrointestinal tract when bioavailability is maximized and pre-systemic degradation is minimized. Additional agents can be included to facilitate absorption of a selective binding agent. Diluents, flavorings, low melting point waxes, vegetable oils, lubricants, suspending agents, tablet disintegrating agents, and binders also can be employed.

Another preparation can involve an effective quantity of an anti-IL-1β antibody or binding fragment thereof in a mixture with non-toxic excipients which are suitable for the manufacture of tablets. By dissolving the tablets in sterile water, or another appropriate vehicle, solutions can be prepared in unit dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Suitable and/or preferred pharmaceutical formulations can be determined in view of the present disclosure and general knowledge of formulation technology, depending upon the intended route of administration, delivery format, and desired dosage. Regardless of the manner of administration, an effective dose can be calculated according to patient body weight, body surface area, or organ size. Further refinement of the calculations for determining the appropriate dosage for treatment involving each of the formulations described herein are routinely made in the art and is within the ambit of tasks routinely performed in the art. Appropriate dosages can be ascertained through use of appropriate dose-response data.

Additional formulations will be evident in light of the present disclosure, including formulations involving anti-IL-1β antibodies or binding fragments thereof in combination with one or more other therapeutic agents. For example, in some formulations, an anti-IL-1β antibody or binding fragment thereof, nucleic acid, or vector of the invention is formulated with a second inhibitor of an IL-1 signaling pathway. Representative second inhibitors include, but are not limited to, antibodies, antibody fragments, peptides, polypeptides, compounds, nucleic acids, vectors and pharmaceutical compositions, such as, for example, those described in U.S. 6899878, U.S. 2003/022869, U.S. 2006/0094663, U.S. 2005/0186615, U.S. 2003/0166069, WO 04/022718, WO 05/084696, and WO 05/019259. For example, a composition may comprise an anti-IL-1β antibody or binding fragment thereof, nucleic acid, or vector of the invention in combination with another anti-IL-1β antibody or binding fragment thereof, or a nucleic acid or vector encoding such an antibody or fragment.

The pharmaceutical compositions can comprise anti-IL-1β antibodies or binding fragments thereof in combination with other active agents (*e.g*., other than anti-IL-1β antibodies or binding fragments thereof). Alternatively, the pharmaceutical compositions can comprise anti-IL-1β antibodies or binding fragments thereof in combination with other pharmaceutical compositions, including, for example, pharmaceutical compositions comprising one or more active agents (*e.g*., other than anti-IL-1β antibodies or binding fragments thereof). Such combinations are those useful for their intended purpose. The combinations which are part of this invention can be IL-1β antibodies and fragments, such as for example those described herein, and at least one additional agent. Examples of active agents that may be used in combination set forth below are illustrative for purposes and not intended to be limited. The combination can also include more than one additional agent, *e.g*., two or three additional agents if the combination is such that the formed composition can perform its intended function.

The disclosure further contemplates that additional pharmaceutical compositions comprising one or more other active agents may be administered separately from the anti-IL-1β antibodies or binding fragments thereof (*e.g*., concurrent treatment regimen, subject receiving concurrent treatment), and such separate administrations may be performed at the same time or at different times, such as for example the same or different days, or different times of the same day. Administration of the other pharmaceutical compositions and/or active agents may be according to standard medical practices known in the art, or the administration may be modified (*e.g*., longer intervals between doses, smaller dosage levels, delayed initiation) when used in conjunction with administration of anti-IL-1β antibodies or binding fragments thereof, such as disclosed herein.

In some embodiments, active agents may include anti-microbial agents including, for example, antibiotics such as a penicillin (*e.g*., penicillin, amoxicillin, benzylpenicillin, ampicillin, augmentin), a polyketide antibiotic (*e.g.*, a macrolide, azithromycin, erythromycin, clarithromycin), a cephalosporin (*e.g.*, cefadroxil, cefixime, cephalexin), a lincosamide (*e.g.,* clindamycin), a quinolone (*e.g.,* ciprofloxacin, levofloxacin, moxifloxacin), a folic acid synthesis inhibitor (*e.g*., a dihydrofolate reductase inhibitor, trimethoprim, dapsone, co-trimoxazole), a tetracycline (*e.g*., tetracycline, minocycline, doxycycline, demeclocycline, oxytetracycline), a rifamycin (*e.g*., rifampicin, rifabutin, rifapentine), a sulfonamide (*e.g*., sulfamethoxazole, sulfacetamide), an aminoglycoside (*e.g.,* neomycin, amikacin, tobramycin), fusidic acid, a polypeptide antibiotic (*e.g.,* bacitracin, polymixin B), a lipopeptide antibiotic (*e.g*., daptomycin), chloramphenicol and mupirocin.

In some embodiments, active agents may include active agents that are used and/or approved for the treatment of acne including, for example, benzoyl peroxide, retinol, retinal, tretinoin, isotretinoin, adapalene, isotretinoin, prednisone, tretinoin, clindamycin, zithromycin, erythromycin, minocycline, or tetracycline.

It is further contemplated that an anti-IL-1β antibody or binding fragment thereof administered to a subject in accordance with the disclosure may be administered in combination (*e.g.*, concurrently) with treatment with at least one additional pharmaceutical composition (*e.g*., comprising an active agent), such as for example any of the aforementioned active agents. In one embodiment, treatment with the at least one active agent is maintained. In another embodiment, treatment with the at least one active agent is reduced (*e.g.,* tapered) or discontinued (*e.g.,* when the subject is stable) during the course of anti-IL-1β antibody or binding fragment thereof treatment (*e.g*., with the anti-IL-1β antibody or binding fragment thereof maintained at a constant dosing regimen). In another embodiment, treatment with the at least one active agent is reduced (*e.g*., tapered) or discontinued (*e.g*., when the subject is stable), and treatment with the anti-IL-1β antibody or binding fragment thereof is reduced (*e.g*., lower dose, less frequent dosing, shorter treatment regimen). In another embodiment, treatment with the at least one active agent is reduced (*e.g.,* tapered) or discontinued (*e.g.,* when the subject is stable), and treatment with the anti-IL-1β antibody or binding fragment thereof is increased (*e.g*., higher dose, more frequent dosing, longer treatment regimen). In yet another embodiment, treatment with the at least one active agent is maintained and treatment with the anti-IL-1β antibody or binding fragment thereof is reduced or discontinued (*e.g.*, lower dose, less frequent dosing, shorter treatment regimen). In yet another embodiment, treatment with the at least one active agent and treatment with the anti-IL-1β antibody or binding fragment thereof are reduced or discontinued (*e.g*., lower dose, less frequent dosing, shorter treatment regimen).

In some embodiments, reducing the treatment with at least one active agent (*e.g*., other than anti-IL-1β antibody or binding fragment thereof) is a reduction in the cumulative amount of active agent administered during a course of treatment. In some embodiments, reducing the treatment with at least one active agent (*e.g*., other than anti-IL-1β antibody or binding fragment thereof) is a reduction in the actual dose amount of active agent administered. In some embodiments, reducing the treatment with at least one active agent provides a reduction in systemic immunosuppression.

The pharmaceutical compositions used in the disclosure may include a therapeutically effective amount or a prophylactically effective amount of the anti-IL-1β antibodies or binding fragments thereof. A therapeutically effective amount refers to an amount effective, at dosages and for periods of time necessary, to achieve the desired therapeutic result. A therapeutically effective amount of the antibody or antibody portion may vary according to factors such as the disease state, age, sex, and weight of the individual, and the ability of the antibody or antibody portion to elicit a desired response in the individual. A therapeutically effective amount is also one in which any toxic or detrimental effects of the antibody or antibody portion are outweighed by the therapeutically beneficial effects. A prophylactically effective amount refers to an amount effective, at dosages and for periods of time necessary, to achieve the desired prophylactic result.

A therapeutically or prophylactically effective amount of a pharmaceutical composition comprising an anti-IL-1β antibody or binding fragment thereof will depend, for example, upon the therapeutic objectives such as the indication for which the composition is being used, the route of administration, and the condition of the subject. Pharmaceutical compositions are administered in a therapeutically or prophylactically effective amount to treat an IL-1 related condition (*e.g*., acne).

### Methods of Use

anti-IL-1β antibodies or binding fragments thereof may be used as disclosed herein for the treatment of acne (*e.g*., moderate and/or severe acne) and/or acne that is not responsive to treatment with conventional therapy (*e.g*., an anti-microbial agent) including, for example, one or more oral anti-acne agents (*e.g*., oral antibiotics and/or oral retinoids or retinoid derivatives such as 13-cis retinoic acid). The present disclosure also contemplates the use of other IL-1 pathway inhibitors, as an alternative or in addition to the anti-IL-1β antibodies or binding fragments thereof. Treatment of acne with an anti-IL-1β antibody or binding fragment thereof, alone or on combination with one or more additional anti-acne agents, may be effective to improve one or more acne parameters selected from the group consisting of: scaling, erythema, itching, burning, and stinging and/or may be effective to reduce size of acne lesions, redness of acne lesions, and/or itchiness of acne lesions. Additionally, treatment of acne with an anti-IL-1β antibody or binding fragment thereof, alone or on combination with one or more additional anti-acne agents, may be effective to reduce a symptom of skin inflammation including, for example, redness, swelling, leukocyte infiltration, and/or lesion development.

In some embodiments, the anti-microbial agent is an antibiotic including, for example, an antibiotic selected from the group consisting of: a penicillin (*e.g*., penicillin, amoxicillin, benzylpenicillin, ampicillin, augmentin), a polyketide antibiotic (*e.g*., a macrolide, azithromycin, erythromycin, clarithromycin), a cephalosporin (*e.g*., cefadroxil, cefixime, cephalexin), a lincosamide (*e.g.,* clindamycin), a quinolone (*e.g.,* ciprofloxacin, levofloxacin, moxifloxacin), a folic acid synthesis inhibitor (*e.g*., a dihydrofolate reductase inhibitor, trimethoprim, dapsone, co-trimoxazole), a tetracycline (*e.g*., tetracycline, minocycline, doxycycline, demeclocycline, oxytetracycline), a rifamycin (*e.g*., rifampicin, rifabutin, rifapentine), a sulfonamide (*e.g*., sulfamethoxazole, sulfacetamide), an aminoglycoside (*e.g*., neomycin, amikacin, tobramycin), fusidic acid, a polypeptide antibiotic (*e.g.,* bacitracin, polymixin B), a lipopeptide antibiotic (*e.g.,* daptomycin), chloramphenicol and mupirocin.

The terms "treatment", "treat" and "treating" as used with respect to methods as described herein refer to eliminating, reducing, suppressing or ameliorating, either temporarily or permanently, either partially or completely, a clinical symptom, manifestation or progression of an event, disease or condition (*e.g*., diagnosed symptom, manifestation or progression of an event, disease or condition), such as, for example, acne (*e*.*g.*, acute acne outbreak). In addition, or alternatively, the terms "treatment", "treat" and "treating" as used herein with respect to the methods as described refer to inhibiting, delaying, suppressing, reducing, treating, eliminating or ameliorating, either temporarily or permanently, either partially or completely, a clinical symptom or manifestation of an event, disease or condition, such as, for example, acne (*e.g*., acute acne outbreak). An acute acne outbreak (*e.g*., attack, episode, event, exacerbation, or flare-up) refers to an occurrence or re-occurrence of one or more clinical symptoms or manifestations (*e.g*., parameters) of acne in a subject. An acute acne outbreak includes, for example, where a subject has previously experienced one or more clinical symptoms or manifestations (*e.g*., parameters) of acne, with an intervening period of clinically significant improvement in one or more acne symptoms (*e.g*., decrease and/or clinical control of symptoms, symptom free interval). In some embodiments the treating is effective to reduce a symptom, sign, and/or condition of acne in a subject by at least about 10% (*e.g.,* 15%, 20%, 25%, 30%, 35%, 40%, 45%, 50%, 55%, 60%, 65%, 70%, 75%, 80%, 85%, 90%, 95%, or 100%) including, as compared to a baseline measurement of the symptom, sign, and/or condition made prior to the treatment. In some embodiments, the treating is effective to improve an assessment (*e.g*., dermatological assessment or quality of life assessment) used to score acne in a subject including, as compared to a baseline assessment made prior to the treatment. Such treating as provided herein need not be absolute to be useful.

The term "in need of treatment" as used herein refers to a judgment made by a caregiver that a patient requires or will benefit from treatment. This judgment is made based on a variety of factors that are in the realm of a caregiver's expertise, but that includes the knowledge that the patient exhibits a clinical symptom or manifestation of acne, or will exhibit a clinical symptom or manifestation of acne.

The term "effective amount" as used herein refers to an amount of a compound (*e.g*., IL-1β antibody), either alone or as a part of a pharmaceutical composition, that is capable of having any detectable, positive effect on any symptom, aspect, parameter or characteristics of a disease state or condition when administered to a subject (*e.g.,* as one or more doses), including, for example, improving an acne parameter as referred to herein. Such effect need not be absolute to be beneficial.

"Conventional therapy" includes use of one or more active agents that are used and/or approved for the treatment of acne that are administered as a topical treatment, an oral treatment or a combination treatment. Topical treatment includes, for example, use of active agents such as benzoyl peroxide, topical retinoids (*e.g*., tretinoin, adapalene, isotretinoin, tazarotene), and/or topical antibiotics (*e.g*., clindamycin, erythromycin, tetracycline). Such topical treatment may include other topical therapies (*e.g*., salicylic acid, sulphur, resorcinol, sodium sulfacetamide, aluminium chloride, zinc, nicotinamide, triethyl citrate/ethyl linoleate, dapsone, taurine bromamine, azelaic acid, sodium sulfacetamide 10%/sulfur 5%) as well as combination topicals (*e.g*., combinations of topical treatments with different mechanisms of action). Oral treatment includes, for example, use of active agents such as oral antibiotics (*e.g*., tetracyclines such as tetracycline, oxytetracycline, doxycycline, lymecycline or less preferably minocycline; cotrimoxazole; quinolones such as ciprofloxacin; aminoglycosides; chloramphenicol; clindamycin; macrolides such as erythromycin and azitromycin; trimethoprim), oral contraceptives (*e.g*., combined oral contraceptives that contain an estrogen such as ethinylestradiol and a progestogen; progestogen; estrogen), and/or oral isotretinoin (*e.g*., ACCUTANE). Combination treatment includes, for example, use of active agents in combination, such as retinoid +/- benzoyl peroxide +/- topical antibiotic; retinoid +/- topical antibiotic or benzoyl peroxide; sulfacetamide/sulfur or topical antibiotic + benzoyl peroxide +/- oral antibiotic(s); sulfacetamide/sulfur or topical antibiotic + benzoyl peroxide +/- oral antibiotic(s) + retinoid. Conventional therapy also includes, for example, use of any agent that is known to be useful and/or effective in the treatment of acne such as agents that improve one or more symptoms, signs, and/or conditions of acne (*e.g*., inflammatory lesion count, non-inflammatory lesion count, total lesion count, proportion of clear or almost clear skin, size of lesions, redness of lesions, and/or itchiness of lesions). Conventional therapy also includes, for example, use of prescription based and/or over-the-counter (OTC) treatments. Common OTC acne products include cleansers, pads, lotions, cover-up products, masks, and facials. Active agents in OTC treatments include benzoyl peroxide, coal tar, resorcinol, sulfur, and salicylic acid. Representative examples of some OTC treatments comprising benzoyl peroxide include Oxy® 5 (5% benzoyl peroxide lotion), Benzoyl® 10 (10% benzoyl peroxide lotion), Benzashave® (5% or 10% benzoyl peroxide cream), Advanced Formula Oxy Sensitive® or Benzac® AC 2.5 or Desquam-E® or PanOxyl AO® 2.5 (2.5% benzoyl peroxide gel), Benzac® 5 or Benzac AC® 5 or 5-Benzagel® or Desquam-E 5® or Desquam-X 5® or PanOxyl AQ® 5 or PanOxyl® 5 (5% benzoyl peroxide gel), Benzac® 10 or Benzac AC® 10 or 10-Benzagel® or Desquam-E® 10 or Desquam-X® 10 or PanOxyl AQ® 10 or PanOxyl® 10 (10% benzoyl peroxide gel). Examples of some OTC treatments comprising salicylic acid include, for instance, Stri-Dex® pads, Fostex® cleansing pads, and Clearasil Maximum Strength® cleansing pads (2% salicylic acid pads). In addition, vitamins and minerals, including zinc, vitamin C and vitamin E, are used in the treatment of acne.

Acne may be considered "not responsive to conventional therapy" or "not responsive to treatment with conventional therapy" when prior treatment of the acne with conventional therapy did not result in an improvement (*e.g.*, a decrease in the number and/or severity of acne lesions) as determined by any medically accepted practices known in the art including, for example, dermatological assessments of the skin, or alternatively or in addition, when prior treatment with conventional therapy caused an adverse reaction (*e.g.*, adverse reaction in a subject which limits use of the conventional therapy). A prior treatment of acne with conventional therapy may be considered to not result in an improvement when the acne failed to respond or partially responded to the conventional therapy (*e.g*., inadequate or partial therapeutic effect, inadequate response, insufficient response, incomplete response, or partial response). In some embodiments, acne is considered not responsive to conventional therapy when prior treatment did not result in an improvement in grade of the subject's skin, as measured by any scoring system used to assess acne including, for example, the IGA scoring system *(see, e.g.,* Table 1) or the facial cutaneous scoring system (*see, e.g.,* Table 2), after the acne has been treated with the conventional therapy. In some embodiments, acne is considered not responsive to conventional therapy when prior treatment did not result in an improvement in a quality of life assessment, as measured by any scoring system used to assess one or more measures of quality of life including, for example, the Cardiff Acne Disability Index (CADI) scoring system (see, *e.g*., Table 3), after the conventional therapy. In some embodiments, acne is considered not responsive to conventional therapy when prior treatment did not result in an improvement in the number and/or severity of acne lesions present on the skin (*e.g*., facial and/or non-facial acne), after conventional therapy. In some embodiments, acne is considered not responsive to conventional therapy when prior treatment did not result in an improvement in at least one assessment (*e.g*., dermatological assessment or quality of life assessment) used to score acne. In such embodiments, acne may be considered not responsive to conventional therapy when the acne exhibits an improvement in one or more assessments used to score acne but fails to result in an improvement in at least one assessment. In some embodiments, an assessment of whether acne is non-responsive to conventional therapy may be made by inquiring from a subject with acne whether their acne was responsive to the conventional therapy (*e.g*., when a subject indicates that their acne did not respond to the conventional therapy the acne is considered non-responsive to the conventional therapy). In some embodiments, an assessment of whether acne is non-responsive to conventional therapy may employ standard medically accepted practices known in the art including, for example, dermatological assessments of the skin and/or quality of life assessments. In such embodiments, such standard medically accepted practices may include an examination of a subject's prior medical history. The terms "not responsive to conventional therapy" or "not responsive to treatment with conventional therapy" as used herein include treatment refractory acne, treatment resistant acne and recalcitrant acne.

A subject with acne may be considered "not responsive to conventional therapy" or "not responsive to treatment with conventional therapy", when prior treatment of the subject with conventional therapy did not result in an improvement (*e.g*., a decrease in the number and/or severity of acne lesions) as determined by any medically accepted practices known in the art including, for example, dermatological assessments of the skin, or alternatively of in addition, when prior treatment of the subject with conventional therapy caused an adverse reaction (*e.g*., adverse reaction in a subject which limits use of the conventional therapy). A prior treatment of a subject with conventional therapy may be considered to not result in an improvement when the subject failed to respond or partially responded to the prior conventional therapy (*e.g*., inadequate or partial therapeutic effect, inadequate response, insufficient response, incomplete response, or partial response). In some embodiments, a subject is considered not responsive to conventional therapy when prior treatment did not result in an improvement in grade of the subject's skin, as measured by any scoring system used to assess acne including, for example, the IGA scoring system (*see, e.g.,* Table 1) or the facial cutaneous scoring system (*see, e.g.,* Table 2), after the subject has been treated with the conventional therapy. In some embodiments, a subject is considered not responsive to conventional therapy when prior treatment did not result in an improvement in a quality of life assessment, as measured by any scoring system used to assess one or more measures of quality of life including, for example, the Cardiff Acne Disability Index (CADI) scoring system (see, *e.g*., Table 3), after the conventional therapy. In some embodiments, a subject is considered not responsive to treatment with conventional therapy when prior treatment did not result in an improvement in the number and/or severity of acne lesions present on the skin (*e.g*., facial and/or non-facial acne), after the subject has been treated with the conventional therapy. In some embodiments, a subject is considered not responsive to treatment with conventional therapy when prior treatment did not result in an improvement in at least one assessment (*e.g*., dermatological assessment or quality of life assessment) used to score acne. In such embodiments, the subject with acne may be considered not responsive to conventional therapy when the subject exhibits an improvement in one or more assessments used to score acne but fails to result in an improvement in at least one assessment. In some embodiments, a subject is considered not responsive to conventional therapy where the subject cannot tolerate the conventional therapy (*e.g*., the conventional therapy is toxic to the subject or the subject exhibits an allergic response to the conventional therapy). In some embodiments, an assessment of whether a subject with acne is non-responsive to conventional therapy may be made by inquiring from the subject with acne whether their acne was responsive to the conventional therapy (*e.g*., when a subject indicates that their acne did not respond to prior conventional therapy they are considered non-responsive to the conventional therapy). In some embodiments, an assessment of whether a subject with acne is non-responsive to conventional therapy may employ standard medically accepted practices known in the art including, for example, dermatological assessments of the skin and/or quality of life assessments. In such embodiments, such standard medically accepted practices may include an examination of a subject's prior medical history. The terms "not responsive to conventional therapy" or "not responsive to treatment with conventional therapy", as used herein include treatment refractory acne, treatment resistant acne and recalcitrant acne.

Acne may be considered "not responsive to treatment with one or more anti-microbial agents" or "not responsive to treatment with an anti-microbial agent", including where the anti-microbial agent is an antibiotic, when prior treatment of the acne with one or more anti-microbial agents (*e.g*., an antibiotic such as an orally administered antibiotic (oral antibiotic), a topically administered antibiotic (topical antibiotic), or an intravenously administered antibiotic (IV antibiotic)) did not result in an improvement (*e.g*., a decrease in the number and/or severity of acne lesions) as determined by any medically accepted practices known in the art including, for example, dermatological assessments of the skin, or alternatively or in addition, when prior treatment of the acne with one or more anti-microbial agents caused an adverse reaction (*e.g*., adverse reaction in a subject which limits use of the anti-microbial agent(s)). A prior treatment of acne with one or more anti-microbial agents may be considered to not result in an improvement when the acne failed to respond or partially responded to the prior anti-microbial treatment (*e.g*., inadequate or partial therapeutic effect, inadequate response, insufficient response, incomplete response, or partial response). In some embodiments, acne is considered not responsive to treatment with an anti-microbial agent when prior treatment did not result in an improvement in grade of the subject's skin, as measured by any scoring system used to assess acne including, for example, the IGA scoring system (*see, e.g.,* Table 1) or the facial cutaneous scoring system (*see, e.g.,* Table 2), after the acne has been treated with the anti-microbial agent. In some embodiments, acne is considered not responsive to treatment with an anti-microbial agent when prior treatment did not result in an improvement in a quality of life assessment, as measured by any scoring system used to assess one or more measures of quality of life including, for example, the Cardiff Acne Disability Index (CADI) scoring system (see, *e.g*., Table 3), after the acne has been treated with the anti-microbial agent. In some embodiments, acne is considered not responsive to treatment with an anti-microbial agent when prior treatment did not result in an improvement in the number and/or severity of acne lesions present on the skin (*e.g*., facial and/or non-facial acne), after the acne has been treated with the anti-microbial agent. In some embodiments, acne is considered not responsive to treatment with an anti-microbial agent when prior treatment did not result in an improvement in at least one assessment (*e.g*., dermatological assessment or quality of life assessment) used to score acne. In such embodiments, acne may be considered not responsive to treatment with an anti-microbial agent when the acne exhibits an improvement in one or more assessments used to score acne but fails to result in an improvement in at least one assessment. In some embodiments, an assessment of whether acne is non-responsive to treatment with an anti-microbial agent may be made by inquiring from a subject with acne whether their acne was responsive to prior treatment with the anti-microbial agent (*e.g*., when a subject indicates that their acne did not respond to prior treatment with an anti-microbial agent the acne is considered non-responsive to the anti-microbial agent). In some embodiments, an assessment of whether acne is non-responsive to treatment with an anti-microbial agent may employ standard medically accepted practices known in the art including, for example, dermatological assessments of the skin and/or quality of life assessments. In such embodiments, such standard medically accepted practices may include an examination of a subject's prior medical history. The terms "not responsive to treatment with one or more anti-microbial agents" and "not responsive to treatment with an anti-microbial agent" as used herein include treatment refractory acne, treatment resistant acne and recalcitrant acne.

A subject with acne may be considered "not responsive to treatment with one or more anti-microbial agents" or "not responsive to treatment with an anti-microbial agent", including where the anti-microbial agent is an antibiotic, when prior treatment of the subject with one or more anti-microbial agents (*e.g*., an antibiotic such as an orally administered antibiotic (oral antibiotic), a topically administered antibiotic (topical antibiotic), or an intravenously administered antibiotic (IV antibiotic)) did not result in an improvement (*e.g.,* a decrease in the number and/or severity of acne lesions) as determined by any medically accepted practices known in the art including, for example, dermatological assessments of the skin, or alternatively of in addition, when prior treatment of the subject with one or more anti-microbial agents caused an adverse reaction (*e.g.*, adverse reaction in a subject which limits use of the anti-microbial agent(s)). A prior treatment of a subject with one or more anti-microbial agents may be considered to not result in an improvement when the subject failed to respond or partially responded to the prior anti-microbial treatment (*e.g*., inadequate or partial therapeutic effect, inadequate response, insufficient response, incomplete response, or partial response). In some embodiments, a subject is considered not responsive to treatment with an anti-microbial agent when prior treatment did not result in an improvement in grade of the subject's skin, as measured by any scoring system used to assess acne including, for example, the IGA scoring system (*see, e.g.,* Table 1) or the facial cutaneous scoring system (*see, e.g.,* Table 2), after the subject has been treated with the anti-microbial agent. In some embodiments, a subject is considered not responsive to treatment with an anti-microbial agent when prior treatment did not result in an improvement in a quality of life assessment, as measured by any scoring system used to assess one or more measures of quality of life including, for example, the Cardiff Acne Disability Index (CADI) scoring system (see, *e.g*., Table 3), after the subject has been treated with the anti-microbial agent. In some embodiments, a subject is considered not responsive to treatment with an anti-microbial agent when prior treatment did not result in an improvement in the number and/or severity of acne lesions present on the skin (*e.g*., facial and/or non-facial acne), after the subject has been treated with the anti-microbial agent. In some embodiments, a subject is considered not responsive to treatment with an anti-microbial agent when prior treatment did not result in an improvement in at least one assessment (*e.g*., dermatological assessment or quality of life assessment) used to score acne. In such embodiments, the subject with acne may be considered not responsive to treatment with an anti-microbial agent when the subject exhibits an improvement in one or more assessments used to score acne but fails to result in an improvement in at least one assessment. In some embodiments, a subject is considered not responsive to treatment with an anti-microbial agent where the subject cannot tolerate treatment with the anti-microbial agent (*e.g*., the anti-microbial agent is toxic to the subject or the subject exhibits an allergic response to the anti-microbial agent). In some embodiments, an assessment of whether a subject with acne is non-responsive to treatment with an anti-microbial agent may be made by inquiring from the subject with acne whether their acne was responsive to prior treatment with the anti-microbial agent (*e.g*., when a subject indicates that their acne did not respond to prior treatment with an anti-microbial agent they are considered non-responsive to the anti-microbial agent). In some embodiments, an assessment of whether a subject with acne is non-responsive to treatment with an anti-microbial agent may employ standard medically accepted practices known in the art including, for example, dermatological assessments of the skin and/or quality of life assessments. In such embodiments, such standard medically accepted practices may include an examination of a subject's prior medical history. The terms "not responsive to treatment with one or more anti-microbial agents" and "not responsive to treatment with an anti-microbial agent" as used herein include treatment refractory acne, treatment resistant acne and recalcitrant acne.

The terms "reduction in the dosage", "reduction in dosage", "dosage reduction" and "reduced dosage" as used herein refers to a change in a prevention or treatment regimen for a pharmaceutical composition, as compared to a previous prevention or treatment regimen for the same pharmaceutical composition (*e.g*., prior to administering an anti-IL-1β antibody or binding fragment thereof). Preferably, such change in prevention or treatment regimen is a decrease in some aspect of the prevention or treatment regimen, such as for example, a decrease (*e.g.,* reduction) in the dose (*e.g.,* amount), a decrease (*e.g.,* reduction) in the frequency of doses, or a decrease (*e.g.,* reduction) in the cumulative exposure (*e.g.,* area under the curve, AUC) over a period of time. Such change in prevention or treatment regimen for a pharmaceutical composition may be a change in a prevention or treatment regimen as compared to a previous prevention or treatment regimen in the same subject, or alternatively or in addition, a change in a prevention or treatment regimen for a pharmaceutical composition when used concurrently with an anti-IL-1β antibody or binding fragment thereof as compared when not used concurrently with an anti-IL-1β antibody or binding fragment thereof.

A variety of methods and techniques for detecting the presence of and/or changes in symptoms, aspects, parameters, signs, conditions, or characteristics of disease states or conditions (*e.g.*, acne or skin inflammation) referred to herein are known and accepted by those of skill in the art. Representative examples of acne parameters (*e.g*., symptoms) that may be examined for changes, such as for example, in the uses of treating acne of the present disclosure, may include any or all of sebum production, hyperkeritinization of the skin, colonization of the skin by *P. acnes,* release of inflammatory mediators into the skin, and the number and/or severity of inflammatory (*e.g*., papules, pustules, and/or nodules) and/or non-inflammatory (*e.g*., open and/or closed comedones) acne lesions. Additional representative examples of acne parameters that may be examined for changes, such as for example, in the usesof treating acne of the present disclosure, may include scaling, erythema, itching, burning, and/or stinging. Further representative examples of acne parameters that may be examined for changes include the number of lesions, size of lesions, redness of lesions (erythema), and/or itchiness of lesions.

An assessment of acne (*e.g*., number of acne lesions and/or severity of acne) may employ standard medically accepted practices known in the art including, for example, dermatological assessments of the skin and/or quality of life assessments. In some embodiments, two or more assessments of acne may be made (*e.g*., lesion count and IGA score). In further embodiments, where two or more assessments of acne are made, the separate scores obtained from each individual assessment may be combined to create a single aggregate score.

A baseline assessment of a symptom, sign, and/or condition of acne (*e.g*., number, type, and/or severity of lesions, including as graded by any known dermatological scoring system) may be performed prior to treatment of a subject with any of the anti-IL-1β antibodies or binding fragments thereof and compared to an assessment of the symptom, sign, and/or condition of acne made after treatment of the subject with the anti-IL-1β antibodies or binding fragments thereof. In some embodiments the treatment is effective to reduce a symptom, sign, and/or condition of acne by at least about 10% (*e.g*., 15%, 20%, 25%, 30%, 35%, 40%, 45%, 50%, 55%, 60%, 65%, 70%, 75%, 80%, 85%, 90%, 95%, or 100%) or reduce a score given by any known dermatological scoring system by one or more grades (*e.g.*, two grades, three grades, or four grades).

Dermatological assessments of the skin (*e.g*., an examination for improvement in acne) may employ any system that assesses (*e.g*., scores) acne including, for example, severity of acne lesions, number of acne lesions, and/or type of acne lesions (*e.g*., inflammatory and/or non-inflammatory), and assigns the acne to at least one of two or more grades (*e.g*., categories).

Acne (*e.g*., severity of a subject's acne) may be assessed by a scoring system that grades the severity of the acne lesions.

In some embodiments, acne may be graded and assigned one of five grades using an Investigator's Global Assessment (IGA) scoring system as shown in Table 1 below.

**Table 1: IGA Scoring System for Acne Vulgaris (Facial and Non-facial)**

| **Grade** | **Description** |
|---|---|
| **0** | Clear skin with no inflammatory or non-inflammatory lesions |
| **1** | Almost clear; rare non-inflammatory with no more than one small inflammatory lesion |
| **2** | Mild severity; greater than Grade 1; some non-inflammatory lesions with no more than a few inflammatory lesions (papules/pustules only; no active nodulo-cystic lesions) |
| **3** | Moderate severity; greater than Grade 2; up to many non-inflammatory lesions and may have some inflammatory lesions, but no more than one active small nodular lesion |
| **4** | Severe; greater than Grade 3; up to many non-inflammatory and inflammatory lesions, but no more than a few active nodular lesions |

In some embodiments, uses of the present disclosure may provide an improvement in grade of a subject's skin, as measured by the IGA scoring system (see, *e.g*., Table 1), and as compared to the subject's skin pre-treatment. For example, the uses may provide an improvement (*e.g*., decrease) in 1 to 4 grades. For example, the uses may provide for an improvement in grade from 4 to 3, 4 to 2, 4 to 1, or 4 to 0. The uses may also provide for an improvement in grade from 3 to 2, 3 to 1, or 3 to 0. The uses may also provide for an improvement in grade from 2 to 1, or 2 to 0. The uses may also provide for an improvement in grade from 1 to 0.

Acne (*e.g*., severity of a subject's acne) may be assessed by evaluation of one or more symptoms, aspects, parameters, signs, conditions, or characteristics of acne including, for example, scaling, erythema, itching, burning, stinging, size of lesions, swelling, leukocyte infiltration, and/or lesion development. For example, acne may be assessed by a scoring system that grades scaling, redness/erythema, itching, burning, and/or stinging of one or more areas of the skin (e.g., skin on the face). In some embodiments, a grading system that scores scaling may comprise four grades: i) None - no scaling; ii) Mild - barely perceptible, fine scales present in limited areas such as the face; iii.) Moderate - fine scales generalized to all areas such as the face, and iv) Severe - scaling and peeling of skin all over areas such as the face. In some embodiments, a grading system that scores redness/erythema may comprise four grades: i) None - no evidence of erythema present; ii) Mild - slight pink coloration; iii) Moderate - definite redness; and iv) Severe - bright red to dusky dark red in color. In some embodiments, a grading system that scores itching may comprise four grades: i) None - no itching; ii) Mild - slight itching, not really bothersome; iii) Moderate - definite itching that is somewhat bothersome; and iv) Severe -intense itching that may interrupt daily activities and/or sleep. In some embodiments, a grading system that scores burning may comprise four grades: i) None - no burning; ii) Mild - slight burning, not really bothersome; iii) Moderate - definite warm burning sensation that is somewhat bothersome; and iv) Severe - hot burning sensation that causes definite discomfort and may interrupt daily activities and/or sleep. In some embodiments, a grading system that scores stinging may comprise four grades: i) None - no stinging; ii) Mild - slight stinging sensation, not really bothersome; iii) Moderate - definite stinging sensation that is somewhat bothersome; and iv) Severe - stinging sensation that causes definite discomfort and may interrupt daily activities and/or sleep.

In some embodiments, uses of the present disclosure may provide an improvement in grade of a subject's skin, as assessed by a scoring system that grades scaling, redness/erythema, itching, burning, and/or stinging of one or more areas of the skin (e.g., skin on the face) including, as compared to the subject's skin pre-treatment. For example, the uses may provide an improvement (*e.g*., decrease) in 1 to 3 grades. For example, the uses may provide for an improvement in grade from severe to moderate, severe to mild, or severe to none. The uses may also provide for an improvement in grade from moderate to mild, or moderate to none. The uses may also provide for an improvement in grade from mild to none.

Acne (*e.g.,* severity of a subject's acne) may also be measured (*e.g.,* graded) using a facial evaluation scoring system including, for example, by the scoring system set forth in Table 2 below.

**Table 2: Facial Cutaneous Scoring System**

| **Condition** | **None** | **Mild** | **Moderate** | **Severe** |
|---|---|---|---|---|
| **Scaling** | No scaling | Barely perceptible, fine scales present in limited areas of the face | Fine scales generalized to all areas of the face | Scaling and peeling of skin over all areas of the face |
| **Erythema** | No evidence of erythema present | Slight pink coloration | Definite redness | Marked erythema, bright red to dusky dark red in color |
| **Itching** | No itching | Slight itching, not really bothersome | Definite itching that is somewhat bothersome | Intense itching that may interrupt daily activities and/or sleep |
| **Burning** | No burning | Slight burning sensation, not really bothersome | Definite warm, burning sensation that is somewhat bothersome | Hot burning sensation that causes definite discomfort and may interrupt daily activities and/or sleep |
| **Stinging** | No stinging | Slight stinging sensation, not really bothersome | Definite stinging sensation that is somewhat bothersome | Stinging sensation that causes definite discomfort and may interrupt daily activities and/or sleep |

In some embodiments, uses of the present disclosure may provide an improvement in grade of a subject's skin, as measured by the facial cutaneous scoring system (see, *e.g*., Table 2) and as compared to the subject's skin pre-treatment. For example, the uses may provide an improvement (*e.g*., decrease) in 1 to 3 grades. For example, the uses may provide for an improvement in grade from severe to moderate, severe to mild, or severe to none. The uses may also provide for an improvement in grade from moderate to mild, or moderate to none. The uses may also provide for an improvement in grade from mild to none.

In other embodiments, the grading system may include the following four grades: grade 1- comedones and occasional small cysts confined to the face; grade 2-comedones with occasional pustules and small cysts confined to the face; grade 3- many comedones and small and large inflammatory papules and pustules, more extensive but confined to the face; and grade 4- many comedones and deep lesions tending to coalesce and canalize, and involving the face and the upper aspects of the trunk (*see, e.g.,* Witkowski et al. (2004) Clin Dermatol 22:394-7).

In other embodiments, the grading system includes the following four grades: grade 1- simple non-inflammatory acne - comedones and a few papules; grade 2-comedones, papules and a few pustules; grade 3- larger inflammatory papules, pustules and a few cysts, a more severe form involving the face, neck and upper portions of the trunk; and grade 4- more severe, with cysts becoming confluent (see, *e.g.,* Witkowski et al. (2004) Clin Dermatol 22:394-7).

In other embodiments, acne may be categorized into one of four grades as follows. Grade 1: Comedones, occasional papules. Grade 2: Papules, comedones, few pustules. Grade 3: Predominant pustules, nodules, abscesses. Grade 4: Mainly cysts, abscesses, widespread scarring (see, *e.g.,* Tutakne et al. (2003) IADVL Textbook and atlas of dermatology, 2nd ed., Mumbai: Bhalani publishing House; p. 689-710).

In other embodiments, acne may be categorized into one of three categories: i.) mild acne - up to five inflammatory lesions; ii.) moderate acne - 6-20 inflammatory lesions; and iii.) very severe acne - more than 50 inflammatory lesions (see, *e.g.,* Hayashi et al. (2008) Journal of Dermatology 2008; 35:255-260).

In some embodiments, the number of acne lesions on the face, chest and back may be counted and assigned a score based upon lesion type. For example, comedones may be valued (given a severity index) at 0.5; papules, at 1.0; pustules, at 2.0; infiltrates, at 3.0; and cysts, at 4.0. A total score may then be obtained by multiplying the number of each type of lesion by its severity index to obtain a total score that represents the severity of the disease. See, Michaelsson et al. (1977) Acta Derm Venereol 57:372.

In some embodiments, the grading system includes the following five grades: i.) clear - indicating no inflammatory or noninflammatory lesions; ii.) almost clear-rare noninflammatory lesions with no more than one papules/pustule; iii.) mild- some noninflammatory lesions, no more than a few papules/pustules but no nodules; iv.) moderate- up to many noninflammatory lesions, may have some inflammatory lesions, but no more than one small nodule; and v.) severe- up to many noninflammatory and inflammatory lesions, but no more than a few nodules.

Alternatively, in some embodiments, acne (*e.g*., severity of a subject's acne) may be assessed by the global acne grading system (GAGS). This system is a quantitative scoring system in which the total severity score is derived from summation of six regional subscores. Each is derived by multiplying the factor for each region (factor for forehead and each cheek is 2, chin and nose is 1 and chest and upper back is 3) by the most heavily weighted lesion within each region (1 for ≥ one comedone, 2 for ≥ one papule, 3 for ≥ one pustule and 4 for ≥ one nodule). *See, e.g.,* Doshi et al. (1997) Int J Dermatol 36:416-8.

Alternatively, in some embodiments, acne (*e.g.*, severity of a subject's acne) may be assessed by using a three-category system for inflammatory acne (*e.g*., acne on the face, chest and/or back), where mild acne is comprised of few to several papules/pustules; moderate acne of several to many papules/pustules and few to several nodules; and severe acne of numerous or extensive papules/pustules and many nodules.

Alternatively, in some embodiments, the Leed technique may be used to assess acne which grades acne on a scale of 0 (no acne) to 10 (the most severe) (see, *e.g.,* Burke et al. (1984) Br J Dermatol 111:83-92) presented the Leeds technique.

Acne (*e.g.*, severity of a subject's acne) may be assessed by a comparison of the acne to photographic standards.

In some embodiments, the overall severity of acne may be based upon a 0-8 scale anchored to photographic standards that illustrate grades 0, 2, 4, 6 and 8 (see, Cook et al. (1979) Arch Dermatol 115:571-5). In some embodiments, the Leeds Revised Acne Grading System may be employed as a photographic standard for acne grading of the face, back and chest. This system is comprised of 15 facial grades (three solely for comedonal acne) and eight each for the chest and back (see, *e.g.,* O'brien et al. (1998) Journal of Dermatological Treatment 9(4):215-220).

In other embodiments, a combination of photographic standards and lesion counting may be used to classify acne into four groups (*see*, *e.g.,* Hayashi et al. (2008) J Dermatol 35:255-60). For example, acne may be classified based on the number of inflammatory eruptions on half of the face as 0-5, "mild"; 6-20, "moderate"; 21-50, "severe"; and more than 50, "very severe."

Acne (*e.g.*, severity of a subject's acne) may be assessed by a scoring system that counts the acne lesions and optionally assigns the acne to one of two or more grades.

In some embodiments, a number of lesions of the face including, for example, on one side of the face may be counted (see, Witkowski et al. (1966) JAMA 196:397-400).

In some embodiments acne lesion counts may be recorded on a template divided into five facial segments: Right and left sides of the forehead, right and left cheeks and chin (nose is excluded). Total lesion count and counts of each lesion type (*e.g.*, inflammatory lesions or comedones) may be recorded within each segment of the template. See, *e.g.,* Lucky et al. (1996) J Am Acad Dermatol 35:559-65.

In some embodiments, a subject's acne may be measured (*e.g.*, graded) by counting the number of acne lesions present on the skin (*e.g*., face). The number of inflammatory and/or non-inflammatory acne lesions may be calculated after treatment and compared to the number of inflammatory and/or non-inflammatory acne lesions pre-treatment. An improvement in a subject's acne may include at least a 10%, at least a 20%, at least a 30%, at least a 40%, at least a 50%, at least a 60%, at least a 70%, at least a 80%, at least a 90% or more decrease in the number of inflammatory and/or non-inflammatory acne lesions, as compared to initial (*e.g*., pre-treatment) levels.

Alternatively, in some embodiments, acne (*e.g*., severity of a subject's acne) may be assessed by a combination of lesion counting, a grading system and/or photographic standards.

Further, the uses of the present disclosure may provide an improvement in one or more aspects of quality of life, such as for example as determined by a quality of life (QOL) assessment (*e.g*., Cardiff Acne Disability Index (CADI) questionnaire as shown below in Table 3).

**Table 3: Cardiff Acne Disability Index (CADI)**

| | | |
|---|---|---|
| 1. As a result of having acne, during the last month have you been aggressive, frustrated or embarrassed? | □ | (a) Very much indeed |
| | □ | (b) A lot |
| | □ | (c) A little |
| | □ | (d) Not at all |
| 2. Do you think that having acne during the last month interfered with your daily social life, social events or relationships with members of the opposite sex? | □ | (a) Severely, affecting all activities |
| | □ | (b) Moderately, in most activities |
| | □ | (c) Occasionally or in only some activities |
| | □ | (d) Not at all |
| 3. During the last month have you avoided public changing facilities or wearing swimming costumes because of your acne? | □ | (a) All of the time |
| | □ | (b) Most of the time |
| | □ | (c) Occasionally |
| | □ | (d) Not at all |
| 4. How would you describe your feelings about the appearance of your skin over the last month? | □ | (a) Very depressed and miserable |
| | □ | (b) Usually concerned |
| | □ | (c) Occasionally concerned |
| | □ | (d) Not bothered |
| 5. Please indicate how bad you think your acne is now. | □ | (a) The worst it could possibly be |
| | □ | (b) A major problem |
| | □ | (c) A minor problem |
| | □ | (d) Not a problem |

Alternatively or in addition, assessments may include measures of biologic and clinical activity, including non-acne parameters, such as for example: Inflammatory markers (CRP and/or ESR)

Analysis of cytokines (*e.g*., markers for cytokines, such as inflammatory cytokines), including, but not limited to, adiponectin, resistin, leptin, visfatin, PAI-1, TNFα, IFNγ, IL-1, IL-1Ra, IL-6, IL-8, RANTES, IL-1α and MCP-1.

In addition, uses of the present disclosure may provide an improvement (*e.g*., decrease) in C-reactive protein (CRP) levels. The reduction in CRP levels is readily measured using standard assays (*e.g.*, high-sensitivity CRP, ultra-sensitive CRP). As provided by the uses disclosed herein, the decrease in C-reactive protein levels may, for example, be a decrease of >0.2, >0.4, >0.6, >0.8, >1.0, >1.4, >1.8, >2.2, >2.6, >3.0 mg/L from pre-treatment levels. Alternatively, the decrease in C-reactive protein levels may, for example, be a decrease of >20%, >30%, >40%, >50%, >60%, >70%, >80%, >90%, >95% from pre-treatment levels.

In some embodiments, uses of treating or preventing a disease or condition in accordance with the present disclosure may use a pre-determined or "routine" schedule for administration of the antibody or fragment. As used herein a routine schedule refers to a predetermined designated period of time between dose administrations. The routine schedule may encompass periods of time which are identical or which differ in length, as long as the schedule is predetermined. Any particular combination would be covered by the routine schedule as long as it is determined ahead of time that the appropriate schedule involves administration on a certain day.

### Dosing

A dosage and dosage regimen may be administered to provide the optimal desired response (*e.g.,* therapeutic response). The dose of a composition (*e.g.,* pharmaceutical composition) comprising an IL-1β binding molecule such as an anti-IL-1β antibody or binding fragment thereof may be measured in units of mg/kg of patient body weight. Alternatively, the dose of a composition comprising anti-IL-1β antibodies or binding fragments thereof is measured in units of mg/kg of patient lean body weight (*e.g.*, body weight minus body fat content), in units of mg/m² of patient body surface area, or in units of mg per dose (*e.g*., a fixed dose) administered to a patient. Any measurement of dose can be used in conjunction with the compositions and uses of the invention and dosage units can be converted by means standard in the art.

Those skilled in the art will appreciate that dosages can be selected based on a number of factors including the age, sex, species and condition of the subject (*e.g*., severity of the acne), and/or the particular antibody or antibody binding fragment being used and can be determined by one of skill in the art. For example, effective amounts of the compositions of the invention may be extrapolated from dose-response curves derived in vitro test systems or from animal model test systems. Models and methods for evaluation of the effects of antibodies are known in the art (Wooldridge et al., Blood, 89(8): 2994-2998 (1997)).

In some embodiments, the dosage can be adjusted and/or the infusion rate can be reduced based on patient's immunogenic response to the antibody.

anti-IL-1β antibodies or binding fragments thereof for use in any and/or all of the aforementioned methods and uses may be administered in one or more doses (*e.g*., an initial dose optionally followed by one or more subsequent doses). Those skilled in the art will appreciate that dosages are generally higher and/or frequency of administration greater for initial treatment as compared with maintenance regimens. In some embodiments, the anti-IL-1β antibody or binding fragment thereof is administered in one or more doses of 10 mg/kg or less, 5 mg/kg or less, 3 mg/kg or less, 2 mg/kg or less, 1 mg/kg or less, 0.9 mg/kg or less, 0.8 mg/kg or less, 0.7 mg/kg or less, 0.6 mg/kg or less, 0.5 mg/kg less, 0.4 mg/kg or less, 0.3 mg/kg or less, 0.2 mg/kg or less, 0.1 mg/kg or less, or 0.03 mg/kg or less of antibody or fragment. In some of the aforementioned embodiments, the one or more doses are at least 0.01 mg/kg or at least 0.1 mg/kg of anti-IL-1β antibody or binding fragment thereof. In some embodiments, the anti-IL-1β antibody or binding fragment thereof is administered in one or more doses of about 0.01 mg/kg to 1 mg/kg, about 0.03 mg/kg to 1 mg/kg, about 0.01 mg/kg to 0.3 mg/kg, about 0.1 mg/kg to 0.3 mg/kg, about 0.001 mg/kg to 0.3 mg/kg, about 0.001 mg/kg to 0.1 mg/kg, about 0.001 mg/kg to 0.03 mg/kg, or about 0.001 mg/kg to 0.01 mg/kg. In some embodiments, the anti-IL-1β antibody or binding fragment thereof is administered in one or more doses of 1mg/kg or less or a dose less than or equal to 1 mg/kg (*e.g*., 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg such as 0.2 mg/kg or 0.6 mg/kg).

In other embodiments, the initial dose and one or more subsequent doses of anti-IL-1β antibody or binding fragment thereof are each from about 0.01 mg/kg to about 10 mg/kg of antibody, from about 0.05 to about 5 mg/kg of antibody, from about 0.05 mg/kg to about 3 mg/kg of antibody, from about 0.1 mg/kg to about 3 mg/kg of antibody, from about 0.1 mg/kg to about 1 mg/kg of antibody, from about 0.1 mg/kg to about 0.5 mg/kg of antibody, from about 0.3 mg/kg to about 5 mg/kg of antibody, from about 0.3 mg/kg to about 3 mg/kg of antibody, from about 0.3 mg/kg to about 1 mg/kg of antibody, from about 0.5 mg/kg to about 5 mg/kg of antibody, from about 0.5 mg/kg to about 3 mg/kg of antibody, from about 0.5 mg/kg to about 1 mg/kg of antibody, from about 1 mg/kg to about 5 mg/kg of antibody, or from about 1 mg/kg to about 3 mg/kg of antibody. In certain embodiments, two or more, three or more, four or more, five or more, six or more, seven or more, eight or more, nine or more, ten or more or eleven or more subsequent doses of the antibody are administered. The aforementioned dosage amounts refer to mg (antibody or fragment)/kg (weight of the individual to be treated).

anti-IL-1β antibodies or binding fragments thereof for use in any and/or all of the aforementioned methods and uses may be administered as a fixed dose, independent of a dose per subject weight ratio.

In some embodiments, the anti-IL-1β antibody or binding fragment thereof is administered in one or more fixed doses of about 1000 mg or less, 500 mg or less, or 250 mg or less, 100 mg or less, 90 mg or less, 80 mg or less, 70 mg or less, 60 mg or less, 50 mg or less, 40 mg or less, 30 mg or less, 20 mg or less, or 10 mg or less of antibody or fragment. In some embodiments, the anti-IL-1β antibody or binding fragment thereof is administered in one or more doses of at least 0.5 mg, at least 1 mg of antibody or fragment, or at least 10 mg of antibody or fragment. In some embodiments, the anti-IL-1β antibody or binding fragment thereof is administered in one or more doses of 1 mg to 100 mg of antibody or fragment.

In certain embodiments, the fixed dose of anti-IL-1β antibody or binding fragment thereof is from about 1 mg to about 10 mg, about 1 mg to about 25 mg, about 10 mg to about 25 mg, about 10 mg to about 50 mg, about 10 mg to about 100 mg, about 25 mg to about 50 mg, about 25 mg to about 100 mg, about 50 mg to about 100 mg, about 50 mg to about 150 mg, about 100 mg to about 150 mg, about 100 mg to about 200 mg, about 150 mg to about 200 mg, about 150 mg to about 250 mg, about 200 mg to about 250 mg, about 200 mg to about 300 mg, about 250 mg to about 300 mg, about 250 mg to about 500 mg, about 300 mg to about 400 mg, about 400 mg to about 500 mg, about 400 mg to about 600 mg, about 500 mg to about 750 mg, about 600 mg to about 750 mg, about 700 mg to about 800 mg, or about 750 mg to about 1000 mg. In some embodiments, the fixed dose of anti-IL-1β antibody or binding fragment thereof is less than 100 mg.

In some embodiments of any and/or all of the aforementioned uses, the fixed dose of anti-IL-1β antibody or binding fragment thereof is administered using a prefilled syringe or delivery device.

In some embodiments of any and/or all of the aforementioned uses, the anti-IL-1β antibody or binding fragment thereof is administered by subcutaneous, intravenous, intradermal or intramuscular injection.

In some embodiments of any and/or all of the aforementioned uses, an anti-IL-1β antibody or binding fragment thereof is administered once for an acne outbreak. In some embodiments of any and/or all of the aforementioned methods and uses, administration of an initial dose of anti-IL-1β antibody or binding fragment thereof is followed by the administration of one or more subsequent doses. Examples of dosing regimens (*e.g*., an interval between the first dose and one or more subsequent doses) that can be used in the methods and uses of the disclosure include an interval of about once every week to about once every 12 months, an interval of about once every two weeks to about once every 6 months, an interval of about once every month to about once every 6 months, an interval of about once every month to about once every 3 months, or an interval of about once every 3 months to about once every 6 months. In some embodiments, administration is monthly, every two months, every three months, every four months, every five months, every six months, or on recurrance of the acne.

The disclosure also provides dosing regimens for use in any and/or all of the aforementioned methods and uses, wherein the dosing regimens comprise more than one dosing interval for administration of an anti-IL-1β antibody or binding fragment thereof. In some embodiments, the dosage regimen comprises at least two (*e.g*., two, three, four, five, six) different dosing intervals for administration of the anti-IL-1β antibody or binding fragment thereof. In some embodiments, the dosage regimen comprises two different dosing intervals for administration of the anti-IL-1β antibody or binding fragment thereof. In some embodiments, the dosing regimen comprises two different dosing intervals for administration of the anti-IL-1β antibody or binding fragment thereof, wherein a first dosing interval comprises administration of one or more doses of the anti-IL-1β antibody or binding fragment thereof and a second dosing interval comprises administration of one or more doses of the anti-IL-1β antibody or binding fragment thereof, and wherein the first dosing interval is shorter in time than the second dosing interval. For example, the first dosing interval may be days or weeks, and the second dosing interval may be months. In some embodiments, the first dosing interval is about 5 days to about 28 days, about 7 days to about 21 days, about 12 days to about 16 days, or about 14 days. In some embodiments, the second dosing interval is about 1 month to about 3 months, about 1 month to about 2 months, or about 1 month.

In some embodiments of any and/or all of the aforementioned uses, the dose can be escalated or reduced to maintain a constant dose in the blood or in a tissue, such as, but not limited to, skin. In related embodiments, the dose is escalated or reduced by about 2%, 5%, 8%, 10%, 15%, 20%, 30%, 40%, 50%, 60%, 70%, 80%, 90%, and 95% in order to maintain a desired level of the antibody.

In some embodiments of any and/or all of the aforementioned uses, the anti-IL-1β antibody or binding fragment thereof is administered to a subject such that the interval between doses is a time sufficient to maintain a plasma concentration of said antibody or antibody fragment in the subject at a level of at least about 0.1 µg/mL, at least about 0.3 µg/mL, at least about 1 µg/mL or at least about 2 µg/mL. In some embodiments, these plasma concentration values refer to values obtained for an individual that is treated with the antibody of fragment in accordance with the disclosure herein.

In some embodiments of any and/or all of the aforementioned uses, administration of an initial dose of the anti-IL-1β antibody or binding fragment thereof is followed by the administration of one or more subsequent doses, and wherein said one or more subsequent doses are in an amount that is approximately the same or less than the initial dose.

In some embodiments of any and/or all of the aforementioned uses, administration of an initial dose of the anti-IL-1β antibody or binding fragment thereof is followed by the administration of one or more subsequent doses, and wherein at least one of the subsequent doses is in an amount that is more than the initial dose.

In some embodiments of any and/or all of the aforementioned uses, the anti-IL-1β antibody or binding fragment thereof has a lower IC50 than an IL-1β receptor antagonist in a human whole blood IL-1β inhibition assay that measures IL-1β induced production of IL-8. In some embodiments, the IL-1β receptor antagonist is anakinra.

In some embodiments of any and/or all of the aforementioned uses, an anti-IL-1β antibody or binding fragment thereof is administered, wherein administration of an initial dose of the antibody or antibody fragment is followed by the administration of one or more subsequent doses, and wherein the plasma concentration of said antibody or antibody fragment in the human is permitted to decrease below a level of about 0.1 µg/mL, about 0.07 µg/mL, about 0.05 µg/mL, about 0.03 µg/mL or about 0.01 µg/mL for a period of time greater than about 1 week and less than about 6 months between administrations during a course of treatment with said initial dose and one or more subsequent doses. In some embodiments, the plasma concentration values refer to values obtained for an individual that is treated with the antibody of fragment in accordance with the disclosure herein.

### Combinations

The disclosure also provides that compositions comprising one or more other active agents (*e.g*., an anti-acne agent) may be administered in conjunction with or separately from the IL-1β antibodies or binding fragments thereof, and such administrations may be performed at the same point or different points in time, such as for example the same or different days. Administration of the other active agents may be according to standard medical practices known in the art (*e.g*., current standard of care), or the administration may be modified (*e.g*., longer intervals, smaller dosages, delayed initiation) when used in conjunction with administration of IL-1β antibodies or binding fragments thereof, such as disclosed herein. The active agents set forth below are exemplary and not intended to be limiting. Combinations can also include more than one additional agent, *e.g*., two or three or more additional agents.

Anti-IL-1β antibodies or fragments thereof administered to a subject in as disclosed herein may be administered in combination with treatment with at least one additional active agent (*e.g*., an anti-acne agent), such as for example any of the active agents provided herein. In one embodiment, treatment with the at least one active agent is maintained. In another embodiment, treatment with the at least one active agent is reduced or discontinued (*e.g*., when the subject is stable), while treatment with the anti-IL-1β antibody or binding fragment thereof is maintained at a constant dosing regimen. In another embodiment, treatment with the at least one active agent is reduced or discontinued (*e.g.*, when the subject is stable), and treatment with the anti-IL-1β antibody or binding fragment thereof is reduced (*e.g*., lower dose, less frequent dosing, shorter treatment regimen). In another embodiment, treatment with the at least one active agent is is reduced or discontinued (*e.g*., when the subject is stable), and treatment with the anti-IL-1β antibody or binding fragment thereof is increased (*e.g*., higher dose, more frequent dosing, longer treatment regimen). In yet another embodiment, treatment with the at least one active agent is maintained and treatment with the anti-IL-1β antibody or binding fragment thereof is reduced or discontinued (*e.g*., lower dose, less frequent dosing, shorter treatment regimen). In yet another embodiment, treatment with the at least one active agent and treatment with the anti-IL-1β antibody or binding fragment thereof are reduced or discontinued (*e.g*., lower dose, less frequent dosing, shorter treatment regimen).

In some embodiments of any of the uses described above, one or more active agents including, those agents used and/or approved for the treatment of acne (*e.g*., anti-acne agents) are administered in conjunction with the anti-IL-1β antibody or binding fragment thereof disclosed herein, including for example, one or more active agents selected from the group consisting of: benzoyl peroxide, retinol, retinal, tretinoin, isotretinoin, adapalene, isotretinoin, dapsone, sulfacetamide sodium, azelaic acid, prednisone, tretinoin, clindamycin, zithromycin, erythromycin, minocycline, and tetracycline. Active agents may also include a supplement such as Brewer's yeast (*e.g*., *Saccharomyces cerevisiae*), zinc gluconate, or flaxseed oil; a botanical such as tea tree oil.

In some embodiments, any of the uses described above may further comprise administering at least one other pharmaceutical composition comprising an active agent other than an anti-IL-1β antibody or binding fragment thereof. In some embodiments, the active agent of said at least one other pharmaceutical composition is an antimicrobial agent.

In another embodiment, the use of the IL-1β antibodies or binding fragments is contemplated in the manufacture of a medicament for treating or preventing acne including, for example, acne that is not responsive to one or more antibiotics. In any of the uses, the medicament can be coordinated with treatment using a second active agent.

Anti-acne agents include any agent that is known to be useful and/or effective in the treatment of acne such as agents that improve one or more symptoms, signs, and/or conditions of acne (*e.g*., inflammatory lesion count, non-inflammatory lesion count, total lesion count, proportion of clear or almost clear skin, size of lesions, redness of lesions, and/or itchiness of lesions). Anti-acne agents include, for example, prescription based and/or over-the-counter (OTC) treatments.

There are a number of prescription based anti-acne agents available for the treatment of acne. The dermatologist choice of therapy for an individual patient depends on the extent, severity, duration, and the type of acne lesions exhibited by the patient. Some commonly prescribed topical and oral anti-acne treatments are described below.

Benzoyl peroxide is the most common first-line treatment for acne, particularly in comedonal acne. Benzoyl peroxide is available in a variety of concentrations (1, 2.5, 5, and 10%) and formulations (solution, gel, and lotion). The frequency and dose of benzoyl peroxide can be increased as tolerability to the agent develops.

Retinoids and retinoid derivatives, such as retinol, retinal, tretinoin, isotretinoin, adapalene (6-[3-(1-adamantyl)-4-methoxyphenyl]-2-naphthoic acid), and tazarotene, and the like, are commonly used for the treatment of comedonal acne. Alternatively, salicylic acid which has retinoid-like properties is commonly used for the treatment of acne.

Oral isotretinoin (13-cis retinoic acid) may also be useful in the treatment of acne. Oral isotretinoin is marketed as Accutane® in 10, and 40 mg capsules. This oral retinoid related to Vitamin A is not suitable for all types of acne but is used for control of acne and in the induction of long-term remissions.

Corticosteroids, such as prednisone, are useful in the management of acne. Anti-inflammatory oral agents such as prednisone may be useful for rapidly advancing disease, including during the time that cysts may be improving slowly with other therapies such as with isotretinoin. Intralesional corticosteroid injections, such as with triamcinolone (*e.g*., Kenalog® 10 mg/ml or TAC-3® 3 mg/ml), may also be used.

Additional acne therapies include systemic treatment with hormone manipulation (*e.g.,* hormone therapy) such as therapies used for oral contraception (*e.g.,* estrogens and/or progestins such as norethindrone acetate-ethinyl estradiol, or norgestimate-ethinyl estradiol" glucocorticoids such as prednisone, or antiandrogens, including, spironolactone or flutamide) as well as acne surgery (*e.g*., manual removal of comedones and/or the drainage of pustules or cysts, scar revision, dermabrasion, scar excision or collagen implants). Acne therapies may also include light therapy such as ultraviolet (UV) light therapy.

Topical tretinoin (Retin-A®, Avita™), also known as retinoic acid or Vitamin A acid, may be used for the treatment of acne. Tretinoin is approved for use in comedonal acne and mild forms of papulopustulosa acne. Tretinoin is typically applied once daily, starting with a lower concentration of the cream (available in 0.025, 0.05, and 0.1 % concentrations), gel (0.01 and 0.025%), or microemulsion gel (0.1 %). Currently, there are a number of formulations which utilize lower tretinoin concentration (0.025%) with cream vehicles that are designed to be more emollient and less penetrating. For example, the Retin-® Micro (tretinoin gel, 0.1%) microsphere formulation is designed as a slow delivery of tretinoin gel, in which the active ingredient is incorporated into microsponges, which are macroporous beads (10-25 microns). Tretinoin may be used in combination with other topical agents such as topical antibiotics and/or benzoyl peroxide, and may enhance their penetration.

Tazarotene cream (Tazorac™) is a synthetic acetylenic retinoid and has been approved by the FDA for topical treatment of acne. In addition, tazarotene gel (0.1%) has also been approved for mild-to-moderate acne vulgaris. Tazarotene is typically applied once daily over the entire affected area.

Adapelene (Differin™) is a synthetic naphthoic acid derivative and may be used for the treatment of acne. Adapelene is available as a gel or cream (0.1% concentration). It is initially applied 2-3 times per week, and usage is increased to nightly application over a period of about 2 months.

Azelaic acid is a dicarboxylic acid that may be used in the treatment of acne. Topical azelaic acid has similar efficacy to topical benzoyl peroxide gel (5%), tretinoin cream (0.05%), erythromycin cream (2%), and oral tetracycline (0.5 to 1 g/day), in the treatment of comedonal and mild to moderate inflammatory acne.

Topical antimicrobial agents such as antibiotics are thought to be effective in the treatment of acne by killing or inhibiting *P. acnes* and are used for the treatment of mild forms of papulopustulosa acne. Representative examples of topical antibiotics include clindamycin (*e.g*. lincomycins), zithromycin, erythromycin, minocycline, and tetracycline.

Oral antibiotics have been used for decades for the treatment of papular, pustular and cystic acne. These antibiotics include tetracycline (*e.g.*, 250 and 500 mg dosage forms), erythromycin (*e.g*., 250, 333, 400 and 500 mg dosage forms), azithromycin, doxycycline or minocycline (*e.g.,* 50 and 100 mg dosage forms), clindamycin (*e.g.,* 75, 150 and 300 mg dosage forms), ampicillin (*e.g*., 250 and 500 mg dosage forms), amoxicillin, cephalosporins such as cephalexin (*e.g.,* 500 mg dosage forms) and trimethoprim/sulfamethoxazole (*e.g.*, double strength (DS) tablets).

Clindamycin phosphate (Dalacin T™ solution and Cleocin-T™ gel, lotion, or solution by Upjohn, Kalamazoo, Mich.) is a macrolide lincomycin antibiotic that is bacteriostatic and can penetrate sebaceous follicles and reduce *P. acnes* growth. Clindamycin phosphate has been described, for example, in U.S. Pat. No. 3,969,516.

Combinations of antimicrobial agents such as antibiotics in topical formulations have been used in the treatment of acne. One of the first combination topical therapies is a mixture of 5% benzoyl peroxide with 3% erythromycin. Another combination topical therapy is BenzaClin™ which is a mixture of 5% benzoyl peroxide with 1% clindamycin phosphate. BenzaClin™ is used in the treatment of moderate to moderately severe facial acne. Another antibiotic combination that is commonly used is Benzamycin™. Benzamycin™ is a topical gel combination of erythromycin (3%) and benzoyl peroxide (5%).

In addition to prescription based anti-acne agents, there are also numerous over-the-counter (OTC) anti-acne agents available. Common OTC acne products include cleansers, pads, lotions, cover-up products, masks, and facials. Active agents in OTC treatments include benzoyl peroxide, coal tar, resorcinol, sulfur, and salicylic acid. Representative examples of some OTC treatments comprising benzoyl peroxide include Oxy® 5 (5% benzoyl peroxide lotion), Benzoyl® 10 (10% benzoyl peroxide lotion), Benzashave® (5% or 10% benzoyl peroxide cream), Advanced Formula Oxy Sensitive® or Benzac® AC 2.5 or Desquam-E® or PanOxyl AQ® 2.5 (2.5% benzoyl peroxide gel), Benzac® 5 or Benzac AC® 5 or 5-Benzagel® or Desquam-E 5® or Desquam-X 5® or PanOxyl AQ® 5 or PanOxyl® 5 (5% benzoyl peroxide gel), Benzac® 10 or Benzac AC® 10 or 10-Benzagel® or Desquam-E® 10 or Desquam-X® 10 or PanOxyl AQ® 10 or PanOxyl® 10 (10% benzoyl peroxide gel). Examples of some OTC treatments comprising salicylic acid include, for instance, Stri-Dex® pads, Fostex® cleansing pads, and Clearasil Maximum Strength® cleansing pads (2% salicylic acid pads). In addition, vitamins and minerals, including zinc, vitamin C and vitamin E, are used in the treatment of acne.

Compositions that comprise an anti-IL-1β antibody or binding fragment thereof may include one or more additional biological agents including those agents that reduce abnormal keratinization (*e.g.,* abnormal keratinization in the follicular infundibulum), reduce IL-1α or IL-1α receptor antagonism (e.g., an anti- IL-1α antibody or binding fragment thereof), reduce inflammatory mediators (*e.g*., 5-lipoxygenase), reduce leukocyte chemotaxis, reduce the production of reactive oxygen species, improve anti-androgenic effectiveness, enhance the production of endogenous antimicrobial peptides, manipulate the migration of Langerhans cells, and/or manipulate the expression and/or activity of TNFα, integrin, and/or TLR2.

Additionally, antibodies that specifically bind IL-1α may be used in the treatment of acne (see, *e.g.,* WO 2012/125812).

In yet another aspect of the present disclosure, an article of manufacture is provided, comprising a container, a composition within the container comprising an anti-IL-1β antibody or binding fragment thereof, and a package insert containing instructions to administer the antibody or fragment to a subject (*e.g.,* human) as disclosed herein (*e.g.,* for the reduction, prevention or treatment of acne including, for example, acne that is not responsive to one or more antibiotics). In one embodiment, the container further comprises a pharmaceutically suitable carrier, excipient or diluent. In a related embodiment, the composition within the container further comprises a second active agent.

Kits are also contemplated by the disclosure. In one embodiment, a kit comprises a therapeutically or prophylactically effective amount of an anti-IL-1β antibody or binding fragment thereof, packaged in a container, such as a vial or bottle, and further comprising a label attached to or packaged with the container, the label describing the contents of the container and providing indications and/or instructions regarding use of the contents of the container as disclosed herein (*e.g.,* for the reduction, prevention or treatment of acne including, for example, acne that is not responsive to one or more antibiotics). In one embodiment, the container further comprises a pharmaceutically suitable carrier, excipient or diluent. In a related embodiment, the container further contains a second active agent.

In one embodiment, the article of manufacture, kit or medicament is for the treatment or prevention of acne (*e.g*., acne that is not responsive to one or more antibiotics) in a subject (*e.g.,* human) as disclosed herein. In another embodiment, the instructions of a package insert of an article of manufacture or label of a kit comprise instructions for administration of the antibody or fragment according to any of the aforementioned dose amounts and/or dosing regiments. In yet another embodiment, the container of kit or article of manufacture is a pre-filled syringe.

### EXAMPLES

The following examples are intended merely to further illustrate the practice of the present invention, but should not be construed as in any way limiting its scope. The disclosures of all patent and scientific literatures cited within are hereby expressly incorporated in their entirety by reference.

### Example 1: Methods of treating acne with an IL-1β antibody

Studies are conducted to determine the effects (*e.g*., on facial lesions) of an anti-IL1β antibody in subjects with acne, including acne that is not responsive to an antibiotic. For example, a multicenter, randomized, double-blind, placebo-controlled study is undertaken to evaluate treatment with a weight-based dose of an anti-IL-1β antibody or binding fragment thereof in human subjects diagnosed with moderate to severe acne vulgaris that is not responsive to prior treatment with one or more antibiotics. More specifically, a clinical study was performed to examine the efficacy and safety of a high affinity IL-1β antibody (gevokizumab) including, for example, an IL-1β antibody comprising a heavy chain variable region of SEQ ID NO: 6 and a light chain variable region of SEQ ID NO: 5, in subjects with acne that is not responsive to one or more standard of care medications, such as for example, antibiotics (*e.g*., oral antibiotics).

Subjects are enrolled in the clinical trial if they meet the following criteria:
1. Diagnosis of moderate to severe acne vulgaris; inclusion criteria for this trial include the following:
   a. Facial IGA grade of 3 or 4;
   b. Inflammatory facial lesion count of at least 20;
   c. ≤ 3 nodulocystic lesions (defined as > 0.5 cm in diameter);
2. Acne vulgaris unresponsive to oral antibiotics;
3. Agree to maintain consistent and appropriate habits for facial cleaning, shaving, and application of cosmetics from Screening through Day 84.

In addition to the criteria set forth above, the subjects may be 16 years of age or older.

Alternatively, subjects are enrolled in the clinical trial if they meet the following criteria:
1. Diagnosis of moderate to severe acne vulgaris, defined as all of the following:
   a. Facial IGA grade of 3 or 4;
   b. Inflammatory facial lesion count of at least 20;
   c. ≤ 7 nodulocystic lesions (defined as > 0.5 cm in diameter)
2. Acne vulgaris unresponsive to oral antibiotics;
3. Agree to maintain consistent and appropriate habits for facial cleaning, shaving, and application of cosmetics from Screening through Day 84;

In addition to the criteria set forth above, the subject may be of 17 years of age or older and/or may have a weight <100 kg.

Subjects are excluded from the clinical trial if they meet any of the following criteria:
1. Use of medications or treatments from specified pre-treatment time periods through the end of the study;
2. Malignant skin lesions;
3. Beard, moustache, sideburns or other facial hair that may interfere with evaluation;
4. Other forms of acne (*e.g*., acne rosacea, acne excoriee, chloracne, acne conglobata, acne fulminans, acne inversa or drug-induced acne);
5. History of malignancy within 5 years;
6. History of allergic or anaphylactic reactions to monoclonal antibodies;
7. History of tuberculosis or positive PPD test;
8. History of chronic systemic infections;
9. Female subjects who are pregnant, planning to become pregnant.

Alternatively, subjects are excluded from the clinical trial if they meet any of the following criteria:
1. Use of the following medications or treatments from the specified pre-treatment time period through Day 84:
   a. Within 5 months prior to Screening
      i. Use of systemic retinoids
   b. Within 3 months prior to Screening
      i. Any biologic or immunosuppressive therapy (investigational or marketed)
      ii. Major surgery or any planned surgical procedure during the study
   c. Within 2 months prior to Screening
      i. Hospitalization or intravenous (IV) antibiotics to treat active, recent, or chronic infection
   d. From Screening
      i. Topical or systemic corticosteroids. Use of inhaled, ophthalmic, or intranasal corticosteroids is acceptable.
      ii. Any live (attenuated) vaccine; killed virus vaccines are permitted
      iii. Any systemic or oral acne therapies including, but not limited to, antibiotics or anti-androgenic agents
      iv. UV or light-based therapies including, but not limited to, tanning booths/lamps, ClearLight™, ZENO®, Smoothbeam™
      v. Chemical peel, laser peel, or microdermabrasion
      vi. Any topical acne therapies or facial products (other than mild cleansers or moisturizers) including, but not limited to, those that contain retinoids, salicylic acid, antibiotics, benzoyl peroxide, α- or β-hydroxy acids, or physically abradant agents
2. Malignant skin lesions;
3. Beard, moustache, sideburns (temporal zygomatic hair) or any other facial hair that may interfere with evaluation, in the opinion of the investigator
4. Other forms of acne (e.g., acne rosacea, acne excoriee, chloracne, acne conglobata, acne fulminans, acne inversa or drug-induced acne)
5. History of malignancy within 5 years
6. History of allergic or anaphylactic reactions to monoclonal antibodies;
7. History of tuberculosis or positive PPD test;
8. History of clinically significant chronic systemic infections;
9. Female subjects who are pregnant, planning to become pregnant.

The duration of subject participation is approximately 28 weeks, including a screening period of 2-4 weeks, a treatment/follow-up period of 12 weeks ending on Day 84, and a blood draw for ADA/PK analysis collected 12 weeks later on Day 168.

Approximately 171 subjects with moderate to severe acne are randomized to one of the three treatment groups (0.2 mg/kg antibody, 0.6 mg/kg antibody, or placebo) in a 1:1:1 ratio. Approximately 57 subjects will be randomized to each of the three treatment groups. No adjustment for multiple testing is planned.

Subjects are stratified by study site and their baseline inflammatory lesion count (20 to 40 vs. 41 or more). Study drug is administered subcutaneously (SC) on Days 0, 28, and 56. Subjects are under close observation and will not be discharged from the facility until at least 1 hour after their injection.

Following initial dosing on Day 0, subjects are assessed every 2 weeks for the first 56 days, and then at the Day 84 visit, which occurs 4 weeks following the last dose of study drug (*see,* Table 4). Clinical evaluations include acne-related lesion counts (inflammatory, non inflammatory, and total facial lesions) and separate acne severity grades are given for the facial and non-facial regions, using the Investigator's Global Assessment (IGA), to be performed by the same investigator over the course of the study (see, Table 1). At select sites, the acne-related facial lesions are photographed. The impact of acne on the subjects' disease burden is measured using the Cardiff Acne Disability Index (CADI; see, Table 3).

**Table 4: Schedule of Assessments**

| | | **Screening** | **Treatment and Follow-up** | | | | | | |
|---|---|---|---|---|---|---|---|---|---|
| | **Study Day** | **-28 to -14** | **0** | **14** | **28** | **42** | **56** | **84** | **168** |
| **Assessment** | **Window³** | | | **±3** | **±3** | **±3** | **±3** | **±3** | **±10** |
| **Informed consent/assent** | | X | | | | | | | |
| **Inclusion/exclusion** | | X | X | | | | | | |
| **Demographics, medical history** | | X | | | | | | | |
| **Adverse events** | | X | X | X | X | X | X | X | |
| **Concomitant medications** | | X | X | X | X | X | X | X | |
| **Chest X-ray** | | X | | | | | | | |
| **Complete physical exam** | | X | | | | | | X | |
| **Vital signs** | | X | X | | X | X | X | X | |
| **Investigator's Global Assessment (IGA)¹** | | X | X | | X | X | X | X | |
| **Inflammatory facial lesion count** | | X | X | | X | X | X | X | |
| **Non-inflammatory facial lesion count** | | X | X | | X | X | X | X | |
| **Cardiff Acne Disability Index (CADI)** | | | X | | X | | X | X | |
| **Photography (select sites only)** | | | X | | X | X | X | X | |
| **Facial cutaneous evaluation²** | | | X | | X | X | X | X | |
| **Pregnancy test** | | X | X | | | | | X | |
| **hs-CRP** | | X | X | | X | | X | X | |
| **Hematology Panel** | | X | X | | X | | X | X | |
| **Chemistry Panel** | | X | X | | X | | X | X | |
| **Urinalysis** | | X | X | | X | | X | X | |
| **Cytokine evaluation** | | | X | | X | | X | X | |
| **Retrospective analysis blood sample** | | | X | | X | | X | X | |
| **PK** | | | X | | X | | X | X | X |
| **ADA** | | | X | | | | | X | X |
| **Study drug administration** | | | X | | X | | X | | |

| | | | | | | | | | |
|---|---|---|---|---|---|---|---|---|---|
| ¹ See Table 1 for the IGA (facial and non-facial). The assessment will be performed separately for facial acne and for non-facial (back, shoulders, chest, and neck) acne. ² See Table 2 for the facial cutaneous evaluation. | | | | | | | | | |

Safety is assessed by pre- and post-treatment measurements of vital signs, clinical laboratory assessments, and the incidence of treatment-emergent adverse events. In addition, facial cutaneous evaluations are performed including, for example, as described in Table 5. Safety is assessed by the following outcome measures: i.) incidence of treatment-emergent adverse events; ii.) clinically significant changes in vital signs compared to baseline (Day 0 pre-dose); iii.) clinically significant abnormal changes in laboratory values (hematology, chemistry and urinalysis) compared to baseline (Day 0 pre-dose); iv.) facial cutaneous evaluation; and v.) immune responses to gevokizumab. Blood samples for pharmacokinetics (PK), anti-drug antibodies (ADA), and cytokine analysis is collected at the time points shown in Table 4. Concomitant medication is recorded at each visit.

**Table 5: Facial Cutaneous Evaluation**

| **Condition** | **None** | **Mild** | **Moderate** | **Severe** |
|---|---|---|---|---|
| Scaling | No scaling | Barely perceptible, fine scales present in limited areas of the face | Fine scales generalized to all areas of the face | Scaling and peeling of skin over all areas of the face |
| Erythema | No evidence of erythema present | Slight pink coloration | Definite redness | Marked erythema, bright red to dusky dark red in color |
| Itching | No itching | Slight itching, not really bothersome | Definite itching that is somewhat bothersome | Intense itching that may interrupt daily activities and/or sleep |
| Burning | No burning | Slight burning sensation, not really bothersome | Definite warm, burning sensation that is somewhat bothersome | Hot burning sensation that causes definite discomfort and may interrupt daily activities and/or sleep |
| Stinging | No stinging | Slight stinging sensation, not really bothersome | Definite stinging sensation that is somewhat bothersome | Stinging sensation that causes definite discomfort and may interrupt daily activities and/or sleep |

The primary efficacy endpoint in this study is the mean absolute change from baseline in inflammatory facial lesion count at Day 84. The mean change in inflammatory facial lesion count at Day 84 is analyzed using an analysis of covariance model. In this statistical model, treatment group is treated as the main effect and study site and baseline inflammatory facial lesion count as covariates. The least squares means and standard errors of each treatment group is reported along with the difference between least squares means and the associated standard errors for the comparison, confidence intervals of the difference, and p-value. A similar approach is used to analyze the non-inflammatory and facial lesion counts (*e.g*., total facial acne lesion counts). Efficacy effects of treatment with an anti-IL-1β antibody may be observed at Day 28, Day 42, Day 56, and/or Day 84 including, for example, a reduction in total inflammatory lesion count as compared to baseline, a reduction in total non-inflammatory lesion count as compared to baseline, and/or a reduction in total lesion count as compared to baseline.

Secondary efficacy endpoints include the following: i) Mean percent change from baseline in inflammatory facial lesion count at Day 84; ii.) Mean absolute and percent change from baseline at Day 84 in non-inflammatory facial lesion count and/or facial lesion count; iii.) the proportion of subjects with a successful treatment outcome (*e.g*., an improvement of ≥ 2 grades from the baseline grade) at Day 84 according to the dichotomized IGA scale for facial acne; iv.) mean percent change from baseline at Day 84 in total score for the CADI; and v.) mean change in IGA for non-facial acne (*e.g*., back, shoulders, chest, and neck). Efficacy effects of treatment with an anti-IL-1β antibody may be observed at Day 28, Day 42, Day 56, and/or Day 84 including, for example, a reduction in total inflammatory lesion count as compared to baseline, a reduction in total non-inflammatory lesion count as compared to baseline, and/or a reduction in total lesion count as compared to baseline.

Additional or alternative efficacy endpoints may include a mean absolute or percent change from baseline in scaling, erythema, itching, burning, and/or stinging of acne in one or more areas of the skin.

Additional or alternative efficacy endpoints may also include a mean absolute or percent change from baseline in scaling, erythema, itching, burning, and/or stinging of acne on one or more non-facial areas of the skin (*e.g.,* skin of the neck, back, shoulders, and/or chest).

Additional assessments include measures of biologic and clinical activity, including non-cutaneous parameters, such as for example i.) Inflammatory markers (*e.g*., CRP and/or ESR); and ii.) analysis of cytokines, including, but not limited to, adiponectin, resistin, leptin, visfatin, PAI-1, TNFα, IFNγ, IL-1, IL-1Ra, IL-6, IL-8, RANTES, IL-1α and MCP-1.

Data obtained from this study demonstrates a clinical benefit in the treatment acne of subjects, resulting from administration of the IL-1β antibody, including, for some subjects, improvement in multiple parameters.

Additionally, an unblinded interim analysis is performed after a portion of the planned number of subjects have enrolled in the clinical trial to provide information useful in evaluating the safety and efficacy of a high affinity anti-IL-1β antibody or binding fragment thereof (*e.g*., gevokizumab). Data obtained from such an unblinded interim analysis demonstrates that the administration of 0.2 mg/kg gevokizumab, 0.6 mg/kg gevokizumab, and/or placebo to a group of subjects results in an improvement in efficacy endpoints including, mean absolute change from baseline in total inflammatory lesions, total non-inflammatory lesions, and total lesions (*see,* Table 6).

**Table 6: Facial Lesion Counts by Visit**

| | **Inflammatory Lesions Total** | | | **Non-Inflammatory Lesions Total** | | | **Total Lesions** | | |
|---|---|---|---|---|---|---|---|---|---|
| **Visit** | **Observed** | **Absolute CFB** | **Percent CFB** | **Observed** | **Absolute CFB** | **Percent CFB** | **Observed** | **Absolute CFB** | **Percent CFB** |
| **Baseline** | | | | | | | | | |
| n | 97 | | | 97 | | | 97 | | |
| Mean | 30.6 | | | 41 | | | 71.7 | | |
| | | | | | | | | | |
| **Day 28** | | | | | | | | | |
| n | 76 | 76 | 76 | 74 | 74 | 74 | 74 | 74 | 74 |
| Mean | 23.4 | -6.6 | -21.1 | 35.2 | -6 | -9.5 | 58.5 | -12.9 | -16.9 |
| | | | | | | | | | |
| **Day 42** | | | | | | | | | |
| n | 64 | 64 | 64 | 63 | 63 | 63 | 63 | 63 | 63 |
| Mean | 19.6 | -9.8 | -32.5 | 34.6 | -6.5 | -0.9 | 3.9 | -16.5 | -23.9 |
| | | | | | | | | | |
| **Day 56** | | | | | | | | | |
| n | 53 | 53 | 53 | 53 | 53 | 53 | 53 | 53 | 53 |
| Mean | 18.1 | -10.6 | -36.4 | 29.2 | -8.3 | -3.6 | 47.3 | -18.8 | -29.7 |
| | | | | | | | | | |
| **Day 84/Early Termination** | | | | | | | | | |
| n | 51 | 51 | 51 | 51 | 51 | 51 | 51 | 51 | 51 |
| Mean | 17.7 | -11.7 | -37.8 | 28.7 | -11.9 | -10.2 | 464 | -23.7 | -35.2 |

| | | | | | | | | | |
|---|---|---|---|---|---|---|---|---|---|
| CFB = Change From Baseline | | | | | | | | | |

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (*i.e.,* meaning "including, but not limited to,") unless otherwise noted. Wherever an open-ended term is used to describe a feature or element of the invention, it is specifically contemplated that a closed-ended term can be used in place of the open-ended term without departing from the spirit and scope of the invention. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*e.g*., "such as") provided herein, is intended merely to better illuminate the invention. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those working in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. An anti-IL-1β antibody or binding fragment thereof for use in treating acne in a subject, wherein the acne is not responsive to treatment with one or more anti-microbial agents.

2. An anti-IL-1β antibody or binding fragment thereof for use of claim 1, wherein the anti-microbial agent is an antibiotic.

3. An anti-IL-1β antibody or binding fragment thereof for use of claim 2, wherein the antibiotic is an oral antibiotic.

4. An anti-IL-1β antibody or binding fragment thereof for use of any one of claims 1 - 3, wherein the acne is acne vulgaris.

5. An anti-IL-1β antibody or binding fragment thereof for use of any one of claims 2-4, wherein the antibiotic is selected from the group consisting of: a penicillin, a polyketide antibiotic, a cephalosporin, a lincosamide, a quinolone, a folic acid synthesis inhibitor, a tetracycline, a rifamycin, a sulfonamide, an aminoglycoside, fusidic acid, a polypeptide antibiotic, a lipopeptide antibiotic, chloramphenicol and mupirocin.

6. An anti-IL-1β antibody or binding fragment thereof for use of claim 5, wherein the antibiotic is an oral antibiotic, or a topical antibiotic; the penicillin is penicillin, amoxicillin, benzylpenicillin, ampicillin, or augmentin; the polyketide antibiotic is macrolide, azithromycin, erythromycin, or clarithromycin; the cephalosporin is cefadroxil, cefixime, or cephalexin; the lincosamide is clindamycin; the quinolone is ciprofloxacin, levofloxacin, or moxifloxacin; the folic acid synthesis inhibitor is a dihydrofolate reductase inhibitor, trimethoprim, dapsone, or co-trimoxazole; the tetracycline is tetracycline, minocycline, doxycycline, demeclocycline, or oxytetracycline; the rifamycin is rifampicin, rifabutin, or rifapentine; the sulfonamide is sulfamethoxazole, or sulfacetamide; the aminoglycoside is neomycin, amikacin, or tobramycin; wherein the polypeptide antibiotic is bacitracin, or polymixin B; or the lipopeptide antibiotic is daptomycin.

7. An anti-IL-1β antibody or binding fragment thereof for use of claim 1, wherein the acne is associated with *P. acnes* or *S. aureus.*

8. An anti-IL-1β antibody or binding fragment thereof for use of any one of claims 1-7, wherein the antibody or binding fragment thereof binds to human IL-1β with a dissociation constant of about 1 nM or less, about 250 pM or less, about 50 pM or less, about 10 pM or less, about 1 pM or less, or about 0.3 pM or less.

9. An anti-IL-1β antibody or binding fragment thereof for use of claims 1-7, wherein the anti-IL-1β antibody or binding fragment thereof is a neutralizing antibody.

10. An anti-IL-1β antibody or binding fragment thereof for use of any one of claims 1-7, wherein the anti-IL-1β antibody or binding fragment thereof binds to an IL-1β epitope such that the bound antibody or fragment substantially permits the binding of IL- 1β to IL-1 receptor I (IL-1RI).

11. An anti-IL-1β antibody or binding fragment thereof for use of any one of claims 1-7, wherein the antibody or binding fragment thereof does not detectably bind to IL-1α, IL-1R or IL-1Ra.

12. An anti-IL-1β antibody or binding fragment thereof for use of any one of claims 1-7, wherein the anti-IL-1β antibody or binding fragment thereof comprises a heavy chain variable region comprising: a heavy chain complementarity determining region 1 (HCDR1) comprising TSGMGVG (SEQ ID NO: 13), a heavy chain complementarity determining region 2 (HCDR2) comprising HIWWDGDESYNPSLK (SEQ ID NO: 14), and/or a heavy chain complementarity determining region 3 (HCDR3) comprising NRYDPPWFVD (SEQ ID NO: 15); and/or a light chain variable region comprising: a light chain complementarity determining region 1 (LCDR1) comprising RASQDISNYLS (SEQ ID NO: 16), a light chain complementarity determining region 2 (LCDR2) comprising YTSKLHS (SEQ ID NO: 17), and/or a light chain complementarity determining region 3 (LCDR3) comprising LQGKMLPWT (SEQ ID NO: 18).

13. An anti-IL-1β antibody or binding fragment thereof for use of any one of claims 1-7, wherein the antibody or binding fragment thereof comprises the light chain variable region of SEQ ID NO: 5 and the heavy chain variable region of SEQ ID NO: 6.

14. An anti-IL-1β antibody or binding fragment thereof for use of any one of claims 1-7, wherein the antibody or binding fragment thereof competes with the binding of an antibody having the light chain variable region of SEQ ID NO: 5 and the heavy chain variable region of SEQ ID NO: 6.

15. An anti-IL-1β antibody or binding fragment thereof for use of any one of claims 1-7, wherein the antibody or binding fragment thereof binds to an epitope of IL-1β that is substantially the same as the epitope bound by an antibody having the light chain variable region of SEQ ID NO: 5 and the heavy chain variable region of SEQ ID NO: 6.

16. An anti-IL-1β antibody or binding fragment thereof for use of any one of claims 1-7, wherein the antibody or binding fragment thereof binds to an epitope contained in the amino acid sequence ESVDPKNYPKKKMEKRFVFNKIE (SEQ ID NO: 1) which corresponds to residues 83-105 of human IL-1β (SEQ ID NO: 35).

17. An anti-IL-1β antibody or binding fragment thereof for use of any one of claims 1-7, wherein the binding fragment is selected from the group of Fab, Fab', F(ab')2, and Fv fragments; diabodies; linear antibodies; single-chain antibody molecules including scFv; multispecific antibody fragments including as bispecific, trispecific, and multispecific antibodies; minibodies; chelating recombinant antibodies; tribodies or bibodies; intrabodies; nanobodies; small modular immunopharmaceuticals (SMIP), adnectins, binding-domain immunoglobulin fusion proteins; camelized antibodies; and VHH containing antibodies

18. An anti-IL-1β antibody or binding fragment thereof for use of any one of claims 1-7, wherein the antibody or binding fragment thereof is human or humanized.

19. An anti-IL-1β antibody or binding fragment thereof for use of any one of claims 1-18, wherein the anti-IL-1β antibody or binding fragment thereof is administered by subcutaneous, intravenous, intradermal or intramuscular injection.

20. An anti-IL-1β antibody or binding fragment thereof for use of any one of claims 1-18, wherein the anti-IL-1β antibody or binding fragment thereof is administered in a dose of 1mg/kg or less.

21. An anti-IL-1β antibody or binding fragment thereof for use of any one of claims 1-18, wherein the anti-IL-1β antibody or binding fragment thereof is administered in a dose of 0.2 mg/kg or 0.6 mg/kg.

22. An anti-IL-1β antibody or binding fragment thereof for use of any one of claims 1-18, wherein the anti-IL-1β antibody or binding fragment thereof is administered in a dose of less than 100 mg.

23. An anti-IL-1β antibody or binding fragment thereof for use of any one of claims 1-18, wherein the anti-IL-1β antibody or binding fragment thereof is administered monthly, every two months, every three months, or every six months.

24. An anti-IL-1β antibody or binding fragment thereof for use of any one of claims 1-23, wherein one or more active agents used for the treatment of acne are administered in conjunction with the anti-IL-1β antibody or binding fragment thereof.

25. An anti-IL-1β antibody or binding fragment thereof for use of claim 24, wherein the one or more active agents are administered as a topical treatment or an oral treatment.

26. An anti-IL-1β antibody or binding fragment thereof for use of claim 24, wherein the one or more active agents are selected from the group consisting of: benzoyl peroxide, a retinoid, isotretinoin, a corticosteroid, a hormone therapy, azelaic acid, a topical antibiotic, and an oral antibiotic.

27. An anti-IL-1β antibody or binding fragment thereof for use of claim 26, wherein the retinoid is retinol, retinal, tretinoin, isotretinoin, adapalene, or tazarotene, wherein the corticosteroid is prednisone, wherein the hormone therapy is an estrogen and/or progestin, a glucocorticoid, or an antiandrogen, wherein the glucocorticoid is prednisone, wherein the antiandrogen is spironolactone or flutamide, wherein the topical antibiotic is clindamycin, zithromycin, erythromycin, minocycline, or tetracycline, or wherein the oral antibiotic is tetracycline, erythromycin, doxycycline, minocycline, clindamycin, ampicillin, cephalosporin, or trimethoprim/sulfamethoxazole.

## Patentansprüche

1. Ein anti-IL-1β Antikörper oder Bindungsfragment davon zur Verwendung bei der Behandlung von Akne in einer Person, wobei die Akne nicht auf die Behandlung mit einem oder mehreren anti-mikrobiellen Wirkstoffen anspricht.

2. Ein anti-IL-1β Antikörper oder Bindungsfragment davon zur Verwendung gemäß Anspruch 1, wobei der anti-mikrobielle Wirkstoff ein Antibiotikum ist.

3. Ein anti-IL-1β Antikörper oder Bindungsfragment davon zur Verwendung gemäß Anspruch 2, wobei das Antibiotikum ein orales Antibiotikum ist.

4. Ein anti-IL-1β Antikörper oder Bindungsfragment davon zur Verwendung gemäß einem der Ansprüche 1-3, wobei die Akne acne vulgaris ist.

5. Ein anti-IL-1β Antikörper oder Bindungsfragment davon zur Verwendung gemäß einem der Ansprüche 2-4, wobei das Antibiotikum ausgewählt ist aus der Gruppe bestehend aus: einem Penicillin, einem Polyketideantibiotikum, einem Cephalosporin, einem Lincosamid, einem Chinolon, einem Folsäure-Synthesisinhibitor, einem Tetrazyklin, einem Rifamycin, einem Sulfonamid, einem Aminoglycosid, Fusidinsäure, einem PolypeptidAntibiotikum, einem Lipopeptid-Antibiotikum, Chloramphenicol und Mupirocin.

6. Ein anti-IL-1β Antikörper oder Bindungsfragment davon zur Verwendung gemäß Anspruch 5, wobei das Antibiotikum ein orales Antibiotikum ist oder ein topisches Antibiotikum; das Penicillin ist Penicillin, Amoxicillin, Benzylpenicillin, Ampicillin, oder Augmentin; das Polyketidantibiotikum ist Macrolid, Azithromycin, Erythromycin, oder Clarithromycin; das Cephalosporin ist Cefadroxil, Cefixim, oder Cephalexin; das Lincosamide ist Clindamycin; das Chinolon ist Ciprofloxacin, Levofloxacin, oder Moxifloxacin; der Folsäure-Syntheseinhibitor ist ein Dihydrofolatreduktaseinhibitor, Trimethoprim, Dapson, oder Co-Trimoxazol; das Tetracyclin ist Tetracyclin, Minocyclin, Doxycyclin, Demeclocyclin, oder Oxytetracyclin; das Rifamycin ist Rifampicin, Rifabutin, oder Rifapentin; das Sulfonamid ist Sulfamethoxazol, oder Sulfacetamid; das Aminoglycosid ist Neomycin, Amikacin, oder Tobramycin; wobei das Polypeptidantibiotikum Bacitracin oder Polymixin B ist; oder das Lipopeptid-Antibiotikum Daptomycin ist.

7. Ein anti-IL-1β Antikörper oder Bindungsfragment davon zur Verwendung gemäß Anspruch 1, wobei die Akne mit *P. acnes* oder *S. aureus* assoziiert ist.

8. Ein anti-IL-1β Antikörper oder Bindungsfragment davon zur Verwendung gemäß einem der Ansprüche 1-7, wobei der Antikörper oder Bindungsfragment davon an humanes IL-1β mit einer Dissoziationskonstante von etwa 1 nM oder weniger, etwa 250 pM oder weniger, etwa 50 pM oder weniger, etwa 10 pM oder weniger, etwa 1 pM oder weniger, oder etwa 0,3 pM oder weniger, bindet.

9. Ein anti-IL-1β Antikörper oder Bindungsfragment davon zur Verwendung gemäß einem der Ansprüche 1-7, wobei der Anti-IL-1β Antikörper oder Bindungsfragment davon ein neutralisierender Antikörper ist.

10. Ein anti-IL-1β Antikörper oder Bindungsfragment davon zur Verwendung gemäß einem der Ansprüche 1-7, wobei der Anti-IL-1β Antikörper oder Bindungsfragment davon an ein In-1β Epitop bindet, derart, dass der gebundene Antikörper oder Fragment im Wesentlichen die Bindung von IL-1β an IL-1 Rezeptor I (IL-1RI) erlaubt.

11. Ein anti-IL-1β Antikörper oder Bindungsfragment davon zur Verwendung gemäß einem der Ansprüche 1-7, wobei der Antikörper oder Bindungsfragment davon nicht nachweisbar an IL-1α, IL-1R oder IL-1Ra bindet.

12. Ein anti-IL-1β Antikörper oder Bindungsfragment davon zur Verwendung gemäß einem der Ansprüche 1-7, wobei der anti-IL-1β Antikörper oder Bindungsfragment davon eine variable Region der schweren Kette umfassend: eine Komplementarität bestimmende Region 1 (HCDR1) der schweren Kette umfassend TSGMGVG (SEQ ID NO: 13) umfasst, eine Komplementarität bestimmende Region 2 (HCDR2) der schweren Kette umfassend HIWWDGDESYNPSLK (SEQ ID NO: 14), und/oder eine Komplementarität bestimmende Region 3 (HCDR3) der schweren Kette umfassend NRYDPPWFVD (SEQ ID NO: 15); und/oder eine variable Region der leichten Kette umfassend: eine Komplementarität bestimmende Region 1 (LCDR1) der leichten Kette umfassend RASQDISNYLS (SEQ ID NO: 16), eine Komplementarität bestimmende Region 2 (LCDR2) der leichten Kette umfassend YTSKLHS (SEQ ID NO: 17), und/oder Komplementarität bestimmende Region 3 (LCDR3) der leichten Kette umfassend LQGKMLPWT (SEQ ID NO: 18).

13. Ein anti-IL-1β Antikörper oder Bindungsfragment davon zur Verwendung gemäß einem der Ansprüche 1-7, wobei der Antikörper oder Bindungsfragment davon die variable Region der leichten Kette der SEQ ID NO: 5 umfasst und die variable Region der schweren Kette der SEQ ID NO: 6.

14. Ein anti-IL-1β Antikörper oder Bindungsfragment davon zur Verwendung gemäß einem der Ansprüche 1-7, wobei der Antikörper oder Bindungsfragment mit der Bindung eines Antikörpers mit der variablen Region der leichten Kette der SEQ ID NO: 5 und der variablen Region der schweren Kette der SEQ ID NO: 6 konkurriert.

15. Ein anti-IL-1β Antikörper oder Bindungsfragment davon zur Verwendung gemäß einem der Ansprüche 1-7, wobei der Antikörper oder Bindungsfragment davon an ein Epitop des In-1β bindet, das im Wesentlichen dasselbe ist wie das Epitop, das von einem Antikörper mit der variablen Region der leichten Kette der SEQ ID NO: 5 und der variablen Region der schweren Kette der SEQ ID NO: 6 gebunden wird.

16. Ein anti-IL-1β Antikörper oder Bindungsfragment davon zur Verwendung gemäß einem der Ansprüche 1-7, wobei der Antikörper oder Bindungsfragment davon das an ein Epitop bindet das in der Aminosäuresequenz ESVDPKNYPKKKMEKRFVFNKIE (SEQ ID NO: 1) enthalten ist, die den Resten 83-105 des humanen In-1β (SEQ ID NO: 35) entspricht.

17. Ein anti-IL-1β Antikörper oder Bindungsfragment davon zur Verwendung gemäß einem der Ansprüche 1-7, wobei das Bindungsfragment ausgesucht ist aus der Gruppe von Fab, Fab', F(ab')2, und Fv Fragmenten; Diabodies; linearer Antikörper; Einzelkettenantikörpermoleküle enthaltend scFv; multispezifische Antikörperfragmente enthaltend bispezifische, trispezifische und multispezifische Antikörper; Minibodies; chelatierende, rekombinante Antikörper; Tribodies oder Bibodies; Intrabodies; Nanobodies; kleine moduläre Immunopharmazeutika (SMIP), Adnectine, Binde-Domain Immunoglobulinfusionsproteine; Kamelantikörper; und VHH enthaltende Antikörper.

18. Ein anti-IL-1β Antikörper oder Bindungsfragment davon zur Verwendung gemäß einem der Ansprüche 1-7, wobei der Antikörper oder Bindungsfragment davon human oder humanisiert ist.

19. Ein anti-IL-1β Antikörper oder Bindungsfragment davon zur Verwendung gemäß einem der Ansprüche 1-18, wobei der anti-IL-1β Antikörper oder Bindungsfragment davon mittels subkutaner, intravenöser, intradermaler oder intramuskulärer Injektion verabreicht wird.

20. Ein anti-IL-1β Antikörper oder Bindungsfragment davon zur Verwendung gemäß einem der Ansprüche 1-18, wobei der anti-IL-1β Antikörper oder Bindungsfragment davon in einer Dosis von 1 mg/kg oder weniger verabreicht wird.

21. Ein anti-IL-1β Antikörper oder Bindungsfragment davon zur Verwendung gemäß einem der Ansprüche 1-18, wobei der anti-IL-1β Antikörper oder Bindungsfragment in einer Dosis von 0,2 mg/kg oder 0,6 mg/kg verabreicht wird.

22. Ein anti-IL-1β Antikörper oder Bindungsfragment davon zur Verwendung gemäß einem der Ansprüche 1-18, wobei der anti-IL-1β Antikörper oder Bindungsfragment in einer Dosis von weniger als 100 mg verabreicht wird.

23. Ein anti-IL-1β Antikörper oder Bindungsfragment davon zur Verwendung gemäß einem der Ansprüche 1-18, wobei der anti-IL-1β Antikörper oder Bindungsfragment davon monatlich, jeden zweiten Monat, alle drei Monate, oder alle sechs Monate verabreicht wird.

24. Ein anti-IL-1β Antikörper oder Bindungsfragment davon zur Verwendung gemäß einem der Ansprüche 1-23, wobei ein oder mehrere Wirkstoffe, die für die Behandlung von Akne verwendet werden, in Verbindung mit dem anti-IL-1β Antikörper oder Bindungsfragment davon verabreicht werden.

25. Ein anti-IL-1β Antikörper oder Bindungsfragment davon zur Verwendung gemäß Anspruch 24, wobei der eine oder mehrere Wirkstoffe als topische Behandlung oder als orale Behandlung verabreicht werden.

26. Ein anti-IL-1β Antikörper oder Bindungsfragment davon zur Verwendung gemäß Anspruch 24, wobei der eine oder mehrere Wirkstoffe ausgesucht sind aus der Gruppe bestehend aus: Benzoylperoxid, einem Retinoid, Isotretinoin, einem Corticosteroid, einer Hormontherapie, Azelainsäure, einem topischen Antibiotikum, und einem oralen Antibiotikum.

27. Ein anti-IL-1β Antikörper oder Bindungsfragment davon zur Verwendung gemäß Anspruch 26, wobei das Retinoid Retinol, Retinal, Tretinoin, Isotretinoin, Adapalen, oder Tazaroten ist, wobei das Corticosteroid Prednison ist, wobei die Hormontherapie eine Östrogen- und/oder Progestin-, eine Glucocorticoid- oder eine Antiandrogentherapie ist, wobei das Glucocorticoid Prednison ist, wobei das Antiandrogen Spironolacton oder Flutamid ist, wobei das topische Antibiotikum Clindamycin, Zithromycin, Erythromycin, Minocyclin, oder Tetracyclin ist, oder wobei das orale Antibiotikum Tetracyclin, Erythromycin, Doxycyclin, Minocyclin, Clindamycin, Ampicillin, Cephalosporin, oder Trimethoprim/Sulfamethoxazol ist.

## Revendications

1. Anticorps anti-IL-1β ou fragment de fixation de celui-ci pour une utilisation dans le traitement de l'acné chez un sujet, dans lequel l'acné ne répond pas au traitement avec un ou plusieurs agents antimicrobiens.

2. Anticorps anti-IL-1β ou fragment de fixation de celui-ci pour une utilisation selon la revendication 1, dans lequel l'agent antimicrobien est un antibiotique.

3. Anticorps anti-IL-1β ou fragment de fixation de celui-ci pour une utilisation selon la revendication 2, dans lequel l'antibiotique est un antibiotique oral.

4. Anticorps anti-IL-1β ou fragment de fixation de celui-ci pour une utilisation selon l'une quelconque des revendications 1 à 3, dans lequel l'acné est de l'acné vulgaire.

5. Anticorps anti-IL-1β ou fragment de fixation de celui-ci pour une utilisation selon l'une quelconque des revendications 2 à 4, dans lequel l'antibiotique est choisi dans le groupe constitué par : une pénicilline, un antibiotique polycétidique, une céphalosporine, un lincosamide, une quinolone, un inhibiteur de la synthèse de l'acide folique, une tétracycline, une rifamycine, un sulfamide, un aminoglycoside, de l'acide fusidique, un antibiotique polypeptidique, un antibiotique lipopeptidique, du chloramphénicol, et du mupirocine.

6. Anticorps anti-IL-1β ou fragment de fixation de celui-ci pour une utilisation selon la revendication 5, dans lequel l'antibiotique est un antibiotique oral, ou un antibiotique topique ; la pénicilline est de la pénicilline, de l'amoxicilline, de la benzylpénicilline, de l'ampicilline, ou de l'augmentin ; l'antibiotique polycétidique est un macrolide, de l'azithromycine, de l'érythromycine, ou de la clarithromycine ; la céphalosporine est du céfadroxil, de la céfixime, ou de la céphalexine ; le lincosamide est de la clindamycine ; la quinolone est de la ciprofloxacine, de la lévofloxacine, ou de la moxifloxacine ; l'inhibiteur de la synthèse de l'acide folique est un inhibiteur de la dihydrofolate réductase, du triméthoprime, de la dapsone, ou du cotrimoxazole ; la tétracycline est de la tétracycline, de la minocycline, de la doxycycline, de la déméclocycline, ou de l'oxytétracycline ; la rifamycine est de la rifampicine, de la rifabutine, ou de la rifapentine ; le sulfamide est du sulfaméthoxazole, ou du sulfacétamide ; l'aminoglycoside est de la néomycine, de l'amikacine, ou de la tobramycine ; dans lequel l'antibiotique polypeptidique est de la bacitracine, ou de la polymixine B ; ou l'antibiotique lipopeptidique est de la daptomycine.

7. Anticorps anti-IL-1β ou fragment de fixation de celui-ci pour une utilisation selon la revendication 1, dans lequel l'acné est associé à la *P. acnes* ou au *S. aureus.*

8. Anticorps anti-IL-1β ou fragment de fixation de celui-ci pour une utilisation selon l'une quelconque des revendications 1 à 7, dans lequel l'anticorps ou le fragment de fixation de celui-ci se lie à l'IL-1β humain avec une constante de dissociation d'environ 1 nM ou moins, d'environ 250 pM ou moins, d'environ 50 pM ou moins, d'environ 10 pM ou moins, d'environ 1 pM ou moins, ou d'environ 0,3 pM ou moins.

9. Anticorps anti-IL-1β ou fragment de fixation de celui-ci pour une utilisation selon les revendications 1 à 7, dans lequel l'anticorps anti-IL-1β ou le fragment de fixation de celui-ci est un anticorps neutralisant.

10. Anticorps anti-IL-1β ou fragment de fixation de celui-ci pour une utilisation selon l'une quelconque des revendications 1 à 7, dans lequel l'anticorps anti-IL-1β ou le fragment de fixation de celui-ci se lie à un épitope IL-1β de sorte que l'anticorps ou le fragment fixé permet essentiellement la fixation de l'IL-1β au récepteur de type I de l'IL-1 (IL-1RI).

11. Anticorps anti-IL-1β ou fragment de fixation de celui-ci pour une utilisation selon l'une quelconque des revendications 1 à 7, dans lequel l'anticorps ou le fragment de fixation de celui-ci ne se fixe pas de manière détectable sur IL-1α, IL-1R ou IL-1Ra.

12. Anticorps anti-IL-1β ou fragment de fixation de celui-ci pour une utilisation selon l'une quelconque des revendications 1 à 7, dans lequel l'anticorps anti-IL-1β ou le fragment de liaison de celui-ci comprend une région variable de chaîne lourde comprenant : une région 1 déterminant la complémentarité de la chaîne lourde (HCDR1) comprenant TSGMGVG (SEQ ID NO _{:} 13), une région 2 déterminant la complémentarité de la chaîne lourde (HCDR2) comprenant HIWWDGDESYNPSLK (SEQ ID NO _{:} 14), et/ou une région 3 déterminant la complémentarité de la chaîne lourde (HCDR3) comprenant NRYDPPWFVD (SEQ ID NO : 15) ; et/ou une région variable de chaîne légère comprenant : une région 1 déterminant la complémentarité de la chaîne légère (LCDR1) comprenant RASQDISNYLS (SEQ ID NO _{:} 16), une région 2 déterminant la complémentarité de la chaîne légère (LCDR2) comprenant YTSKLHS (SEQ ID NO _{:} 17), et/ou une région 3 déterminant la complémentarité de la chaîne légère 3 (LCDR3) comprenant LQGKMLPWT (SEQ ID NO : 18).

13. Anticorps anti-IL-1β ou fragment de fixation de celui-ci pour une utilisation selon l'une quelconque des revendications 1 à 7, dans lequel l'anticorps ou le fragment de fixation de celui-ci comprend la région variable de la chaîne légère de SEQ ID NO _{:} 5 et la région variable de la chaîne lourde de SEQ ID NO _{:} 6.

14. Anticorps anti-IL-1β ou fragment de fixation de celui-ci pour une utilisation selon l'une quelconque des revendications 1 à 7, dans lequel l'anticorps ou le fragment de fixation de celui-ci entre en concurrence avec la fixation d'un anticorps ayant la région variable de la chaîne légère de SEQ ID NO _{:} 5 et la région variable de la chaîne lourde de SEQ ID NO _{:} 6.

15. Anticorps anti-IL-1β ou fragment de fixation de celui-ci pour une utilisation selon l'une quelconque des revendications 1 à 7, dans lequel l'anticorps ou le fragment de fixation de celui-ci se fixe à un épitope d'IL-1β qui est essentiellement le même que l'épitope fixé par un anticorps ayant la région variable de la chaîne légère de SEQ ID NO _{:} 5 et la région variable de la chaîne lourde de SEQ ID NO _{:} 6.

16. Anticorps anti-IL-1β ou fragment de fixation de celui-ci pour une utilisation selon l'une quelconque des revendications 1 à 7, dans lequel l'anticorps ou le fragment de fixation de celui-ci se fixe à un épitope contenu dans la séquence d'acides aminés ESVDPKNYPKKKMEKRFVFNKIE (SEQ ID NO _{:} 1) qui correspond aux résidus 83 à 105 de l'IL-1β humain (SEQ ID NO _{:} 35).

17. Anticorps anti-IL-1β ou fragment de fixation de celui-ci pour une utilisation selon l'une quelconque des revendications 1 à 7, dans lequel le fragment de fixation est choisi dans le groupe parmi les fragments Fab, Fab', F(ab')2, and Fv ; les anticorps bivalents ; les anticorps linéaires ; les molécules d'anticorps à chaîne unique comprenant scFv ; les fragments d'anticorps multispécifiques notamment comme les anticorps bispécifiques, trispécifiques, et multispécifiques ; les minicorps ; les anticorps recombinants chélatants ; les tricorps ou les bicorps ; les intracorps ; les nanocorps ; les agents immunopharmaceutiques modulaires de petite taille (SMIP), les adnectines, les protéines de fusion du domaine de fixation de l'immunoglobuline ; les anticorps de type camélidé ; et les VHH contenant les anticorps.

18. Anticorps anti-IL-1β ou fragment de fixation de celui-ci pour une utilisation selon l'une quelconque des revendications 1 à 7, dans lequel l'anticorps ou le fragment de fixation de celui-ci est humain ou humanisé.

19. Anticorps anti-IL-1β ou fragment de fixation de celui-ci pour une utilisation selon l'une quelconque des revendications 1 à 18, dans lequel l'anticorps anti-IL-1β ou le fragment de fixation de celui-ci est administré par injection sous cutanée, intraveineuse, intradermique ou intramusculaire.

20. Anticorps anti-IL-1β ou fragment de fixation de celui-ci pour une utilisation selon l'une quelconque des revendications 1 à 18, dans lequel l'anticorps anti-IL-1β ou le fragment de fixation de celui-ci est administré à une dose de 1 mg / kg ou moins.

21. Anticorps anti-IL-1β ou fragment de fixation de celui-ci pour une utilisation selon l'une quelconque des revendications 1 à 18, dans lequel l'anticorps anti-IL-1β ou le fragment de fixation de celui-ci est administré à une dose de 0,2 mg / kg ou de 0,6 mg / kg.

22. Anticorps anti-IL-1β ou fragment de fixation de celui-ci pour une utilisation selon l'une quelconque des revendications 1 à 18, dans lequel l'anticorps anti-IL-1β ou le fragment de fixation de celui-ci est administré à une dose de moins de 100 mg.

23. Anticorps anti-IL-1β ou fragment de fixation de celui-ci pour une utilisation selon l'une quelconque des revendications 1 à 18, dans lequel l'anticorps anti-IL-1β ou le fragment de fixation de celui-ci est administré de façon mensuelle, tous les deux mois, tous les trois mois, ou tous les six mois.

24. Anticorps anti-IL-1β ou fragment de fixation de celui-ci pour une utilisation selon l'une quelconque des revendications 1 à 23, dans lequel un seul ou plusieurs agents actifs utilisés pour le traitement de l'acné sont administrés en association avec l'anticorps anti-IL-1β ou le fragment de fixation de celui-ci.

25. Anticorps anti-IL-1β ou fragment de fixation de celui-ci pour une utilisation selon la revendication 24, dans lequel le seul ou les plusieurs agents actifs sont administrés en tant que traitement topique ou traitement oral.

26. Anticorps anti-IL-1β ou fragment de fixation de celui-ci pour une utilisation selon la revendication 24, dans lequel le seul ou les plusieurs agents actifs sont choisis dans le groupe constitué par : le peroxyde de benzoyle, un rétinoïde, l'isotrétinoïne, un corticostéroïde, une thérapie hormonale, l'acide azélaïque, un antibiotique topique, et un antibiotique oral.

27. Anticorps anti-IL-1β ou fragment de fixation de celui-ci pour une utilisation selon la revendication 26, dans lequel le rétinoïde est le rétinol, le rétinal, la trétinoïne, l'isotrétinoïne, l'adapalène, ou le tazarotène, dans lequel le corticostéroïde est la prednisone, dans lequel la thérapie hormonale est un estrogène et/ou une progestine, un glucocorticoïde, ou un antiandrogène, dans lequel le glucocorticoïde est la prednisone, dans lequel l'antiandrogène est la spironolactone ou le flutamide, dans lequel l'antibiotique topique est la clindamycine, la zithromycine, l'érythromycine, la minocycline, ou la tétracycline, ou dans lequel l'antibiotique oral est la tétracycline, l'érythromycine, la doxycycline, la minocycline, la clindamycine, l'ampicilline, la céphalosporine, ou le triméthoprime / le sulfaméthoxazole.


### SEQUENCE LISTING

<110> XOMA TECHNOLOGY, LTD.
<120> METHODS FOR TREATING ACNE
<130> 3716368.00278
<160> 35
<170> PatentIn version 3.5
<210> 1
   <211> 23
   <212> PRT
   <213> Artificial sequence
<220>
   <223> Synthesized: residues 83-105 of mature IL-1Beta protein
<400> 1
   Glu Ser Val Asp Pro Lys Asn Tyr Pro Lys Lys Lys Met Glu Lys Arg
   1               5                   10                  15
   Phe Val Phe Asn Lys Ile Glu
               20
<210> 2
   <211> 5
   <212> PRT
   <213> Artificial sequence
<220>
   <223> Synthesized: glycine-serine linker
<400> 2
   Gly Gly Gly Gly Ser
   1               5
<210> 3
   <211> 107
   <212> PRT
   <213> Artificial sequence
<220>
   <223> Synthesized: AB5 light chain
<400> 3
   Asp Ile Gln Met Thr Gln Thr Thr Ser Ser Leu Ser Ala Ser Leu Gly
   1               5                   10                  15
   Asp Arg Val Thr Ile Ser Cys Arg Ala Ser Gln Asp Ile Ser Asn Tyr
               20                  25                  30
   Leu Ser Trp Tyr Gln Gln Lys Pro Asp Gly Thr Val Lys Leu Leu Ile
           35                  40                  45
   Tyr Tyr Thr Ser Lys Leu His Ser Gly Val Pro Ser Arg Phe Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Thr Tyr Phe Cys Leu Gln Gly Lys Met Leu Pro Trp
                   85                  90                  95
   Thr Phe Gly Gly Gly Thr Lys Leu Glu Ile Lys
               100                 105
<210> 4
   <211> 120
   <212> PRT
   <213> Artificial sequence
<220>
   <223> Synthesized: AB5 heavy chain
<400> 4
   Gln Val Thr Leu Lys Glu Ser Gly Pro Gly Ile Leu Lys Pro Ser Gln
   1               5                   10                  15
   Thr Leu Ser Leu Thr Cys Ser Phe Ser Gly Phe Ser Leu Ser Thr Ser
               20                  25                  30
   Gly Met Gly Val Gly Trp Ile Arg Gln Pro Ser Gly Lys Gly Leu Glu
           35                  40                  45
   Trp Leu Ala His Ile Trp Trp Asp Gly Asp Glu Ser Tyr Asn Pro Ser
       50                  55                  60
   Leu Lys Thr Gln Leu Thr Ile Ser Lys Asp Thr Ser Arg Asn Gln Val
   65                  70                  75                  80
   Phe Leu Lys Ile Thr Ser Val Asp Thr Val Asp Thr Ala Thr Tyr Phe
                   85                  90                  95
   Cys Ala Arg Asn Arg Tyr Asp Pro Pro Trp Phe Val Asp Trp Gly Gln
               100                 105                 110
   Gly Thr Leu Val Thr Val Ser Ser
           115                 120
<210> 5
   <211> 107
   <212> PRT
   <213> Artificial sequence
<220>
   <223> Synthesized: AB7 light chain
<400> 5
<210> 6
   <211> 120
   <212> PRT
   <213> Artificial sequence
<220>
   <223> Synthesized: AB7 heavy chain
<400> 6
<210> 7
   <211> 107
   <212> PRT
   <213> Artificial sequence
<220>
   <223> Synthesized: AB6 light chain
<400> 7
   Asp Ile Gln Met Thr Gln Ser Thr Ser Ser Leu Ser Ala Ser Leu Gly
   1 5 10 15
   Asp Arg Val Thr Ile Thr Cys Arg Ala Ser Gln Asp Ile Ser Asn Tyr
   20 25 30
   Leu Ser Trp Tyr Gln Gln Lys Pro Gly Lys Thr Val Lys Leu Leu Ile
   35 40 45
   Tyr Tyr Thr Ser Lys Leu His Ser Gly Val Pro Ser Arg Phe Ser Gly
   50 55 60
   Ser Gly Ser Gly Thr Asp Tyr Thr Leu Thr Ile Ser Ser Leu Gln Gln
   65 70 75 80
   Glu Asp Phe Ala Thr Tyr Phe Cys Leu Gln Gly Lys Met Leu Pro Trp
   85 90 95
   Thr Phe Gly Gln Gly Thr Lys Leu Glu Ile Lys
   100 105
<210> 8
   <211> 120
   <212> PRT
   <213> Artificial sequence
<220>
   <223> Synthesized: AB6 heavy chain
<400> 8
   Gln Val Gln Leu Gln Glu Ser Gly Pro Gly Leu Leu Lys Pro Ser Gln
   1               5                   10                  15
   Thr Leu Ser Leu Thr Cys Ser Phe Ser Gly Phe Ser Leu Ser Thr Ser
               20                  25                  30
   Gly Met Gly Val Gly Trp Ile Arg Gln Pro Ser Gly Lys Gly Leu Glu
           35                  40                  45
   Trp Leu Ala His Ile Trp Trp Asp Gly Asp Glu Ser Tyr Asn Pro Ser
       50                  55                  60
   Leu Lys Ser Gln Leu Thr Ile Ser Lys Asp Thr Ser Lys Asn Gln Val
   65                  70                  75                  80
   Ser Leu Lys Ile Thr Ser Val Thr Ala Ala Asp Thr Ala Thr Tyr Phe
                   85                  90                  95
   Cys Ala Arg Asn Arg Tyr Asp Pro Pro Trp Phe Val Asp Trp Gly Gln
               100                 105                 110
   Gly Thr Leu Val Thr Val Ser Ser
           115                 120
<210> 9
   <211> 107
   <212> PRT
   <213> Artificial sequence
<220>
   <223> Synthesized: AB8 light chain
<400> 9
   Asp Ile Gln Met Thr Gln Ser Thr Ser Ser Leu Ser Ala Ser Leu Gly
   1               5                   10                  15
   Asp Arg Val Thr Ile Thr Cys Arg Ala Ser Gln Asp Ile Ser Asn Tyr
               20                  25                  30
   Leu Ser Trp Tyr Gln Gln Lys Pro Gly Lys Thr Val Lys Leu Leu Ile
           35                  40                  45
   Tyr Tyr Thr Ser Lys Leu His Ser Gly Val Pro Ser Arg Phe Ser Gly
       50                  55                  60
   Ser Gly Ser Gly Thr Asp Tyr Thr Leu Thr Ile Ser Ser Leu Gln Gln
   65                  70                  75                  80
   Glu Asp Phe Ala Thr Tyr Phe Cys Leu Gln Gly Lys Met Leu Pro Trp
                      85                  90                  95
   Thr Phe Gly Gln Gly Thr Lys Leu Glu Ile Lys
               100                 105
<210> 10
   <211> 120
   <212> PRT
   <213> Artificial sequence
<220>
   <223> Synthesized: AB8 heavy chain
<400> 10
   Gln Val Gln Leu Gln Glu Ser Gly Pro Gly Leu Leu Lys Pro Ser Gln
   1               5                   10                  15
   Thr Leu Ser Leu Thr Cys Ser Phe Ser Gly Phe Ser Leu Ser Thr Ser
               20                  25                  30
   Gly Met Gly Val Gly Trp Ile Arg Gln Pro Ser Gly Lys Gly Leu Glu
           35                  40                  45
   Trp Leu Ala His Ile Trp Trp Asp Gly Asp Glu Ser Tyr Asn Pro Ser
       50                  55                  60
   Leu Lys Ser Gln Leu Thr Ile Ser Lys Asn Thr Ser Lys Asn Gln Val
   65                  70                  75                  80
   Ser Leu Lys Ile Thr Ser Val Thr Ala Ala Asp Thr Ala Thr Tyr Phe
                   85                  90                  95
   Cys Ala Arg Asn Arg Tyr Asp Pro Pro Trp Phe Val Asp Trp Gly Gln
               100                 105                 110
   Gly Thr Leu Val Thr Val Ser Ser
           115                 120
<210> 11
   <211> 107
   <212> PRT
   <213> Artificial sequence
<220>
   <223> Synthesized: AB9 light chain
<400> 11
<210> 12
   <211> 120
   <212> PRT
   <213> Artificial sequence
<220>
   <223> Synthesized: AB9 heavy chain
<400> 12
   Gln Val Gln Leu Gln Glu Ser Gly Pro Gly Leu Val Lys Pro Ser Gln
   1 5 10 15
   Thr Leu Ser Leu Thr Cys Ser Phe Ser Gly Phe Ser Leu Ser Thr Ser
   20 25 30
   Gly Met Gly Val Gly Trp Ile Arg Gln Pro Ser Gly Lys Gly Leu Glu
   35 40 45
   Trp Leu Ala His Ile Trp Trp Asp Gly Asp Glu Ser Tyr Asn Pro Ser
   50 55 60
   Leu Lys Ser Arg Leu Thr Ile Ser Lys Asn Thr Ser Lys Asn Gln Val
   65 70 75 80
   Ser Leu Lys Ile Thr Ser Val Thr Ala Ala Asp Thr Ala Val Tyr Phe
   85 90 95
   Cys Ala Arg Asn Arg Tyr Asp Pro Pro Trp Phe Val Asp Trp Gly Gln
   100 105 110
   Gly Thr Leu Val Thr Val Ser Ser
   115 120
<210> 13
   <211> 7
   <212> PRT
   <213> Artificial sequence
<220>
   <223> Synthesized: HCDR1
<400> 13
   Thr Ser Gly Met Gly Val Gly
   1 5
<210> 14
   <211> 15
   <212> PRT
   <213> Artificial sequence
<220>
   <223> Synthesized: HCDR2
<400> 14
   His Ile Trp Trp Asp Gly Asp Glu Ser Tyr Asn Pro Ser Leu Lys
   1 5 10 15
<210> 15
   <211> 10
   <212> PRT
   <213> Artificial sequence
<220>
   <223> Synthesized: HCDR3
<400> 15
   Asn Arg Tyr Asp Pro Pro Trp Phe Val Asp
   1 5 10
<210> 16
   <211> 11
   <212> PRT
   <213> Artificial sequence
<220>
   <223> Synthesized: LCDR1
<400> 16
   Arg Ala Ser Gln Asp Ile Ser Asn Tyr Leu Ser
   1 5 10
<210> 17
   <211> 7
   <212> PRT
   <213> Artificial sequence
<220>
   <223> Synthesized: LCDR2
<400> 17
   Tyr Thr Ser Lys Leu His Ser
   1 5
<210> 18
   <211> 9
   <212> PRT
   <213> Artificial sequence
<220>
   <223> Synthesized: LCDR3
<400> 18
   Leu Gln Gly Lys Met Leu Pro Trp Thr
   1 5
<210> 19
   <211> 30
   <212> PRT
   <213> Artificial sequence
<220>
   <223> Synthesized: AB5 HFW1
<400> 19
   Gln Val Thr Leu Lys Glu Ser Gly Pro Gly Ile Leu Lys Pro Ser Gln
   1 5 10 15
   Thr Leu Ser Leu Thr Cys Ser Phe Ser Gly Phe Ser Leu Ser
   20 25 30
<210> 20
   <211> 14
   <212> PRT
   <213> Artificial sequence
<220>
   <223> Synthesized: AB5 HFW2
<400> 20
   Trp Ile Arg Gln Pro Ser Gly Lys Gly Leu Glu Trp Leu Ala
   1 5 10
<210> 21
   <211> 33
   <212> PRT
   <213> Artificial sequence
<220>
   <223> Synthesized: AB5 HFW3
<400> 21
   Thr Gln Leu Thr Ile Ser Lys Asp Thr Ser Arg Asn Gln Val Phe Leu
   1 5 10 15
   Lys Ile Thr Ser Val Asp Thr Val Asp Thr Ala Thr Tyr Phe Cys Ala
   20 25 30
   Arg
<210> 22
   <211> 11
   <212> PRT
   <213> Artificial sequence
<220>
   <223> Synthesized: AB5 HFW4
<400> 22
   Trp Gly Gln Gly Thr Leu Val Thr Val Ser Ser
   1 5 10
<210> 23
   <211> 23
   <212> PRT
   <213> Artificial sequence
<220>
   <223> Synthesized: AB5 LFW1
<400> 23
   Asp Ile Gln Met Thr Gln Thr Thr Ser Ser Leu Ser Ala Ser Leu Gly
   1               5                   10                  15
   Asp Arg Val Thr Ile Ser Cys
               20
<210> 24
   <211> 15
   <212> PRT
   <213> Artificial sequence
<220>
   <223> Synthesized: AB5 LFW2
<400> 24
   Trp Tyr Gln Gln Lys Pro Asp Gly Thr Val Lys Leu Leu Ile Tyr
   1               5                   10                  15
<210> 25
   <211> 32
   <212> PRT
   <213> Artificial sequence
<220>
   <223> Synthesized: AB5 LFW3
<400> 25
   Gly Val Pro Ser Arg Phe Ser Gly Ser Gly Ser Gly Thr Asp Tyr Ser
   1               5                   10                  15
   Leu Thr Ile Ser Asn Leu Glu Gln Glu Asp Ile Ala Thr Tyr Phe Cys
               20                  25                  30
<210> 26
   <211> 10
   <212> PRT
   <213> Artificial sequence
<220>
   <223> Synthesized: AB5 LFW4
<400> 26
   Phe Gly Gly Gly Thr Lys Leu Glu Ile Lys
   1               5                   10
<210> 27
   <211> 30
   <212> PRT
   <213> Artificial sequence
<220>
   <223> Synthesized: AB7 HFW1
<400> 27
   Gln Val Gln Leu Gln Glu Ser Gly Pro Gly Leu Val Lys Pro Ser Gln
   1               5                   10                  15
   Thr Leu Ser Leu Thr Cys Ser Phe Ser Gly Phe Ser Leu Ser
               20                  25                  30
<210> 28
   <211> 14
   <212> PRT
   <213> Artificial sequence
<220>
   <223> Synthesized: AB7 HFW2
<400> 28
   Trp Ile Arg Gln Pro Ser Gly Lys Gly Leu Glu Trp Leu Ala
   1               5                   10
<210> 29
   <211> 33
   <212> PRT
   <213> Artificial sequence
<220>
   <223> Synthesized: AB7 HFW3
<400> 29
   Ser Arg Leu Thr Ile Ser Lys Asp Thr Ser Lys Asn Gln Val Ser Leu
   1               5                   10                  15
   Lys Ile Thr Ser Val Thr Ala Ala Asp Thr Ala Val Tyr Phe Cys Ala
               20                  25                  30
   Arg
<210> 30
   <211> 11
   <212> PRT
   <213> Artificial sequence
<220>
   <223> Synthesized: AB7 HFW4
<400> 30
   Trp Gly Gln Gly Thr Leu Val Thr Val Ser Ser
   1 5 10
<210> 31
   <211> 23
   <212> PRT
   <213> Artificial sequence
<220>
   <223> Synthesized: AB7 LFW1
<400> 31
   Asp Ile Gln Met Thr Gln Ser Thr Ser Ser Leu Ser Ala Ser Val Gly
   1 5 10 15
   Asp Arg Val Thr Ile Thr Cys
   20
<210> 32
   <211> 15
   <212> PRT
   <213> Artificial sequence
<220>
   <223> Synthesized: AB7 LFW2
<400> 32
   Trp Tyr Gln Gln Lys Pro Gly Lys Ala Val Lys Leu Leu Ile Tyr
   1 5 10 15
<210> 33
   <211> 32
   <212> PRT
   <213> Artificial sequence
<220>
   <223> Synthesized: AB7 LFW3
<400> 33
   Gly Val Pro Ser Arg Phe Ser Gly Ser Gly Ser Gly Thr Asp Tyr Thr
   1 5 10 15
   Leu Thr Ile Ser Ser Leu Gln Gln Glu Asp Phe Ala Thr Tyr Phe Cys
   20 25 30
<210> 34
   <211> 10
   <212> PRT
   <213> Artificial sequence
<220>
   <223> Synthesized: AB7 LFW4
<400> 34
   Phe Gly Gln Gly Thr Lys Leu Glu Ile Lys
   1               5                   10
<210> 35
   <211> 153
   <212> PRT
   <213> Artificial sequence
<220>
   <223> Synthesized: human IL-1Beta
<400> 35
   Ala Pro Val Arg Ser Leu Asn Cys Thr Leu Arg Asp Ser Gln Gln Lys
   1               5                   10                  15
   Ser Leu Val Met Ser Gly Pro Tyr Glu Leu Lys Ala Leu His Leu Gln
               20                  25                  30
   Gly Gln Asp Met Glu Gln Gln Val Val Phe Ser Met Ser Phe Val Gln
           35                  40                  45
   Gly Glu Glu Ser Asn Asp Lys Ile Pro Val Ala Leu Gly Leu Lys Glu
       50                  55                  60
   Lys Asn Leu Tyr Leu Ser Cys Val Leu Lys Asp Asp Lys Pro Thr Leu
   65                  70                  75                  80
   Gln Leu Glu Ser Val Asp Pro Lys Asn Tyr Pro Lys Lys Lys Met Glu
                   85                  90                  95
   Lys Arg Phe Val Phe Asn Lys Ile Glu Ile Asn Asn Lys Leu Glu Phe
               100                 105                 110
   Glu Ser Ala Gln Phe Pro Asn Trp Tyr Ile Ser Thr Ser Gln Ala Glu
           115                 120                 125
   Asn Met Pro Val Phe Leu Gly Gly Thr Lys Gly Gly Gln Asp Ile Thr
       130                 135                 140
   Asp Phe Thr Met Gln Phe Val Ser Ser
   145                 150